Europäisches Patentamt

European Patent Office

Office européen des brevets

(11) Publication number: **0 263 933 B1**

# EUROPEAN PATENT SPECIFICATION

(45) Date of publication of patent specification: **20.10.93**

(51) Int. Cl.⁵: **C12N 15/12**, C07K 13/00, A61K 37/02, G01N 33/574, C12Q 1/68, C12N 5/10

(21) Application number: **87111168.8**

(22) Date of filing: **03.08.87**

(54) A cDNA coding for carcinoembryonic antigen.

(30) Priority: **13.08.86 US 896361**
**19.02.87 US 16683**
**19.06.87 US 60031**

(43) Date of publication of application:
**20.04.88 Bulletin 88/16**

(45) Publication of the grant of the patent:
**20.10.93 Bulletin 93/42**

(84) Designated Contracting States:
**AT BE CH DE ES FR GB IT LI LU NL SE**

(56) References cited:
**US-A- 4 180 499**
**US-A- 4 228 236**

**CHEMICAL ABSTRACTS, vol. 101, no. 21, 19th November 1984, page 187, abstract no. 185160x, Columbus, Ohio, US; J.E. SHIVELY et al.: "Structural studies on carcinoembryonic antigen: molecular cloning of carcinoembryonic antigen using mixed synthetic oligonucleotide probes"**

(73) Proprietor: **MILES INC.**
**One Mellon Center**
**500 Grant Str.**
**Pittsburgh, PA 15219-2502(US)**

(72) Inventor: **Barnett, Thomas R.**
**27 Jeffrey Road**
**East Haven, CT 06513(US)**
Inventor: **Elting, James J.**
**5 Heatherwood Drive**
**Madison, CT 06443(US)**
Inventor: **Kamarck, Michael E.**
**46 Wauwinet Trail**
**Guilford, CT 06437(US)**

(74) Representative: **Dänner, Klaus, Dr. et al**
**Bayer AG**
**Konzernverwaltung RP**
**Patente Konzern**
**D-51368 Leverkusen (DE)**

CHEMICAL ABSTRACTS, vol. 105, no. 7, 18th August 1986, page 162, abstract no. 55557d, Columbus, Ohio, US; T. HIGASHIDE: "Studies on DNA methylation and gene expression. With special reference to the oncodevelopmental protein genes"

CHEMICAL ABSTRACTS, vol. 100, no. 23, 4th June 1984, page 216, abstract no. 187487n, Columbus, Ohio, US; W. ZIMMERMANN et al.: "Characterization of messenger RNA specific for carcinoembryonic antigen"

PROCEEDINGS OF THE NATIONAL ACADEMY OF SCIENCES OF THE USA, vol. 84, no. 16, August 1987, pages 5350-5354; M.E. KAMARCK et al.: "Carcinoembryonic antigen family: Expression in a mouse L-cell transfectant and characterization of a partial cDNA in bacteriophage lambdagt11"

PROCEEDINGS OF THE NATIONAL ACADEMY OF SCIENCES OF THE USA, vol. 84, no. 9, May 1987, pages 2960-2964; W. ZIMMERMANN et al.: "Isolation and characterization of cDNA clones encoding the human carcinoembryonic antigen reveal a highly conserved repeating structure"

PROCEEDINGS OF THE NATIONAL ACADEMY OF SCIENCES OF THE USA, vol. 84, no. 9, May 1987, pages 2965-2969; J.A. THOMPSON et al.: "Molecular cloning of a gene belonging to the carcinoembryonic antigen gene family and discussion of a domain model"

Ann. N.Y. Acad. Sci. (1983), vol. 417, pages 21-30

Prog. Cancer. Res. Ther. (1984), vol. 29, pages 147-157

**Description**

Field of the Invention

The present invention concerns an isolated nucleic acid sequence which codes for a polypeptide belonging to the CEA gene family.

Background Information

Carcinoembryonic antigen ("CEA") was first described by Gold and Freedman, J. Exp. Med., 121, 439-462, (1965). CEA is characterized as a glycoprotein of approximately 200,000 molecular weight with 50-60% by weight of carbohydrate. CEA is present during normal human fetal development, but only in very low concentration in the normal adult intestinal tract. It is produced and secreted by a number of different tumors.

CEA is a clinically useful tumor marker for the management of colorectal cancer patients. CEA can be measured using sensitive immunoassay methods. When presurgical serum levels of CEA are elevated, a postsurgical drop in serum CEA to the normal range typically indicates successful resection of the tumor. Postsurgical CEA levels that do not return to normal often indicate incomplete resection of the tumor or the presence of additional tumor sites in the patient. After returning to normal levels, subsequent rapid rises in serum CEA levels usually indicate the presence of metastases. Slower postsurgical rises from the normal level are most often interpreted to indicate the presence of new primary tumors not previously detected. Post surgical management of colon cancer patients is thus facilitated by the measurement of CEA.

CEA is a member of an antigen family. Because of this, the immunoassay of CEA by presently available methods is complicated by the fact that CEA is but one of several potentially reactive antigens. There have been at least sixteen CEA-like antigens described in the literature. Since some of these appear to be the same antigen described by different investigators, the actual number of different antigens is somewhat less than this number. Nonetheless, there is a complex array of cross-reactive antigens which can potentially interfere with an immunoassay of the CEA released by tumors. It is known that serum levels of CEA-like antigens are elevated in many non-cancerous conditions such an inflammatory liver diseases and also in smokers. It is important that immunoassays used for the monitoring of cancer patient status not be interfered with by these other CEA-like antigens. Conversely, it is important to be able to distinguish the antigens by immunoassay because of the possibility that different tumor types may preferentially express different forms of CEA. If so, then the ability to reliably measure the different forms of CEA might provide the means to diagnose or more successfully treat different forms of cancer.

U.S.P. 3,663,684, entitled "Carcinoembryonic Antigen and Diagnostic Method Using Radioactive Iodine", concerns purification and radioiodination of CEA for use in a RIA.

U.S.P. 3,697,638 describes that CEA is a mixture of antigens (Components A and B in this case). U.S.P. 3,697,638 mentions methods for separating and radioiodinating each component and their use in specific RIA's.

U.S.P. 3,852,415, entitled "Compositions for Use in Radioimmunoassay, as Substitute for Blood Plasma Extract in Determination of Carcinoembryonic Antigen" relates to the use of a buffer containing EDTA and bovine serum albumin as a substitute for plasma as a diluent for CEA RIA's.

U.S.P. 3,867,363, entitled "Carcinoembryonic Antigens", is directed to the isolation of CEA components A and B, their labelling and use in a RIA.

U.S.P. 3,927,193, entitled "Localization of Tumors by Radiolabelled Antibodies", concerns the use of radiolabelled anti-CEA antibodies in whole body tumor imaging.

U.S.P. 3,956,258, entitled "Carcinoembryonic Antigens", relates to the isolation of CEA components A and B.

U.S.P. 4,086,217, entitled "Carcinoembryonic Antigens", is directed to the isolation of CEA components A and B.

U.S.P. 4,140,753, entitled "Diagnostic Method and Reagent", concerns the purification of a CEA isomer called CEA-S1 and its use in a RIA.

U.S.P. 4,145,336, entitled "Carcinoembryonic Antigen Isomer", relates to the antigen CEA-S1.

U.S.P. 4,180,499, entitled "Carcinoembryonic Antigens", describes a process for producing CEA component B.

U.S.P. 4,228,236, entitled "Process of Producing Carcinoembryonic Antigen", is directed to the use of the established cell lines LS-174T and LS-180 or clones or derivatives thereof for the production of CEA.

U.S.P. 4,272,504, entitled "Antibody Adsorbed Support Method for Carcinoembryonic Antigen Assay", concerns two concepts for the radioimmunoassay of CEA. First, U.S.P. 4,272,504 relates to a sample pretreatment in the form of heating to 65 to 85°C at pH 5 to precipitate and eliminating extraneous protein. Second, it describes the use of a solid phase antibody (either on beads or tubes) as a means to capture analyte and radiolabelled CEA tracer.

U.S.P. 4,299,815, entitled "Carcinoembryonic Antigen Determination", concerns diluting a CEA sample with water and pretreating by heating to a temperature below which precipitation of protein will occur. The pretreated sample is then immunoassayed using RIA, EIA, FIA or chemiluminescent immunoassay.

U.S.P. 4,349,528, entitled "Monoclonal Hybridoma Antibody Specific for High Molecular Weight Carcinoembryonic Antigen", is directed to a monoclonal antibody reacting with 180 kD CEA, but not with other molecular weight forms.

U.S.P. 4,467,031, entitled "Enzyme-Immunoassay for Carcinoembryonic Antigen", relates to a sandwich enzyme immunoassay for CEA in which the first of two anti-CEA monoclonal antibodies is attached to a solid phase and the second monoclonal is conjugated with peroxidase.

U.S.P. 4,489,167, entitled "Methods and Compositions for Cancer Detection", describes that CEA shares an antigenic determinant with alpha-acid glycoprotein (AG), which is a normal component of human serum. The method described therein concerns a solid-phase sandwich enzyme immunoassay using as one antibody an antibody recognizing AG and another antibody recognizing CEA, but not AG.

U.S.P. 4,578,349, entitled "Immunoassay for Carcinoembryonic Antigen (CEA)", is directed to the use of high salt containing buffers as diluents in CEA immunoassays.

EP 113072-A, entitled "Assaying Blood Sample for Carcinoembryonic Antigen - After Removal of Interfering Materials by Incubation with Silica Gel", relates to the removal from a serum of a plasma sample of interfering substances by pretreatment with silica gel. The precleared sample is then subjected to an immunoassay.

EP 102008-A, entitled "Cancer Diagnostics Carcinoembryonic Antigen - Produced from Perchloric Acid Extracts Without Electrophoresis", relates to a procedure for the preparation of CEA from perchloric acid extracts, without the use of an electrophoresis step.

EP 92223-A, entitled "Determination of Carcinoembryonic Antigen in Cytosol or Tissue - for Therapy Control and Early Recognition of Regression", concerns an immunoassay of CEA, not in serum or plasma, but in the cytosol fraction of the tumor tissue itself.

EP 83103759.6, entitled "Cytosole-CEA-Measurement as Predictive Test in Carcinoma, Particularly Mammacarcinoma", is similar to EP 92223-A.

EP 83303759, entitled "Monoclonal Antibodies Specific to Carcinoembryonic Antigen", relates to the production of "CEA specific" monoclonal antibodies and their use in immunoassays.

WO 84/02983, entitled "Specific CEA-Family Antigens, Antibodies Specific Thereto and Their Methods of Use", is directed to the use of monoclonal antibodies to CEA-meconium (MA)-, and non-specific cross-reacting antigen (NCA)-specific epitopes in immunoassays designed to selectively measure each of these individual components in a sample.

An article in Ann. N Y. Acad. Sci., 1983, vol. 417, pages 21 - 30 describes a CEA-m RNA enriched 15-fold in a mixture with a lot of other m RNA's.

An article in Prog. Cancer Res. Ther., Vol. 29 (1984) pages 147 - 157 discloses partial amino acid sequences of CEA and six cDNA clones hybridising strongly to oligonucleotide probes based on one such partial sequence.

All of the heretofore CEA assays utilize either monoclonal or polyclonal antibodies which are generated by immunizing animals with the intact antigen of choice. None of them address the idea of making sequence specific antibodies for the detection of a unique primary sequence of the various antigens. They do not cover the use of any primary amino acid sequence for the production of antibodies to synthetic peptides or fragments of the natural product. They do not include the concept of using primary amino acid sequences to distinguish the CEA family members. None of them covers the use of DNA or RNA clones for isolating the genes with which to determine the primary sequence.

DEFINITIONS

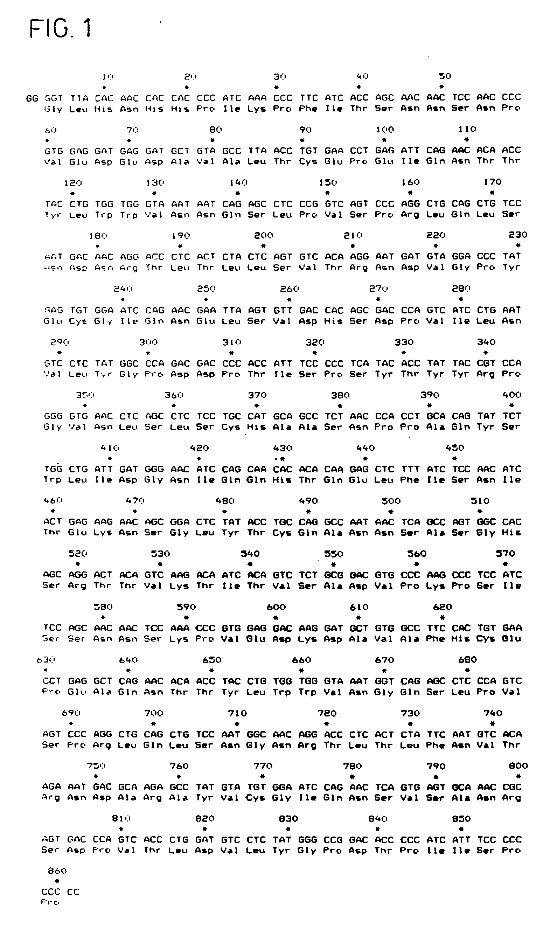

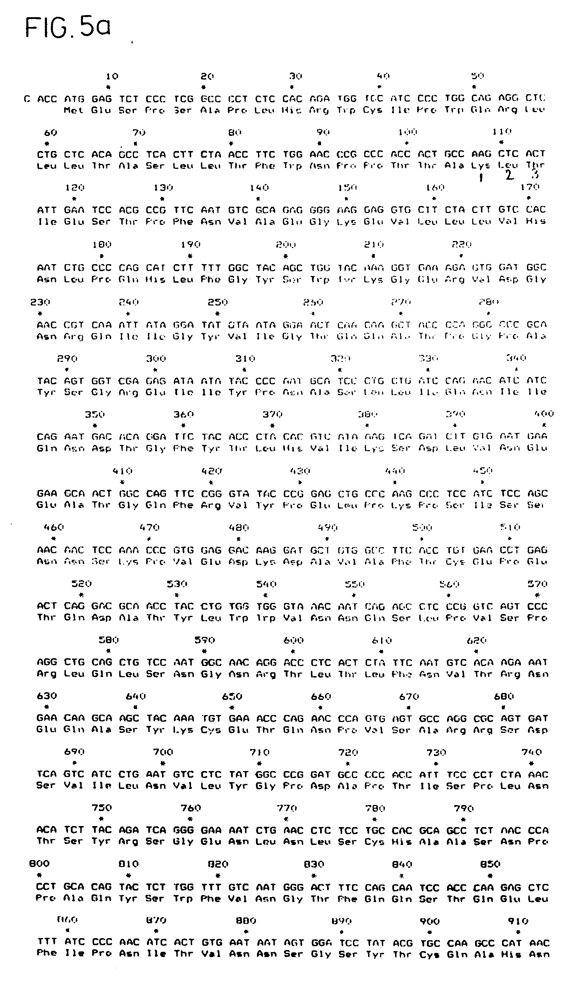

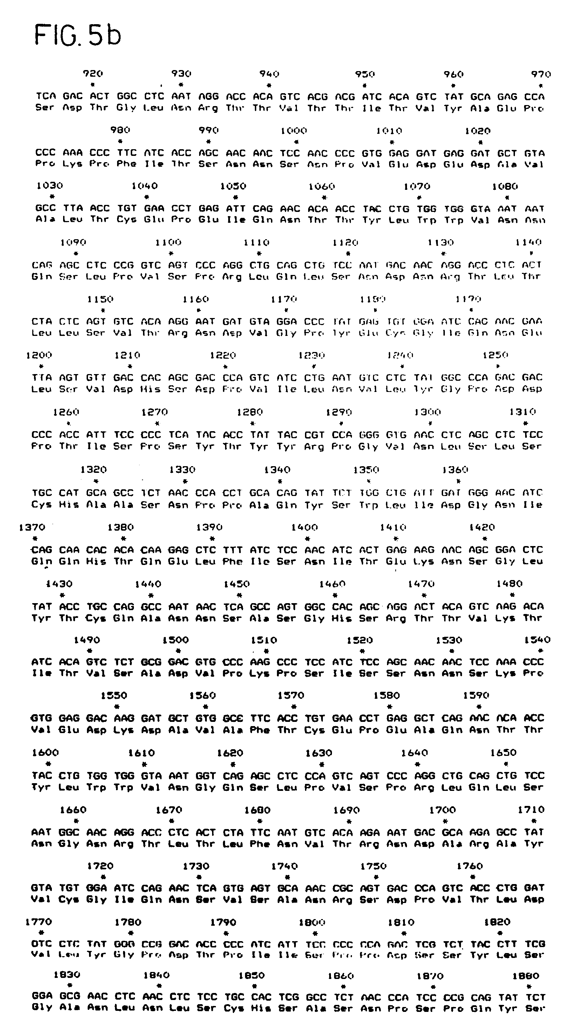

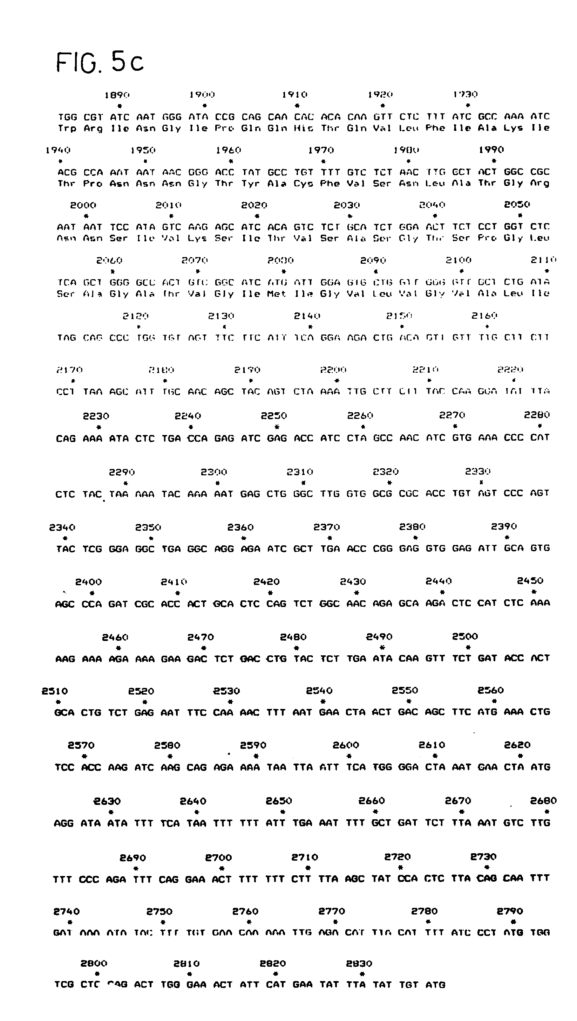

Nucleic Acid Abbreviations as Appearing, for example, in Fig. 1 and in Fig. 5:

A      adenine
G      guanine
C      cytosine

4

T     thymidine

U     uracil

Amino Acid Abbreviations as Appearing, for example, in Fig. 1 and in Fig. 5.:

Asp    aspartic acid
Asn    asparagine
Thr    threonine
Ser    serine
Glu    glutamic acid
Gln    glutamine
Pro    proline
Gly    glycine
Ala    alanine
Cys    cysteine
Val    valine
Met    methionine
Ile    isoleucine
Leu    leucine
Tyr    Tyrosine
Phe    phenylalanine
Trp    tryptophan
Lys    lysine
His    histidine
Arg    arginine

Nucleotide - A monomeric unit of DNA or RNA containing a sugar moiety (pentose), a phosphate, and a nitrogenous heterocyclic base. The base is linked to the sugar moiety via the glycosidic carbon (1' carbon of the pentose) and that combination of base and sugar is called a nucleoside. The base characterizes the nucleotide. The four DNA bases are adenine ("A"), guanine ("G"), cytosine ("C"), and thymine ("T"). The four RNA bases are A, G, C and uracil ("U").

DNA Sequence - A linear array of nucleotides connected one to the other by phosphodiester bonds between the 3' and 5' carbons of adjacent pentoses.

Functional equivalents - It is well known in the art that in a DNA sequence some nucleotides can be replaced without having an influence on the sequence of the expression product. With respect to the peptide this term means that one or more amino acids which have no function in a particular use can be deleted or replaced by another one.

Codon - A DNA sequence of three nucleotides (a triplet) which encodes through mRNA an amino acid, a translation start signal or a translation termination signal. For example, the nucleotide triplets TTA, TTG, CTT, CTC, CTA and CTG encode for the amino acid leucine ("Leu"), TAG, TAA and TGA are translation stop signals and ATG is a translation start signal.

Reading Frame - The grouping of codons during translation of mRNA into amino acid sequences. During translation, the proper reading frame must be maintained. For example, the sequence GCTGGTTGTAAG may be translated in three reading frames or phases, each of which affords a different amino acid sequence

```
GCT GGT TGT AAG    - Ala-Gly-Cys-Lys
G CTG GTT GTA AG   - Leu-Val-Val
GC TGG TTG TAA G   - Trp-Leu-(STOP).
```

Polypeptide - A linear array of amino acids connected one to the other by peptide bonds between the alpha-amino and carboxy groups of adjacent amino acids.

Genome - The entire DNA of a cell or a virus. It includes inter alia the structural genes coding for the polypeptides of the cell or virus, as well as its operator, promoter and ribosome binding and interaction sequences, including sequences such as the Shine-Dalgarno sequences.

Structural Gene - A DNA sequence which encodes through its template or messenger RNA ("mRNA") a sequence of amino acids characteristic of a specific polypeptide.

Transcription - The process of producing mRNA from a structural gene.

Translation - The process of producing a polypeptide from mRNA.

Expression - The process undergone by a structural gene to produce a polypeptide. It is a combination of transcription and translation.

Plasmid - A non-chromosomal double-stranded DNA sequence comprising an intact "replicon" such that the plasmid is replicated in a host cell. When the plasmid is placed within a unicellular organism, the characteristics of that organism may be changed or transformed as a result of the DNA of the plasmid. For example, a plasmid carrying the gene for tetracycline resistance (Tet®) transforms a cell previously sensitive to tetracycline into one which is resistant to it. A cell transformed by a plasmid is called a "transformant".

Phage or Bacteriophage - Bacterial virus, many of which consist of DNA sequences encapsilated in a protein envelope or coat ("capsid protein").

Cloning Vehicle - A plasmid, phage DNA or other DNA sequence which is capable of replicating in a host cell, which is characterized by one or a small number of endonuclease recognition sites at which such DNA sequences may be cut in a determinable fashion without attendant loss of an essential biological function of the DNA, e.g., replication, production of coat proteins or loss of promoter or binding sites, and which contains a marker suitable for use in the identification of transformed cells, e.g., tetracycline resistance or ampicillin resistance. A cloning vehicle is often called a vector.

Cloning - The process of obtaining a population of organisms or DNA sequences derived from one such organism or sequence by asexual reproduction.

Recombinant DNA Molecule or Hybrid DNA - A molecule consisting of segments of DNA from different genomes which have been joined end-to-end outside of living cells and have the capacity of infect some host cell and be maintained therein.

Expression Control Sequence - A sequence of nucleotides that controls and regulates expression of structural genes when operatively linked to those genes. They include the lac system, the trp system, major operator and promoter regions of phage $\lambda$, the control region of fd coat protein and other sequences known to control the expression of genes of prokaryotic or eukaryotic cells of their viruses.

Transformation/Transfection - DNA or RNA is introduced into cells to allow gene expression.

"Infected" referred to herein concerns the introduction of RNA or DNA by a viral vector into the host.

"Injected" referred to herein concerns the microinjection (use of a small syringe) of DNA into a cell.

CEA-A, CEA-B, CEA-C, CEA-D mentioned hereinbelow are members of the CEA family which are described in the examples below.

## SUMMARY OF THE INVENTION

The present invention concerns an isolated nucleic acid which codes for a polypeptide belonging to the CEA gene family, selected from the sequences listed below, and fragments thereof comprising at least 50 sequential bases.

```
  1  GGGGTTTACA CAACCACCAC CCCATCAAAC CCTTCATCAC CAGCAACAAC TCCAACCCCG
 61  TGGAGGATGA GGATGCTGTA GCCTTAACCT GTGAACCTGA GATTCAGAAC ACAACCTACC
121  TGTGGTGGGT AAATAATCAG AGCCTCCCGG TCAGTCCCAG GCTGCAGCTG TCCAATGACA
181  ACAGGACCCT CACTCTACTC AGTGTCACAA GGAATGATGT AGGACCCTAT GAGTGTGGAA
241  TCCAGAACGA ATTAAGTGTT GACCACAGCG ACCCAGTCAT CCTGAATGTC CTCTATGGCC
301  CAGACGACCC CACCATTTCC CCCTCATACA CCTATTACCG TCCAGGGGTG AACCTCAGCC
361  TCTCCTGCCA TGCAGCCTCT AACCCACCTG CACAGTATTC TTGGCTGATT GATGGGAACA
421  TCCAGCAACA CACACAAGAG CTCTTTATCT CCAACATCAC TGAGAAGAAC AGCGGACTCT
481  ATACCTGCCA GGCCAATAAC TCAGCCAGTG GCCACAGCAG GACTACAGTC AAGACAATCA
541  CAGTCTCTGC GGACGTGCCC AAGCCCTCCA TCTCCAGCAA CAACTCCAAA CCCGTGGAGG
601  ACAAGGATGC TGTGGCCTTC CACTGTGAAC CTGAGGCTCA GAACACAACC TACCTGTGGT
661  GGGTAAATGG TCAGAGCCTC CCAGTCAGTC CCAGGCTGCA GCTGTCCAAT GGCAACAGGA
721  CCCTCACTCT ATTCAATGTC ACAAGAAATG ACGCAAGAGC CTATGTATGT GGAATCCAGA
781  ACTCAGTGAG TGCAAACCGC AGTGACCCAG TCACCCTGGA TGTCCTCTAT GGGCCGGACA
841  CCCCCATCAT TTCCCCCCCC CC
```

Sequence displayed from position 1 to end (position 862)
Sequence numbered from position 1

6

The nucleic acid which codes for the complete CEA protein and comprises the above sequence will have a total of no more than about 5000, more usually no more than about 3600, bases. Fragments of cDNA of the above sequences will have at least 10, and in some cases at least about 50, bases.

The above DNA sequence of 859 bases codes for an immunoreactive fragment of CEA which can be expressed, such as in lambda gt11. The sequence has an internal repeat sequence approximately 300 bases long and codes for a theoretical protein 285 amino acids long. This cDNA encodes for sequences found in other human genes and it is believed that this entire family of genes may code for the CEA family of proteins.

The present invention also concerns a DNA sequence having 2839 bp comprising the base sequence set forth below or a fragment thereof

```
              10          20          30          40          50
C ACC ATG GAG TCT CCC TCG GCC CCT CTC CAC AGA TGG TGC ATC CCC TGG CAG AGG CTC

     60          70          80          90         100         110
CTG CTC ACA GCC TCA CTT CTA ACC TTC TGG AAC CCG CCC ACC ACT GCC AAG CTC ACT

    120         130         140         150         160         170
ATT GAA TCC ACG CCG TTC AAT GTC GCA GAG GGG AAG GAG GTG CTT CTA CTT GTC CAC

    180         190         200         210         220
AAT CTG CCC CAG CAT CTT TTT GGC TAC AGC TGG TAC AAA GGT GAA AGA GTG GAT GGC

230         240         250         260         270         280
AAC CGT CAA ATT ATA GGA TAT GTA ATA GGA ACT CAA CAA GCT ACC CCA GGG CCC GCA

    290         300         310         320         330         340
TAC AGT GGT CGA GAG ATA ATA TAC CCC AAT GCA TCC CTG CTG ATC CAG AAC ATC ATC

     350         360         370         380         390         400
CAG AAT GAC ACA GGA TTC TAC ACC CTA CAC GTC ATA AAG TCA GAT CTT GTG AAT GAA

    410         420         430         440         450
GAA GCA ACT GGC CAG TTC CGG GTA TAC CCG GAG CTG CCC AAG CCC TCC ATC TCC AGC

460         470         480         490         500         510
AAC AAC TCC AAA CCC GTG GAG GAC AAG GAT GCT GTG GCC TTC ACC TGT GAA CCT GAG
```

EP 0 263 933 B1

```
        520          530          540          550        .      560            570
         •            •            •            •                  •
 ACT CAG GAC GCA ACC TAC CTG TGG TGG GTA AAC AAT CAG AGC CTC CCG GTC AGT CCC

              580           590          600          610             620
               •            •            •            •                •
 AGG CTG CAG CTG TCC AAT GGC AAC AGG ACC CTC ACT CTA TTC AAT GTC ACA AGA AAT

    630          640          650          660          670             680
     •            •            •            •            •                •
 GAA CAA GCA AGC TAC AAA TGT GAA ACC CAG AAC CCA GTG AGT GCC AGG CGC AGT GAT

       690          700          710          720          730            740
        •            •            •            •            •              •
 TCA GTC ATC CTG AAT GTC CTC TAT GGC CCG GAT GCC CCC ACC ATT TCC CCT CTA AAC

           750          760          770          780          790
            •            •            •            •            •
 ACA TCT TAC AGA TCA GGG GAA AAT CTG AAC CTC TCC TGC CAC GCA GCC TCT AAC CCA

 800          810          820          830          840          850
  •            •            •            •            •            •
 CCT GCA CAG TAC TCT TGG TTT GTC AAT GGG ACT TTC CAG CAA TCC ACC CAA GAG CTC

     860          870          880          890          900            910
      •            •            •            •            •              •
 TTT ATC CCC AAC ATC ACT GTG AAT AAT AGT GGA TCC TAT ACG TGC CAA GCC CAT AAC

        920          930          940          950          960            970
         •            •            •            •            •              •
 TCA GAC ACT GGC CTC AAT AGG ACC ACA GTC ACG ACG ATC ACA GTC TAT GCA GAG CCA

            980          990         1000         1010         1020
             •            •            •            •            •
 CCC AAA CCC TTC ATC ACC AGC AAC AAC TCC AAC CCC GTG GAG GAT GAG GAT GCT GTA

    1030         1040         1050         1060         1070         1080
     •            •            •            •            •            •
 GCC TTA ACC TGT GAA CCT GAG ATT CAG AAC ACA ACC TAC CTG TGG TGG GTA AAT AAT

       1090         1100         1110         1120         1130         1140
        •            •            •            •            •            •
 CAG AGC CTC CCG GTC AGT CCC AGG CTG CAG CTG TCC AAT GAC AAC AGG ACC CTC ACT

           1150         1160         1170         1180         1190
            •            •            •            •            •
 CTA CTC AGT GTC ACA AGG AAT GAT GTA GGA CCC TAT GAG TGT GGA ATC CAG AAC GAA

 1200         1210         1220         1230         1240         1250
  •            •            •            •            •            •
 TTA AGT GTT GAC CAC AGC GAC CCA GTC ATC CTG AAT GTC CTC TAT GGC CCA GAC GAC

    1260         1270         1280         1290         1300         1310
     •            •            •            •            •            •
 CCC ACC ATT TCC CCC TCA TAC ACC TAT TAC CGT CCA GGG GTG AAC CTC AGC CTC TCC

        1320         1330         1340         1350         1360
         •            •            •            •            •
 TGC CAT GCA GCC TCT AAC CCA CCT GCA CAG TAT TCT TGG CTG ATT GAT GGG AAC ATC

 1370         1380         1390         1400         1410         1420
  •            •            •            •            •            •
 CAG CAA CAC ACA CAA GAG CTC TTT ATC TCC AAC ATC ACT GAG AAG AAC AGC GGA CTC
```

8

EP 0 263 933 B1

```
        1430            1440            1450            1460            1470            1480
TAT ACC TGC CAG GCC AAT AAC TCA GCC AGT GGC CAC AGC AGG ACT ACA GTC AAG ACA

        1490            1500            1510            1520            1530            1540
ATC ACA GTC TCT GCG GAC GTG CCC AAG CCC TCC ATC TCC AGC AAC AAC TCC AAA CCC

        1550            1560            1570            1580            1590
GTG GAG GAC AAG GAT GCT GTG GCC TTC ACC TGT GAA CCT GAG GCT CAG AAC ACA ACC

    1600            1610            1620            1630            1640            1650
TAC CTG TGG TGG GTA AAT GGT CAG AGC CTC CCA GTC AGT CCC AGG CTG CAG CTG TCC

        1660            1670            1680            1690            1700            1710
AAT GGC AAC AGG ACC CTC ACT CTA TTC AAT GTC ACA AGA AAT GAC GCA AGA GCC TAT

        1720            1730            1740            1750            1760
GTA TGT GGA ATC CAG AAC TCA GTG AGT GCA AAC CGC AGT GAC CCA GTC ACC CTG GAT

    1770            1780            1790            1800            1810            1820
GTC CTC TAT GGG CCG GAC ACC CCC ATC ATT TCC CCC CCA GAC TCG TCT TAC CTT TCG

        1830            1840            1850            1860            1870            1880
GGA GCG AAC CTC AAC CTC TCC TGC CAC TCG GCC TCT AAC CCA TCC CCG CAG TAT TCT

        1890            1900            1910            1920            1930
TGG CGT ATC AAT GGG ATA CCG CAG CAA CAC ACA CAA GTT CTC TTT ATC GCC AAA ATC

    1940            1950            1960            1970            1980            1990
ACG CCA AAT AAT AAC GGG ACC TAT GCC TGT TTT GTC TCT AAC TTG GCT ACT GGC CGC

        2000            2010            2020            2030            2040            2050
AAT AAT TCC ATA GTC AAG AGC ATC ACA GTC TCT GCA TCT GGA ACT TCT CCT GGT CTC

        2060            2070            2080            2090            2100            2110
TCA GCT GGG GCC ACT GTC GGC ATC ATG ATT GGA GTG CTG GTT GGG GTT GCT CTG ATA

        2120            2130            2140            2150            2160
TAG CAG CCC TGG TGT AGT TTC TTC ATT TCA GGA AGA CTG ACA GTT GTT TTG CTT CTT

    2170            2180            2190            2200            2210            2220
CCT TAA AGC ATT TGC AAC AGC TAC AGT CTA AAA TTG CTT CTT TAC CAA GGA TAT TTA

        2230            2240            2250            2260            2270            2280
CAG AAA ATA CTC TGA CCA GAG ATC GAG ACC ATC CTA GCC AAC ATC GTG AAA CCC CAT

        2290            2300            2310            2320            2330
CTC TAC TAA AAA TAC AAA AAT GAG CTG GGC TTG GTG GCG CGC ACC TGT AGT CCC AGT
```

9

```
     2340          2350           2360           2370            2380                 2390
      •             •              •              •               •                    •
    TAC TCG GGA GGC TGA GGC AGG AGA ATC GCT TGA ACC CGG GAG GTG GAG ATT GCA GTG

         2400           2410            2420           2430            2440                2450
          •              •               •              •               •                  •
       AGC CCA GAT CGC ACC ACT GCA CTC CAG. TCT GGC AAC AGA GCA AGA CTC CAT CTC AAA

            2460           2470           2480           2490            2500
             •              •              •              •               •
       AAG AAA AGA AAA GAA GAC TCT GAC CTG TAC TCT TGA ATA CAA GTT TCT GAT ACC ACT

     2510          2520           2530           2540            2550            2560
      •             •              •              •               •               •
       GCA CTG TCT GAG AAT TTC CAA AAC TTT AAT GAA CTA ACT GAC AGC TTC ATG AAA CTG

         2570          2580           2590           2600            2610              2620
          •             •              •              •               •                 •
       TCC ACC AAG ATC AAG CAG AGA AAA TAA TTA ATT TCA TGG GGA CTA AAT GAA CTA ATG

            2630          2640           2650           2660            2670              2680
             •             •              •              •               •                 •
       AGG ATA ATA TTT TCA TAA TTT TTT ATT TGA AAT TTT GCT GAT TCT TTA AAT GTC TTG

            2690          2700           2710           2720            2730
             •             •              •              •               •
       TTT CCC AGA TTT CAG GAA ACT TTT TTT CTT TTA AGC TAT CCA CTC TTA CAG CAA TTT

     2740          2750           2760           2770            2780            2790
      •             •              •              •               •               •
    GAT AAA ATA TAC TTT TGT GAA CAA AAA TTG AGA CAT TTA CAT TTT ATC CCT ATG TGG

         2800           2810           2820           2830
          •              •              •              •
       TCG CTC CAG ACT TGG GAA ACT ATT CAT GAA TAT TTA TAT TGT ATG
```

Further sequences are shown in the examples hereinbelow which code for proteins which are also members of the CEA family.

The present invention is also directed to a replicable recombinant cloning vehicle ("vector") having an insert comprising a nucleic acid, e.g., DNA, which comprises a base sequence as claimed herewith.

This invention also relates to a cell that is transformed/transfected, infected or injected with the above described replicable recombinant cloning vehicle. Thus the invention also concerns the transfection of cells using free nucleic acid, without the use of a cloning vehicle.

Still further, the present invention concerns a polypeptide expressed by the above described transfected, infected or injected cell, which polypeptide exhibits immunological cross-reactivity with a CEA, as well as labelled forms of the polypeptide. The invention also relates to polypeptides having an amino acid sequence, i.e., synthetic peptides, of the expression product of a cell that is transfected, injected, infected with the above described replicable recombinant cloning vehicles, as well as labelled forms thereof. Stated otherwise, the present invention concerns a synthetic peptide having an amino acid sequence corresponding to the entire amino acid sequence or a portion thereof having no less than five amino acids of the aforesaid expression product.

The invention further relates to an antibody preparation specific for the above described polypeptide.

Another aspect of the invention concerns an immunoassay method for detecting CEA or a functional equivalent thereof in a test sample comprising

(a) contacting the sample with the above described antibody preparation, and

(b) determining binding thereof to CEA in the sample.

The invention also is directed to a nucleic acid hybridization method for detecting a CEA or a related nucleic acid (DNA or RNA) sample in a test sample comprising

(a) contacting the test sample with a nucleic acid probe comprising a nucleic acid, which comprises a base sequence which codes for a CEA peptide sequence or a base sequence that is hybridizable therewith, and

(b) determining the formation of the resultant hybridized probe.

10

The present invention also concerns a method for detecting the presence of carcinoembryonic antigen or a functional equivalent thereof in an animal or human patient in vivo comprising

a) introducing into said patient a labeled (e.g., a radio-opaque material that can be detected by X-rays, radiolabeled or labeled with paramagnetic materials that can be detected by NMR) antibody preparation according to the present invention and

b) detecting the presence of such antibody preparation in the patient by detecting the label.

In another aspect, the present invention relates to the use of an antibody preparation according to the present invention for therapeutic purposes, namely, attaching to an antibody preparation radionuclides or toxins to form a complex and introducing an effective amount of such complex into an animal or human patient, e.g., by injection or orally. The labeled complex would attach to CEA in a patient and the radionuclide or toxin would serve to destroy the CEA expressing cell.

## BRIEF DESCRIPTION OF THE DRAWINGS

Fig. 1 is an amino acid and nucleic acid sequence (859 bases) of a cDNA sequence according to the present invention.

Fig. 2 is a graph depicting an enzyme immunoassay of CEA fusion protein according to the present invention with and without antigen ("Ag").

Fig. 3 is an immunoblot of a CEA fusion protein according to the present invention.

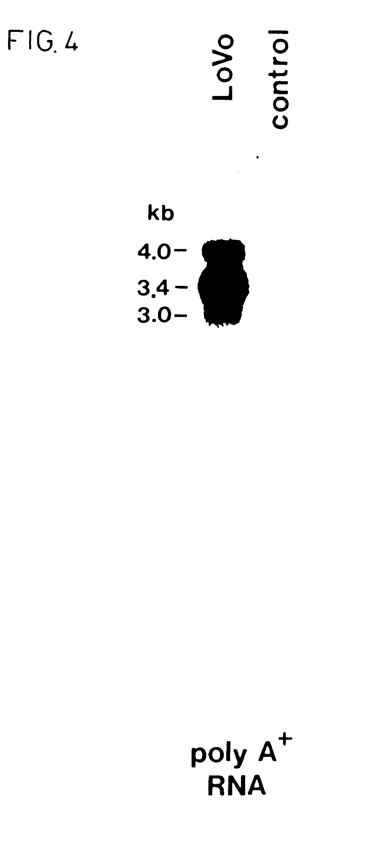

Fig. 4 is an autoradiograph of blots for the detection of a poly A+ RNA according to the present invention.

Fig. 5 is an amino acid and nucleic acid sequence (2839 bases) of a cDNA sequence according to the present invention. In Fig. 5, the first amino acid for residue sequence purposes is Lys in the second row third amino acid from the right. Thereafter, the remaining amino acids are numbered consecutively.

## DETAILED DESCRIPTION OF THE INVENTION

Some CEA epitopes are unique. These are the epitopes which have been useful for distinguishing the various CEA-like antigens immunologically. Peptide epitopes are defined by the linear amino acid sequence of the antigen and/or features resulting from protein folding. The information required for protein folding is encoded in the primary amino acid sequence. Therefore, antigenic differences ultimately result from differences in the primary structure of the different CEA molecules. The differences residing in the CEA protein in the CEA species can thus be determined by determining the primary amino acid sequences. This can be most readily accomplished by cloning and sequencing each of the genes for CEA. To determine which gene products will be most useful for cancer diagnosis, unique probes can be selected for each gene and expression of each gene can be determined in different tumor types by nucleic acid hybridization techniques. The present invention provides a tool with which to identify potential genes coding for different members of the CEA family and to determine the theoretical primary amino acid sequences for them. Using the method of automated peptide synthesis, peptides can then be synthesized corresponding to unique sequences in these antigens. With these peptides, antibodies to these sequences can be produced which, in the intact CEA molecule, might not be recognized by the animal being immunized. Having accomplished this, advantage can then be taken of the differences in these antigens to generate specific immunoassays for the measurement of each antigen.

A wide variety of host/cloning vehicle combinations may be employed in cloning the double-stranded nucleic acid prepared in accordance with this invention. For example, useful cloning vehicles may consist of segments of chromosomal, non-chromosomal and synthetic DNA sequences, such as various known derivatives of SV40 and known bacterial plasmids, e.g., plasmids from E. coli including col E1, pCR1, pBR322, pMB89 and their derivatives, wider host range plasmids, e.g., RP4, and phage DNAs, e.g., the numerous derivatives of phage, e.g., NM989, and other DNA phages, e.g., M13 and Filamenteous single-stranded DNA phages and vectors derived from combinations of plasmids and phage DNAs such as plasmids which have been modified to employ phage DNA or other expression control sequences or yeast plasmids such as the 2 μ plasmid or derivatives thereof. Useful hosts may include bacterial hosts such as strains of E. coli, such as E. coli HB 101, E. coli X1776, E. coli X2282, E. coli MRCI and strains of Pseudomonas, Bacillus subtilis, Bacillus stearothermophilus and other E. coli, bacilli, yeasts and other fungi, animal or plant hosts such as animal (including human) or plant cells in culture or other hosts. Of course, not all host/vector combinations may be equally efficient. The particular selection of host/cloning vehicle combination may be made by those of skill in the art after due consideration of the principles set forth without departing from the scope of this invention.

11

Furthermore, within each specific cloning vehicle, various sites may be selected for insertion of the nucleic acid according to the present invention. These sites are usually designated by the restriction endonuclease which cuts them. For example, in pBR322 the PstI site is located in the gene for beta-lactamase, between the nucleotide triplets that code for amino acids 181 and 182 of that protein. One of the two HindII endonuclease recognition sites is between the triplets coding for amino acids 101 and 102 and one of the several Taq sites at the triplet coding for amino acid 45 of beta-lactamase in pBR322. In similar fashion, the EcoRI site and the PvuII site in this plasmid lie outside of any coding region, the EcoRI site being located between the genes coding for resistance to tetracycline and ampicillin, respectively. These sites are well recognized by those of skill in the art. It is, of course, to be understood that a cloning vehicle useful in this invention need not have a restriction endonuclease site for insertion of the chosen DNA fragment. Instead, the vehicle could be cut and joined to the fragment by alternative means.

The vector or cloning vehicle and in particular the site chosen therein for attachment of a selected nucleic acid fragment to form a recombinant nucleic acid molecule is determined by a variety of factors, e.g., the number of sites susceptible to a particular restriction enzyme, the size of the protein to be expressed, the susceptibility of the desired protein to proteolytic degradation by host cell enzymes, the contamination of the protein to be expressed by host cell proteins difficult to remove during purification, the expression characteristics, such as the location of start and stop codons relative to the vector sequences, and other factors recognized by those of skill in the art. The choice of a vector and an insertion site for a particular gene is determined by a balance of these factors, not all sections being equally effective for a given case.

Methods of inserting nucleic acid sequences into cloning vehicles to form recombinant nucleic acid molecules include, for example, dA-dT tailing, direct ligation, synthetic linkers, exonuclease and polymerase-linked repair reactions followed by ligation, or extension of the nucleic acid strand with an appropriate polymerase and an appropriate single-stranded template followed by ligation.

It should also be understood that the nucleotide sequences or nucleic acid fragments inserted at the selected site of the cloning vehicle may include nucleotides which are not part of the actual structural gene for the desired polypeptide or mature protein or may include only a fragment of the complete structural gene for the desired protein or mature protein.

The cloning vehicle or vector containing the foreign gene is employed to transform an appropriate host so as to permit that host to replicate the foreign gene and to express the protein coded by the foreign gene or portion thereof. The selection of an appropriate host is also controlled by a number of factors recognized by the art. These include, for example, the compatibility with the chosen vector, the toxicity of proteins encoded by the hybrid plasmid, the ease of recovery of the desired protein, the expression characteristics, biosafety and costs. A balance of these factors must be struck with the understanding that not all hosts may be equally effective for expression of a particular recombinant DNA molecule.

The level of production of a protein is governed by two major factors: the number of copies of its gene within the cell and the efficiency with which those gene copies are transcribed and translated. Efficiency of transcription and translation (which together comprise expression) is in turn dependent upon nucleotide sequences, normally situated ahead of the desired coding sequence. These nucleotide sequences or expression control sequences define inter alia, the location at which RNA polymerase interacts to initiate transcription (the promoter sequence) and at which ribosomes bind and interact with the mRNA (the product of transcription) to initiate translation. Not all such expression control sequences function with equal efficiency. It is thus of advantage to separate the specific coding sequences for the desired protein from their adjacent nucleotide sequences and fuse them instead to other known expression control sequences so as to favor higher levels of expression. This having been achieved, the newly engineered nucleic acid, e.g., DNA, fragment may be inserted into a multicopy plasmid or a bacteriophage derivative in order to increase the number of gene copies within the cell and thereby further improve the yield of expressed protein.

Several expression control sequences may be employed as described above. These include the operator, promoter and ribosome binding and interaction sequences (including sequences such as the Shine-Dalgarno sequences) of the lactose operon of E. coli ("the lac system"), the corresponding sequences of the tryptophan synthetase system of E. coli ("the trp system"), the major operator and promoter regions of phage $\lambda$ ($O_LP_L$ and $O_RP_R$), the control region of Filamenteous single-stranded DNA phages, or other sequences which control the expression of genes of prokaryotic or eukaryotic cells and their viruses. Therefore, to improve the production of a particular polypeptide in an appropriate host, the gene coding for that polypeptide may be selected and removed from a recombinant nucleic acid molecule containing it and reinserted into a recombinant nucleic acid molecule closer or in a more appropriate relationship to its former expression control sequence or under the control of one of the above described expression control sequences. Such methods are known in the art.

As used herein "relationship" may encompass many factors, e.g., the distance separating the expression enhancing and promoting regions of the recombinant nucleic acid molecule and the inserted nucleic acid sequence, the transcription and translation characteristics of the inserted nucleic acid sequence or other sequences in the vector itself, the particular nucleotide sequence of the inserted nucleic acid sequence and other sequences of the vector and the particular characteristics of the expression enhancing and promoting regions of the vector.

Further increases in the cellular yield of the desired products depend upon an increase in the number of genes that can be utilized in the cell. This is achieved, for illustration purposes, by insertion of recombinant nucleic acid molecules engineered into the temperate bacteriophage $\lambda$ (NM989), most simply by digestion of the plasmid with a restriction enzyme, to give a linear molecule which is then mixed with a restricted phage $\lambda$ cloning vehicle (e.g., of the type described by N. E. Murray et al, "Lambdoid Phages That Simplify the Recovery of In Vitro Recombinants", Molec. Gen. Genet., 150, pp. 53-61 (1977) and N. E. Murray et al, "Molecular Cloning of the DNA Ligase Gene From Bacteriophage T4", J. Mol. Biol., 132, pp. 493-505 (1979)) and the recombinant DNA molecule recircularized by incubation with DNA ligase. The desired recombinant phage is then selected as before and used to lysogenise a host strain of E. coli.

Particularly useful $\lambda$ cloning vehicles contain a temperature-sensitive mutation in the repression gene cl and suppressible mutations in gene S, the product of which is necessary for lysis of the host cell, and gene E, the product which is major capsid protein of the virus. With this system, the lysogenic cells are grown at 32°C and then heated to 45°C to induce excision of the prophage. Prolonged growth at 37°C leads to high levels of production of the protein, which is retained within the cells, since these are not lysed by phage gene products in the normal way, and since the phage gene insert is not encapsilated it remains available for further transcription. Artificial lysis of the cells then releases the desired product in high yield.

In addition, it should be understood that the yield of polypeptides prepared in accordance with this invention may also be improved by substituting different codons for some or all of the codons of the present DNA sequences, these substituted codons coding for amino acids identical to those coded for by the codons replaced.

Finally, the activity of the polypeptides produced by the recombinant nucleic acid molecules of this invention may be improved by fragmenting, modifying or derivatizing the nucleic acid sequences or polypeptides of this invention by well-known means, without departing from the scope of this invention.

Regarding the synthetic peptides according to the invention, chemical synthesis of peptides is described in the following publications: S.B.H. Kent, Biomedical Polymers, eds. Goldberg, E.P. and Nakajima, A. (Academic Press, New York), 213-242, (1980); A.R. Mitchell, S.B.H. Kent, M. Engelhard and R.B. Merrifield, J. Org. Chem., 43, 2845-2852, (1978); J.P. Tam, T.-W. Wong, M. Riemen, F.-S. Tjoeng and R.B. Merrifield, Tet. Letters, 4033-4036, (1979); S. Mojsov, A.R. Mitchell and R.B. Merrifield, J. Org. Chem., 45, 555-560, (1980); J.P. Tam, R.D. Diharchi and R.B. Merrifield, Tet. Letters, 2851-2854, (1981); and S.B.H. Kent, M. Riemen, M. Le Doux and R.B. Merrifield, Proceedings of the IV International Symposium on Methods of Protein Sequence Analysis, (Brookhaven Press, Brookhaven, NY), in press, 1981.

In the Merrifield solid phase procedure, the appropriate sequence of L-amino acids is built up from the carboxyl terminal amino acid to the amino terminal amino acid. Starting with the appropriate carboxyl terminal amino acid attached to a polystyrene (or other appropriate) resin via chemical linkage to a chloromethyl group, benzhydrylamine group, or other reactive group of the resin, amino acids are added one by one using the following procedure. The peptide-resin is:

(a) washed with methylene chloride;

(b) neutralized by making for 10 minutes at room temperature with 5% (v/v) diisopropylethylamine (or other hindered base) in methylene chloride;

(c) washed with methylene chloride;

(d) an amount of amino acid equal to six times the molar amount of the growing peptide chain is activated by combining it with one-half as many moles of a carbodiimide (e.g., dicyclohexylcarbodiimide, or diisopropylcarbodiimide) for ten minutes at 0°C, to form the symmetric anhydride of the amino acid. The amino acid used should be provided originally as the N-alpha-tert.-butyloxycarbonyl derivative, with side chains protected with benzyl esters (e.g., aspartic or glutamic acids), benzyl ethers (e.g., serine, threonine, cysteine or tyrosine), benzyloxycarbonyl groups (e.g., lysine) or other protecting groups commonly used in peptide synthesis;

(e) the activated amino acid is reacted with the peptide-resin for two hours at room temperature, resulting in addition of the new amino acid to the end of the growing peptide chain;

(f) the peptide-resin is washed with methylene chloride;

(g) the N-alpha-(tert.-butyloxycarbonyl) group is removed from the most recently added amino acid by reacting with 30 to 65%, preferably 50% (v/v) trifluoroacetic acid in methylene chloride for 10 to 30 minutes at room temperature;

(h) the peptide-resin is washed with methylene chloride;

(i) steps (a) through (h) are repeated until the required peptide sequence has been constructed.

The peptide is then removed from the resin and simultaneously the side-chain protecting groups are removed, by reaction with anhydrous hydrofluoric acid containing 10% v/v of anisole or other suitable (aromatic) scavenger. Subsequently, the peptide can be purified by gel filtration, ion exchange, high pressure liquid chromatography, or other suitable means.

In some cases, chemical synthesis can be carried out without the solid phase resin, in which case the synthetic reactions are performed entirely in solution. The reactions are similar and well known in the art, and the final product is essentially identical.

Digestion of the polypeptide can be accomplished by using proteolytic enzymes, especially those enzymes whose substrate specificity results in cleavage of the polypeptide at sites immediately adjacent to the desired sequence of amino acids.

Cleavage of the polypeptide can be accomplished by chemical means. Particular bonds between amino acids can be cleaved by reaction with specific reagents. Examples include the following: bonds involving methionine are cleaved by cyanogen bromide; asparaginyl-glycine bonds are cleaved by hydroxylamine.

The following is a non-limiting list of sequences believed to be important for the purpose of making synthetic peptides and antibodies to them. Each antigen is categorized into three levels: 1) large regions containing potentially useful sites within; 2) sub regions of 1 and; 3) individual sites considered to be the prime targets for use as synthetic peptide antigens.

| Antigen | Size Category (see above) | Amino Acid from Residue | Amino Acid to Residue |
|---|---|---|---|
| CEA (see Fig. 5) | 1 | 135 | 386 |
| | 2 | 174 | 220 |
| | | 285 | 386 |
| | 3 | 174 | 200 |
| | | 208 | 216 |
| | | 285 | 290 |
| | | 292 | 308 |
| | | 312 | 321 |
| | | 360 | 372 |
| | | 380 | 386 |
| FL-5 (the sequence for FL-5 is given herein-below) | 1 | 135 | 386 |
| | | 411 | 492 |
| | 2 | 174 | 220 |
| | | 285 | 389 |
| | 3 | 174 | 200 |
| | | 208 | 222 |
| | | 285 | 292 |
| | | 295 | 311 |
| | | 315 | 328 |
| | | 331 | 345 |
| | | 348 | 360 |
| | | 363 | 377 |
| | | 383 | 390 |
| BT 20 (the sequence for BT 20 is given herein-below) | 1 | 135 | 285 |
| | 2 | 168 | 220 |
| | 3 | 168 | 196 |
| | | 210 | 220 |
| | | 264 | 283 |

The present invention has the following advantages:

EP 0 263 933 B1

(1) The nucleic acid coding for CEA according to the invention can be used as a probe to isolate the complete gene of which it is a part.

(2) It can be used as a probe to isolate other members of the CEA gene family.

(3) It can be used as an oligonucleotide probe to determine the expression of this gene in various tumor types.

(4) The nuclcotide sequence can be used to predict the primary amino acid sequence of the protein for production of synthetic peptides and can be used to distinguish members of the CEA family.

(5) The synthetic peptides derived from the above sequences can be used to produce sequence-specific antibodies.

(6) Immunoassays for each member of the CEA family can be produced with these sequence-specific antibodies and synthetic peptides.

(7) These immunoassays can be used as diagnostics for different types of cancer if it is determined that different members of the CEA family are clinically useful markers for different types of cancer.

Polypeptides according to the present invention are labelled by conventional means using radioactive moieties, e.g., $I^{125}$ , enzymes, dyes and fluorescent compounds, just to name a few.

Several possible configurations for immunoassays according to the present invention can be used. The readout systems capable of being employed in these assays are numerous and non-limiting examples of such systems include fluorescent and colorimetric enzyme systems, radioisotopic labelling and detection and chemiluminescent systems. Two examples of immunoassay methods are as follows:

(1) An enzyme linked immunoassay (ELISA) using an antibody preparation according to the present invention (including Fab or F(ab)' fragments derived therefrom) to a solid phase (such as a microtiter plate or latex beads) is attached a purified anti-CEA antibody of a specificity other than that which is conjugated to the enzyme. This solid phase antibody is contacted with the sample containing CEA, After washing, the solid phase antibody-CEA complex is contacted with the conjugated anti-peptide antibody (or conjugated fragment). After washing away unbound conjugate, color or fluorescence is developed by adding a chromogenic or fluorogenic substrate for the enzyme. The amount of color or fluorescence developed is proportional to the amount of CEA in the sample.

(2) A competitive fluorometric immunoassay using fluorescently labelled peptide or synthetic peptides of the sequence specified by cLV7. In this example, the purified peptide expressed by cells or synthetic peptides thereof are fluorescently labelled. To a solid phase is attached a purified anti-CEA antibody. This solid phase is then contacted with sample containing CEA to which has been added fluorescent peptide probe. After binding, excess probe is washed away the amount of bound probe is quantitated. The amount of bound fluorescent probe will be inversely proportional to the amount of CEA in the sample.

In the nucleic acid hybridization method according to the present invention, the nucleic acid probe is conjugated with a label, for example, an enzyme, a fluorophore, a radioisotope, a chemiluminescent compound, etc. In the most general case, the probe would be contacted with the sample and the presence of any hybridizable nucleic acid sequence would be detected by developing in the presence of a chromogenic enzyme substrate, detection of the fluorophore by epifluorescence, by autoradiography of the radioisotopically labelled probe or by chemiluminescence. The detection of hybridizable RNA sequences can be accomplished by (1) a dot blot methodology or (2) an in situ hybridization methodology. Methods for these last two techniques are described by D. Gillespie and J. Bresser, "mRNA Immobilization in NaI: Quick Blots", Biotechniques, 184-192, November/December 1983 and J. Lawrence and R. Singer, "Intracellular Localization of Messenger RNAs for Cytosketal Proteins", Cell, 45, 407-415, May 9, 1986, respectively. The readout systems can be the same as described above, e.g., enzyme labelling, radiolabelling, etc.

As stated above, the invention also relates to the use in medicine of the aforementioned complex of the invention.

The present invention provides a pharmaceutical composition containing as an active ingredient a peptide defined in the claims in admixture with a solid, liquid or liquefied gaseous diluent.

The invention further provides a pharmaceutical composition containing as an active ingredient a peptide of the invention in the form of a sterile and/or physiologically isotonic aqueous solution.

The invention also provides a medicament in dosage unit form comprising the peptide of the invention.

The invention also provides a medicament in the form of tablets (including lozenges and granules), dragees, capsules, caplets, pills, ampoules or suppositories comprising the complex of the invention.

"Medicament" as used herein means physically discrete coherent portions suitable for medical administration. "Medicament in dosage unit form" as used herein means physically discrete coherent units suitable for medical administration, each containing a daily dose or a multiple or a sum-multiple of a daily dose of the compound of the invention in association with a carrier and/or enclosed within an envelope.

15

The pharmaceutical compositions according to the invention may, for example, take the form of suspensions, solutions and emulsions of the active ingredient in aqueous or non-aqueous diluents, syrups, granulates or powders.

The diluents to be used in pharmaceutical compositions (e.g., granulates) adapted to be formed into tablets, dragees, capsules, caplets and pills include the following: (a) fillers and extenders, (b) binding agents, (c) moisturizing agents, (d) disintegrating agents, (e) agents for retarding dissolution, (f) resorption accelerators, (g) surface active agents, (h) adsorptive carriers, (i) lubricants.

The tablets, dragees, capsules, caplets and pills formed from the pharmaceutical compositions of the invention can have the customary coatings, envelopes and protective matrices, which may contain opacifiers. They can be so constituted that they release the active ingredient only or preferably in a particular part of the intestinal tract, possibly over a period of time.

The active ingredient can also be made up in microencapsulated form together, with one or several of the above-mentioned diluents.

For parenteral administration, solutions and emulsions should be sterile and, if appropriate, blood-isotonic.

In addition to the peptides of the invention, the pharmaceutical compositions and medicaments according to the invention can also contain other pharmaceutically active compounds.

The discrete coherent portions constituting the medicament according to the invention will generally be adapted by virtue of their shape or packaging for medical administration and may be, for example, any of the following: tablets (including lozenges and granulates), pills, dragees, capsules, caplets, suppositories and ampoules. Some of these forms may be made up for delayed release of the active ingredient. Some, such as capsules, may include a protective envelope which renders the portions of the medicament physically discrete and coherent.

The product of the above-mentioned pharmaceutical compositions and medicaments is carried out by any method known in the art, for example, by mixing the active ingredient(s) with the diluent(s) to form a pharmaceutical composition (e.g., a granulate) and then forming the composition into the medicament (e.g., tablets).

It is envisaged that the active complex will be administered perorally, parenterally (for example, intramuscularly, intraperitoneally, subcutaneously or intravenously), rectally or locally.

## Examples

### Examples 1 to 8: Preparation of LV7 cDNA Which Codes for CEA-A

### Example 1: RNA Preparation

Tumor messenger RNA was prepared by the proteinase K-phenol extraction method of J. Favolaro, E. Treisman and R. Kamen, Methods in Enzymology, 65, 718, Academic Press, Inc., (1980), followed by oligo dT cellulose chromatography to yield poly A+ RNA (3'-polyadenylated eucaryotic RNA containing most mRNA sequences that can be translated into polypeptides). To obtain approximately 1.2 mg of poly A+ RNA, approximately $5 \times 10^9$ LoVo cells (ATCC CCL 229) were harvested from 100 x 850 $cm^3$ roller bottles after late logarithmic growth. Cells were lysed by homogenization in an ice-cold solution of 140 mM NaCl, 1.5 mm $MgCl_2$, 10 mM Tris-HCl, pH 8, 0.5% NP40®, 4 mM dithiothreitol and 20 units of placental ribonucease inhibitor/ml. Sodium deoxycholate was then added to 0.2%. Cytoplasm and nuclei were separated by centrifugation of the homogenate at 12,000xg for 20 minutes. The cytoplasmic fraction was mixed with an equal volume of 0.2 M Tris-HCl, pH 7.8, 25 mM EDTA, 0.3 M NaCl, 2% sodium dodecyl sulfate and 400 $\mu$g/ml of proteinase K, incubated for 1 hour at 37°C, then extracted once with an equal volume of phenol/chloroform (1:1/v:v) solution. Nucleic acids were obtained by ethanol precipitation of the separated aqueous phase. Total RNA was enriched for poly A+ RNA by passage in 0.5 M NaCl, 10 mM Tris-HCl, pH 7.8, 0.1% sarcosyl through an oligo dT(12-18) cellulose column. After washing, bound RNA was eluted in the same solution without sodium chloride.

### Example 2: Reverse Transcription of mRNA

Five micrograms of poly A+ LoVo RNA were primed for reverse transcription with oligo dT(12-18). Fifty microliter reactions were performed for 90 minutes at 42°C with 75 units AMV reverse transcriptase (Life Sciences, Inc., St. Petersburg, Florida, U.S.A.). RNA was removed by alkaline hydrolysis, and single-stranded cDNA was made double-stranded and blunt-ended by incubation with the large fragment (Klenow) of DNA polymerase I and T$_4$ DNA polymerase, respectively.

Example 3: Cloning of pLV7 (plasmid LV7)

Synthetic EcoRI DNA linkers (5'pGGAATTCC 3') were attached to the ends of cDNA prepared as described in Example 2 by blunt-end ligation with excess T$_4$ DNA ligase (20 Weiss units). Excess linkers were removed by gel permeation chromatography through "SEPHADEX" G-50 (medium), EcoRI sticky ends were generated by cleavage with EcoRI restriction enzyme and fractionation by agarose exclusion chromatography on A15m (BioRad, Laboratories, Richmond, California, U.S.A.). DNA fragments greater than 500 bp were selected after sizing on agarose gels and were precipitated with 95% ethanol. DNA was resuspended in a small volume of 10 mM Tris-HCl, pH 7.8, 1 mM EDTA and added to an equimolar amount of 5' phosphatased and EcoRI terminated arms of lambda gt11 (Promega Biotech, Madison, Wisconsin, U.S.A.). This phage has the advantageous property that foreign DNAs inserted at the EcoRI site of lambda gt11 are translated as part of the beta-galactosidase fusion protein that can be screened for immunoreactivity with antibodies to CEA. DNAs were ligated for 24 hours at a concentration of 100 to 200 $\mu$g/ml in the presence of T$_4$ DNA ligase. Three microliters of ligated DNA were added to an in vitro lambda packaging mix (Stratagene, San Diego, California, U.S.A.) and packaged particles were assayed by infecting E. coli host Y1088 (ATCC 37195). Of four million phage, 1.2 million were determined by beta- galactosidase complementation to have cDNA inserts in them.

Example 4: Screening of Fusion Polypeptides by Immunoblotting

Fifty thousand phage were plated on E. coli Y1090 (ATCC 37197) for screening on each of twenty 150 mm dishes containing LB-broth with 10 mM MgSO$_4$ and 100 $\mu$g/ml of ampicillin. In some cases, phage stocks were prepared by amplification and titering prior to screening. Phage were grown lytically for 4 hours at 42°C, then nitrocellulose circles impregnated with 10 mM IPTG (isopropyl thiogalactoside) were placed on the surface of the dishes and incubated an additional 2 hours at 37°C. During this period, fusion protein synthesis was induced and proteins which were absorbed to the nitrocellulose matrix were screened in a modified ELISA format. In applicants' library some LoVo fusion proteins may express a portion of a CEA or CEA-related epitope that can be recognized by appropriate antibodies. Applicants took advantage of commercially-available antisera directed against native CEA (180 kd) and of in-house prepared antisera against reduced and alkylated CEA to detect antigens on the filters. These rabbit polyclonal antisera were diluted in PBS/T (phosphate buffered saline (50 mM Na phosphate, pH 7.3, (50 mM NaCl) containing 0.05% "TWEEN-20®" and 0.01% thimerosal) and incubated for two hours to allow recognition of proteins adhering to nitrocellulose circles. Excess antibody was removed and rabbit IgG molecules bound to fusion proteins were detected by mouse anti-rabbit IgG antibody conjugated with alkaline phosphatase. Color detection was in the presence of 5-bromo-4-chloro-3-indolyl phosphate and nitroblue tetrazolium. Darkly staining plaque images were marked and keyed with the master phage plate for retrieval of potential positive areas. The phage continuing to express anti-CEA reactive peptides after repeated dilution and screening by immunoblotting and amplification of the assay were used to prepare DNA.

Example 5: DNA Manipulation

Phage DNA was prepared according to T. Maniatis; E.F. Fritsch and J. Sambrook, Molecular Cloning, A Laboratory Manual, Cold Spring Harbor, (1982). DNA segments were isolated from low melting agarose gels and inserted for subcloning in Bluescribe plasmid vectors (Stratagene, San Diego, California, U.S.A.). DNA sequencing was performed by the dideoxy termination method of F. Sanger, s. Nicklen and A.R. Coolson, Proc. Natl. Acad. Sci. (U.S.A.), 74, 5463-5467, (1977).

Example 6: Detection of CEA Reactivity in lambda cLV7 (cDNA LV7) by Enzyme Immunoassay

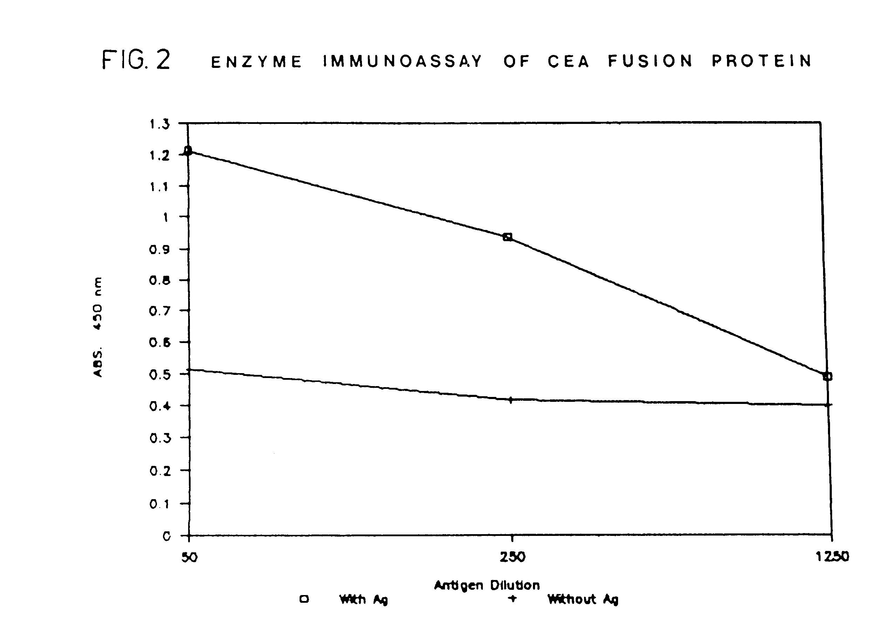

Lambda cLV7 were induced in E. coli Y 1089 for 20 minutes with isopropyl thiogalactoside and grown for 1 hour at 37°C . Cells were centrifuged for 5 minutes in a Beckman Microfuge at room temperature. The cells were resuspended in 1% of the original volume in 50 mM Tris HC1, pH 7.5, containing 10 $\mu$g/ml of leupeptin and pepstatin, 10 mM EDTA, 1 mM phenylmethyl sulfonylfluoride and 0.01% thimerosal. The cells were frozen at -80°C, thawed and sonicated in a for 6 minutes at 0°C. The sonicated suspension was centrifuged and the supernatant fluid was assayed for CEA immunoreactivity by enzyme linked immunoassay. A Linbro 96 well microtiter plate was coated with 400 ng of mouse monoclonal anti-beta galactosidase

17

EP 0 263 933 B1

antibody overnite at 4°C. The unbound antibody was washed away and the plate was blocked with PBS/T (phosphate buffered saline (50 mM Na phosphate, pH 7.3, 150 mM NaCl) containing 0.05% "TWEEN-20"® and 0.01% thimerosal) for 3 hours at room temperature. The supernatant from the sonicated cells was diluted as in PBS/T and 100 μl of the diluted antigen was added to the antibody-coated wells. After incubation for 2 hours at room temperature, unbound antigen was washed away and diluted rabbit anti-CEA antibody was added in 100 μl of PBS/T. The plate was then incubated for 2 hours at room temperature. After washing, each well received 100 μl of horseradish peroxidase-conjugated IgG from goat anti-rabbit IgG in PBS/T. After incubation for 2 hours at room temperature, the wells were washed and peroxidase substrate (3,3,5,5'-tetramethylbenzidine and hydrogen peroxide) was added for 5 minutes. The color development reaction was stopped by addition of 50 μl of 8 M sulfuric acid. An absorbance of 450 nm was determined for each well. The open squares in Figure 2 represent wells receiving fusion protein antigen as described above. The + data points represent control wells treated as above, but receiving 100 μl of PBS/T instead of diluted fusion protein antigen.

Example 7: Immunochemical Identification of lambda LV7 Fusion Protein

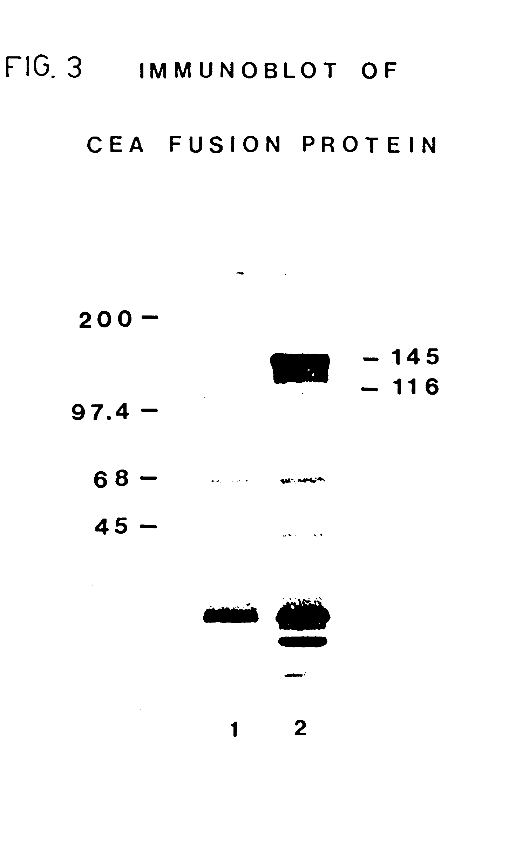

E. coli Y1089 lysate containing fusion protein was run on SDS-PAGE according to the method of U.K. Laemmli, Nature (London), 227, 680-685 (1970). After electrophoresis on 10% acrylamide SDS-PAGE gels, proteins were electrophoretically transferred to nitrocellulose. After transfer, the filter was blocked in PBS/T. The immunoblot was developed by sequential incubation with rabbit anti-CEA and horseradish peroxidase-conjugated IgG from goat anti-rabbit IgG in PBS/T. After washing, the transfer was incubated with 4-chloro-1-naphthol to visualize the bands. Lane 1 in Figure 3 is a control, namely, E. coli lysate without any phage. Lane 2 in Figure 3 is a lysate containing lambda cLV7 fusion protein. The numbers on the left of Figure 3 indicate the mobilities and molecular weights (in kilodaltons) of protein standards. The numbers on the right of Figure 3 indicate the calculated molecular weight and mobility of lambda LV7 fusion protein (upper marker) and of E. coli beta-galactosidase subunit (lower marker).

Example 8: Detection of cLV7-specific poly A+ RNA

Cytoplasmic poly A+ RNA was prepared from LoVo tumor cells and from the lymphoblastoid line GM1989 (control cell line). Five micrograms of each RNA was denatured and electrophoresed in a 1% agarose-2.2M formaldehyde gel and then transferred to nitrocellulose paper. Transfer blots were then challenged with $^{32}$P-radiolabelled cLV7 DNA in 2XSSPE, 5X Denhardt's, 50 μg/ml denatured salmon sperm DNA at 68°C for 18 hours. Blots were washed in 0.2X SSPE, 0.25% SDS at 688°C for 2 hours, then autoradiographed overnight on "KODAK®" X-AR film with two intensifying screens. RNA size markers (BRL, Inc. Gaithersburg, Maryland, U.S.A.) were co-electrophoresed in adjacent wells of the agarose gel and visualized by staining with 0.04% methylene blue.

Results:

Of nearly one million independently derived LoVo cDNA molecules inserted into the lambda gt11 expression system and screened as polypeptides for anti-CEA immunoreactivity, two positive clones were isolated. Inserts were subcloned into the EcoRI site of Bluescribe (+) plasmid cloning vector and one of these, designated, pcLV7, was analyzed further. Deposited with the American Type Culture Collection ("ATCC") on July 30, 1986 was a plasmid in Escherichia Coli containing pcLV7, ATCC No. 67169. By DNA sequence analysis, the insert size of this clone is 859 bp and its sequence is shown in Fig. 1. The upper line represents the nucleotide sequence of the open reading frame of pcLV7. Below it is the peptide sequence for which it codes. The general term used to designate the cDNA clone herein is cLV7 or LV7. When appended to this term; the prefixes lambda- or p- refer, respectively, to this clone as inserted into lambda phage or in plasmid.

Examples 9 to 13: Preparation of 1LV7 cDNA Which Codes for CEA-B

Example 9: RNA Preparation

The same procedure of Example 1 for LV7 cDNA was followed for 1LV7 cDNA.

18

Example 10: Reverse Transcription of mRNA

Fifty micrograms of poly A + RNA were primed for reverse transcription with oligo $dt_{(12-18)}$ and random deoxyhexamers. The 350 microliter reaction was incubated for 2.5 hours at 42°C with 900 units of AMV reverse transcriptase (Life Sciences Inc., St. Petersburg, Florida, U.S.A.). The RNA component of the cDNA/RNA hybrids was then replaced with the cDNA complementary strand by treatment with RNase H, E. coli DNA polymerase I and E. coli DNA ligase at 12°C and 22°C for 1.5 hours each. Molecular ends were polished by treatment with $T_4$ DNA polymerase.

Example 11: Cloning of p1LV7

In order to protect EcoRI sites internal to the newly-synthesized cDNA from subsequent digestions steps, total cDNA was subjected to treatment with EcoRI methylase in the presence of 80 $\mu$M S-adenosyl methionine for 1 hour at 37°C. THe DNA was then size-fractionated by electrophoresis through a 1% low melting point agarose gel. cDNA segments in the size range of 2 to 5 kb were excised, extracted from the gel slice and incubated in the presence of a 100-fold molar excess of synthetic EcoRI linkers (5' pdGGAATTCC 3') with $T_4$ DNA ligase. Excess linkers were removed by gel permeation chromatography through Sephadex® G-50(medium) and EcoRI sticky ends were generated by cleavage with EcoRI restriction enzyme. EcoRI terminated cDNA segments were incubated with EcoRI cleaved and phosphatase arms of bacteriophage vector lambda gt11 in the presence of $T_4$ DNA ligase at 5°C overnight. Aliquots of the ligation mixture were then mixed with in vitro packaging extracts (Stratagene, San Diego, California, U.S.A.) and phage particles were titrated on E. coli Y1090 cells.

Example 12: Screening of Recombinants by Hybridization

Fifty thousand phage (pfu) from the in vitro packaged library were plated on each of four 150 mm LB plates containing 1.4% agar, 10 mM $MgSO_4$ and 100 $\mu$g/ml ampicillin. Phage were permitted to lyse host cells until plaque sizes were 0.2 to 0.5 mm in diameter. Plates were then cooled and nitrocellulose filter replicas were prepared by the method of W. D. Benton and R. W. Davis, "Screening Lambda GT Recombinant Clones by Hybridization to Single Plaques In Situ", Science, 196, 180-182, (1977). Filters were prehybridized and hybridized in 2X SSPE, 5X Denhardt's, 0.1% SDS, 100 ug/ml of denatured salmon sperm DNA and for the hybridization step, 2 ng/ml of $^{32}$P-labelled cLV7 insert DNA. Non-specific DNA hybridization was removed by washing filters in 0.5X SSPE, 0.25% SDS. Positive plaques were detected by autoradiography, picked and screened for two additional rounds of positive hybridization with radiolabeled probe.

Example 13: DNA Manipulation

Inserts from the positive plaques from Example 12 were introduced into a bacterial plasmid vector, and exonuclease III-generated double stranded segments were sequenced by the dideoxy chain termination method. DNA sequences were computer-analyzed using Pustell DNA sequence analysis programs (IBI International, New Haven, Connecticut, U.S.A).

Results:

Of nearly two hundred thousand independently derived LoVo cDNA molecules inserted into lambda gt11 vector and screened for reactivity with a radiolabeled LV7 probe, forty positive phage were selected. The largest of these (approximately 3Kb) was sequenced and its nucleic acid and protein sequence is given in Fig. 5.

Deposited with the ATCC on February 6, 1987 was a plasmid in Escherichia Coli containing Pc1LV7, ATCC No. 67312.

By DNA sequence analysis, the insert size of this clone is 2839 bp and its sequence is shown in Fig. 5. The upper line represents the nucleotide sequence of the open reading frame of pc1LV7. Below it is the peptide sequence for which it codes. The general term used to designate the cDNA clone herein is c1LV7 or 1LV7. When appended to this term, the prefixes lambda- or p-refer, respectively, to this clone as inserted into lambda phage or in plasmid.

EP 0 263 933 B1

## Example 14: Cloning and Sequencing of cFL-CEA Which Codes for CEA-C

A recombinant library containing 2 X 10⁵ independent inserts of fetal liver cDNA in bacteriophage vector lambda gt11 (Clontech, Palo Alto, California, U.S.A) was screened with radiolabeled LV7 cDNA according to W. D. Benton and R. W. Davis, Science, 196, 180-182, (1977). A single positive clone was selected and the EcoRI insert was subcloned into plasmid vector Bluescript KS + (Stratagene, San Diego, California, U.S.A). The deletion clones were prepared in both directions and sequenced by the dideoxy chain termination method of F. Sanger, S. Nicklen and A. R. Coulson, Proc. Natl. Acad, Sci., U.S.A., 74, 5463-5647, (1977). This clone, named FL5, was incomplete as judged by the lack of a translation termination signal. A single copy probe derived from the 3' end of FL5 was used to rescreen the commercial fetal liver library described above. Five positive clones were obtained, inserts were cloned into Bluescript KS + vector, and the longest, named FL4, was sequenced. These two cDNA plasmids, FL5 and FL4 overlap and together contain the entire open reading frame of the polypeptide, designated herein as FL-CEA. The translated sequence of FL-CEA is as follows:

EP 0 263 933 B1

```
      460         470         480         490         500         510
       ↓           ↓           ↓           ↓           ↓           ↓
ATA AAG TCA GAT CTT GTG AAT GAA GAA GCA ACT GGA CAG TTC CAT GTA TAC CCG GAG
Ile Lys Ser Asp Leu Val Asn Glu Glu Ala Thr Gly Gln Phe His Val Tyr Pro Glu
 91  92  93  94  95  96  97  98  99 100 101 102 103 104 105 106 107 108 109

      520         530         540         550         560         570
       ↓           ↓           ↓           ↓           ↓           ↓
CTG CCC AAG CCC TCC ATC TCC AGC AAC AAC TCC AAC CCT GTG GAG GAC AAG GAT GCT
Leu Pro Lys Pro Ser Ile Ser Ser Asn Asn Ser Asn Pro Val Glu Asp Lys Asp Ala
110 111 112 113 114 115 116 117 118 119 120 121 122 123 124 125 126 127 128

      580         590         600         610         620
       ↓           ↓           ↓           ↓           ↓
GTG GCC TTC ACC TGT GAA CCT GAG ACT CAG GAC ACA ACC TAC CTG TGG TGG ATA AAC
Val Ala Phe Thr Cys Glu Pro Glu Thr Gln Asp Thr Thr Tyr Leu Trp Trp Ile Asn
129 130 131 132 133 134 135 136 137 138 139 140 141 142 143 144 145 146 147

      630         640         650         660         670         680
       ↓           ↓           ↓           ↓           ↓           ↓
AAT CAG AGC CTC CCG GTC AGT CCC AGG CTG CAG CTG TCC AAT GGC AAC AGG ACC CTC
Asn Gln Ser Leu Pro Val Ser Pro Arg Leu Gln Leu Ser Asn Gly Asn Arg Thr Leu
148 149 150 151 152 153 154 155 156 157 158 159 160 161 162 163 164 165 166

      690         700         710         720         730         740
       ↓           ↓           ↓           ↓           ↓           ↓
ACT CTA CTC AGT GTC ACA AGG AAT GAC ACA GGA CCC TAT GAG TGT GAA ATA CAG AAC
Thr Leu Leu Ser Val Thr Arg Asn Asp Thr Gly Pro Tyr Glu Cys Glu Ile Gln Asn
167 168 169 170 171 172 173 174 175 176 177 178 179 180 181 182 183 184 185

      750         760         770         780         790         800
       ↓           ↓           ↓           ↓           ↓           ↓
CCA GTG AGT GCG AAC CGC AGT GAC CCA GTC ACC TTG AAT GTC ACC TAT GGC CCG GAC
Pro Val Ser Ala Asn Arg Ser Asp Pro Val Thr Leu Asn Val Thr Tyr Gly Pro Asp
186 187 188 189 190 191 192 193 194 195 196 197 198 199 200 201 202 203 204

      810         820         830         840         850
       ↓           ↓           ↓           ↓           ↓
ACC CCC ACC ATT TCC CCT TCA GAC ACC TAT TAC CGT CCA GGG GCA AAC CTC AGC CTC
Thr Pro Thr Ile Ser Pro Ser Asp Thr Tyr Tyr Arg Pro Gly Ala Asn Leu Ser Leu
205 206 207 208 209 210 211 212 213 214 215 216 217 218 219 220 221 222 223

      860         870         880         890         900         910
       ↓           ↓           ↓           ↓           ↓           ↓
TCC TGC TAT GCA GCC TCT AAC CCA CCT GCA CAG TAC TCC TGG CTT ATC AAT GGA ACA
Ser Cys Tyr Ala Ala Ser Asn Pro Pro Ala Gln Tyr Ser Trp Leu Ile Asn Gly Thr
224 225 226 227 228 229 230 231 232 233 234 235 236 237 238 239 240 241 242

      920         930         940         950         960         970
       ↓           ↓           ↓           ↓           ↓           ↓
TTC CAG CAA AGC ACA CAA GAG CTC TTT ATC CCT AAC ATC ACT GTG AAT AAT AGT GGA
Phe Gln Gln Ser Thr Gln Glu Leu Phe Ile Pro Asn Ile Thr Val Asn Asn Ser Gly
243 244 245 246 247 248 249 250 251 252 253 254 255 256 257 258 259 260 261

      980         990        1000        1010        1020
       ↓           ↓           ↓           ↓           ↓
TCC TAT ACC TGC CAC GCC AAT AAC TCA GTC ACT GGC TGC AAC AGG ACC ACA GTC AAG
Ser Tyr Thr Cys His Ala Asn Asn Ser Val Thr Gly Cys Asn Arg Thr Thr Val Lys
262 263 264 265 266 267 268 269 270 271 272 273 274 275 276 277 278 279 280

     1030        1040        1050        1060        1070        1080
       ↓           ↓           ↓           ↓           ↓           ↓
ACG ATC ATA GTC ACT GAG CTA AGT CCA GTA GTA GCA AAG CCC CAA ATC AAA GCC AGC
Thr Ile Ile Val Thr Glu Leu Ser Pro Val Val Ala Lys Pro Gln Ile Lys Ala Ser
281 282 283 284 285 286 287 288 289 290 291 292 293 294 295 296 297 298 299
```

21

```
                1090          1100          1110          1120·         1130          1140
                 •            •             •             •             •             •
        AAG ACC ACA GTC ACA GGA GAT AAG GAC TCT GTG AAC CTG ACC TGC TCC ACA AAT GAC
        Lys Thr Thr Val Thr Gly Asp Lys Asp Ser Val Asn Leu Thr Cys Ser Thr Asn Asp
        300 301 302 303 304 305 306 307 308 309 310 311 312 313 314 315 316 317 318

                  1150          1160          1170          1180          1190
                   •             •             •             •             •
        ACT GGA ATC TCC ATC CGT TGG TTC TTC AAA AAC CAG AGT CTC CCG TCC TCG GAG AGG
        Thr Gly Ile Ser Ile Arg Trp Phe Phe Lys Asn Gln Ser Leu Pro Ser Ser Glu Arg
        319 320 321 322 323 324 325 326 327 328 329 330 331 332 333 334 335 336 337

        1200          1210          1220          1230          1240          1250
          •             •             •             •             •             •
        ATG AAG CTG TCC CAG GGC AAC ACC ACC CTC AGC ATA AAC CCT GTC AAG AGG GAG GAT
        Met Lys Leu Ser Gln Gly Asn Thr Thr Leu Ser Ile Asn Pro Val Lys Arg Glu Asp
        338 339 340 341 342 343 344 345 346 347 348 349 350 351 352 353 354 355 356

            1260          1270          1280          1290          1300          1310
              •             •             •             •             •             •
        GCT GGG ACG TAT TGG TGT GAG GTC TTA ACC CAA TCA GTA AGA ACC AAA GCG ACC CCA
        Ala Gly Thr Tyr Trp Cys Glu Val Leu Thr Gln Ser Val Arg Thr Lys Ala Thr Pro
        357 358 359 360 361 362 363 364 365 366 367 368 369 370 371 372 373 374 375

                1320          1330          1340          1350          1360          1370
                  •             •             •             •             •             •
        TCA TGG CTG AAC GTA AAC TAT AAT GCT CTA CCA CAA GAA AAT GGC CTC TCA CCT GGG
        Ser Trp Leu Asn Val Asn Tyr Asn Ala Leu Pro Gln Glu Asn Gly Leu Ser Pro Gly
        376 377 378 379 380 381 382 383 384 385 386 387 388 389 390 391 392 393 394

                    1380          1390          1400          1410          1420
                      •             •             •             •             •
        GCC ATT GCT GGC ATT GTG ATT GGA GTA GTG GCC CTG GTT GCT CTG ATA GCA GTA GCC
        Ala Ile Ala Gly Ile Val Ile Gly Val Val Ala Leu Val Ala Leu Ile Ala Val Ala
        395 396 397 398 399 400 401 402 403 404 405 406 407 408 409 410 411 412 413

        1430          1440          1450          1460          1470          1480
          •             •             •             •             •             •
        CTG GCA TGT TTT CTG CAT TTC GGG AAG ACC GGC AGG GCA AGC GAC CAG CGT GAT CTC
        Leu Ala Cys Phe Leu His Phe Gly Lys Thr Gly Arg Ala Ser Asp Gln Arg Asp Leu
        414 415 416 417 418 419 420 421 422 423 424 425 426 427 428 429 430 431 432

            1490          1500          1510          1520          1530          1540
              •             •             •             •             •             •
        ACA GAG CAC AAA CCC TCA GTC TCC AAC CAC ACT CAG GAC CAC TCC AAT GAC CCA CCT
        Thr Glu His Lys Pro Ser Val Ser Asn His Thr Gln Asp His Ser Asn Asp Pro Pro
        433 434 435 436 437 438 439 440 441 442 443 444 445 446 447 448 449 450 451

                1550          1560          1570          1580          1590
                  •             •             •             •             •
        AAC AAG ATG AAT GAA GTT ACT TAT TCT ACC CTG AAC TTT GAA GCC CAG CAA CCC ACA
        Asn Lys Met Asn Glu Val Thr Tyr Ser Thr Leu Asn Phe Glu Ala Gln Gln Pro Thr
        452 453 454 455 456 457 458 459 460 461 462 463 464 465 466 467 468 469 470

        1600          1610          1620          1630          1640          1650
          •             •             •             •             •             •
        CAA CCA ACT TCA GCC TCC CCA TCC CTA ACA GCC ACA GAA ATA ATT TAT TCA GAA GTA
        Gln Pro Thr Ser Ala Ser Pro Ser Leu Thr Ala Thr Glu Ile Ile Tyr Ser Glu Val
        471 472 473 474 475 476 477 478 479 480 481 482 483 484 485 486 487 488 489
```

22

```
       1660        1670        1680        1690        1700        1710
        !           !           !           !           !           !
AAA AAG CAG TAA TGA AAC CTG TCC TGC TCA CTG CAG TGC TGA TGT ATT TCA AGT CTC
Lys Lys Gln ---
490 491 492

          1720        1730        1740        1750        1760
           !           !           !           !           !
TCA CCC TCA TCA CTA GGA GAT TCC TTT CCC CTG TAG GGG TAG AGG GGT GGG GAC AGA


1770        1780        1790        1800        1810        1820
 !           !           !           !           !           !
AAC AGC TTT CTC CTA CTC TTC CTT CCT AAT AGG CAT CTC CAG GCT GCC TGG TCA CTG


    1830        1840        1850        1860        1870        1880
     !           !           !           !           !           !
CCC CTC TCT CAG TGT CAA TAG ATG AAA GTA CAT TGG GAG TCT GTA GGA AAC CCA ACC


       1890        1900        1910        1920        1930        1940
        !           !           !           !           !           !
TTC TTG TCA TTG AAA TTT GGC AAA GCT GAC TTT GGG AAA GAG GGA CCA GAA CTT CCC


          1950        1960        1970        1980        1990
           !           !           !           !           !
CTC CCT TCC CCT TTT CCC AAC CTG GAC TTG TTT TAA ACT TGC CTG TTC AGA GCA CTC


2000        2010        2020        2030        2040        2050
 !           !           !           !           !           !
ATT CCT TCC CAC CCC CAG TCC TGT CCT ATC ACT CTA ATT CGG ATT TGC CAT AGC CTT


    2060        2070        2080        2090        2100        2110
     !           !           !           !           !           !
GAG GTT ATG TCC TTT TCC ATT AAG TAC ATG TGC CAG GAA ACA GCG AGA GAG AGA AAG


       2120        2130        2140        2150        2160
        !           !           !           !           !
TAA ACG GCA GTA ATG CTT CTC CTA TTT CTC CAA AGC CTT GTG TGA ACT AGC AAA GAG


2170        2180        2190        2200        2210        2220
 !           !           !           !           !           !
AAG AAA ACC AAA TAT ATA ACC AAT AGT GAA ATG CCA CAG GTT TGT CCA CTG TCA GGG


    2230        2240        2250        2260        2270        2280
     !           !           !           !           !           !
TTG TCT ACC TGT AGG ATC AGG GTC TAA GCA CCT TGG TGC TTA GCT AGA ATA CCA CCT
```

```
        2290          2300          2310          2320          2330
         ↓             ↓             ↓             ↓             ↓
   AAT CCT TCT GGC AAG CCT GTC TTC AGA GAA CCC ACT AGA AGC AAC TAG GAA AAA TCA


       2340          2350          2360          2370          2380          2390
        ↓             ↓             ↓             ↓             ↓             ↓
   CTT GCC AAA ATC CAA GGC AAT TCC TGA TGG AAA ATG CAA AAG CAC ATA TAT GTT TTA


       2400          2410          2420
        ↓             ↓             ↓
   ATA TCT TTA TGG GCT CTG TTC AAG GCA GTG CTG
```

For residue sequence purposes, the first amino acid is Gln which is under nucleic acid 190. Thereafter the amino acids are numbered consecutiveiy.

Examples 15 to 19

Cloning and Sequencing of cBT-20 Which Codes for CEA-D

Example 15: RNA Preparation

Tumor messenger RNA was prepared by the proteinase K-phenol extraction method of Favolaro, Treisman and Kamen, Methods in Enzymology, 65, 718, (1980), followed by oligo dT-cellulose chromatography to yield poly A+ RNA. To obtain approximately 100 micrograms of pA+ RNA, 1 gram (wet weight) of BT-20 cells (ATCC HTB 19) were resuspended and lysed in 20 ml ice-cold RNA lysis buffer (140 mM NaCl, 1.5 mM $MgCl_2$, 10 mM Tris-HCl, pH 7.8, 0.5% NP-40 4 mM 2-mercatoethanol and 20 units of placental ribonuclease inhibitor per ml). Sodium desoxycholate was added to 0.2%. Cytoplasm and nuclei were separated by centrifugation of the homogenate at 12,000xg for 20 minutes. The cytoplasmic fraction was mixed with an equal volume of PK buffer (0.2 M Tris-HCl, pH 7.8, 25 mM EDTA, 0.3 M NaCl, 2% sodium dodecyl sulfate and 400 $\mu$g of proteinase K per ml), incubated for 2 hours at 37°C, then extracted once with an equal volume of phenol/chloroform (1:1/v:v) solution. Nucleic acids were obtained by ethanol precipitation of the separated aqueous phase. Total RNA was enriched for poly A+ RNA by passage in 0.5 M NaCl, 10 mM Tris-HCl, pH 7.8, 0.1% sarcosyl through an oligo dT (12-18) cellulose column. After washing, bound RNA was eluted in the same solution without sodium chloride.

Example 16: Reverse Transcription of mRNA

Ten micrograms of poly A+ BT-20 RNA were primed for reverse transcription with oligo dT (12-18) and pdN₆ primers. One hundred microliter reaction was performed for 4 hours at 42°C with 200 units of AMV reverse transcriptase (Life Sciences, Inc., St. Petersburg, Florida, U.S.A.). The RNA component of the cDNA/mRNA hybrids was replaced with the second complementary strand by treatment with RNase H, E. coli DNA polymerase I and E. coli DNA ligase at 12°C and 22°C for 1.5 hours each. Molecular ends were polished by treatment with T₄ DNA polymerase. cDNA was phenol/chloroform extracted, ethanol precipitated and purified over a "SEPHADEX G-50" spun column prepared in 10 mM Tris-HCl, pH 7.8, 1 mM EDTA (TE).

Example 17: Cloning of cBT20

Synthetic DNA linkers

$$\left( \begin{array}{l} 5\text{'pCCCGGG} \qquad 3\text{'} \\ 3\text{' GGGCCCTTAA5'} \end{array} \right)$$

24

EP 0 263 933 B1

were attached to the ends of cDNA by blunt end ligation with excess $T_4$ DNA ligase. Excess linkers were removed by chromatography through "SEPHADEX® G-50" (medium) in TE, and by fractionation on 0.8% low melting agarose gel. Based on a Northern blot analysis of poly A + RNA of the BT-20 cell line, the size of the CEA-related mRNA was estimated at 3.0 kb. Therefore, cDNA fragments between 2 and 4 kb were recovered from gel slices and fragments were ethanol precipitated. After resuspension of cDNA in TE, EcoRI-cleaved lambda gt10 arms were added to cDNA at an estimated molar ratio of 1:1. Ligation proceeded at 7°C for 2 days in the presence of $T_4$ DNA ligase. Aliquots of the ligation reaction were added to commercially-obtained packaging mix (Stratagene, San Diego, California, U.S.A.) for preparation of lambda particles. Five million phage particles were obtained after in vitro packaging and infection of E. coli host NM514.

Example 18: Screening of Recombinant Library

Five hundred thousand packaged lambda particles were plated on lawns of E. coli NM514 and replicate patterns were lifted onto nitrocellulose sheets as described by W. D. Benton and R. W. Davis, Science, 196, 180-182, (1977). Positive phage were selected by hybridization with $^{32}$P-labeled LV7 cDNA insert probe that contained a domain repeated among various CEA family. members. By this selection method, twenty positive phage were obtained after multiple rounds of screening. Phage from individual plaques were amplified and titered, and these were used to prepare small quantities of recombinant phage DNA.

Example 19: DNA Manipulation

Phage DNA was prepared according to T. Maniatis, E. F. Fritsch and J. Sambrook, Molecular Cloning, A Laboratory Manual, Cold Spring Harbor, (1982). DNA segments were isolated from low melting agarose gels and inserted for subcloning into Bluescript plasmid vectors (Stratagene, San Diego, California, U.S.A.) DNA sequencing was performed by the dideoxy termination method of F. Sanger, S. Nicklen and A. R. Cornslon, Proc. Natl. Acad, Sci., U.S.A., 74, 5463-5467, (1977). The translated sequence for BT20 is as follows:

```
              10          20          30          40          50
              ↓           ↓           ↓           ↓           ↓
CC GGG GGA CAC GCA GGG CCA ACA GTC ACA GCA GCC CTG ACC AGA GCA TTC CTG GAG CTC


      60          70          80          90          100         110
      ↓           ↓           ↓           ↓           ↓           ↓
AAG CTC TCT ACA AAG AGG TGG ACA GAG AAG ACA GCA GAG ACC ATG GGA CCC CCC TCA
                                                    Met Gly Pro Pro Ser


      120         130         140         150         160         170
      ↓           ↓           ↓           ↓           ↓           ↓
GCC CCT CCC TGC AGA TTG CAT GTC CCC TGG AAG GAG GTC CTG CTC ACA GCC TCA CTT
Ala Pro Pro Cys Arg Leu His Val Pro Trp Lys Glu Val Leu Leu Thr Ala Ser Leu


      180         190         200         210         220         230
      ↓           ↓           ↓           ↓           ↓           ↓
CTA ACC TTC TGG AAC CCA CCC ACC ACT GCC AAG CTC ACT ATT GAA TCC ACG CCA TTC
Leu Thr Phe Trp Asn Pro Pro Thr Thr Ala Lys Leu Thr Ile Glu Ser Thr Pro Phe


      240         250         260         270         280
      ↓           ↓           ↓           ↓           ↓
AAT GTC GCA GAG GGG AAG GAG GTT CTT CTA CTC GCC CAC AAC CTG CCC CAG AAT CGT
Asn Val Ala Glu Gly Lys Glu Val Leu Leu Leu Ala His Asn Leu Pro Gln Asn Arg
```

25

```
        290           300           310           320           330           340
         •             •             •             •             •             •
ATT GGT TAC AGC TGG TAC AAA GGC GAA AGA GTG GAT GGC AAC AGT CTA ATT GTA GGA
Ile Gly Tyr Ser Trp Tyr Lys Gly Glu Arg Val Asp Gly Asn Ser Leu Ile Val Gly
 27  30  31  32  33  34  35  36  37  38  39  40  41  42  43  44  45  46  47

        350           360           370           380           390           400
         •             •             •             •             •             •
TAT GTA ATA GGA ACT CAA CAA GCT ACC CCA GGG CCC GCA TAC AGT GGT CGA GAG ACA
Tyr Val Ile Gly Thr Gln Gln Ala Thr Pro Gly Pro Ala Tyr Ser Gly Arg Glu Thr
 48  49  50  51  52  53  54  55  56  57  58  59  60  61  62  63  64  65  66

        410           420           430           440           450
         •             •             •             •             •
ATA TAC CCC AAT GCA TCC CTG CTG ATC CAG AAC GTC ACC CAG AAT GAC ACA GGA TTC
Ile Tyr Pro Asn Ala Ser Leu Leu Ile Gln Asn Val Thr Gln Asn Asp Thr Gly Phe
 67  68  69  70  71  72  73  74  75  76  77  78  79  80  81  82  83  84  85

        460           470           480           490           500           510
         •             •             •             •             •             •
TAC ACC CTA CAA GTC ATA AAG TCA GAT CTT GTG AAT GAA GAA GCA ACC GGA CAG TTC
Tyr Thr Leu Gln Val Ile Lys Ser Asp Leu Val Asn Glu Glu Ala Thr Gly Gln Phe
 86  87  88  89  90  91  92  93  94  95  96  97  98  99 100 101 102 103 104

        520           530           540           550           560           570
         •             •             •             •             •             •
CAT GTA TAC CCG GAG CTG CCC AAG CCC TCC ATC TCC AGC AAC AAC TCC AAC CCC GTG
His Val Tyr Pro Glu Leu Pro Lys Pro Ser Ile Ser Ser Asn Asn Ser Asn Pro Val
105 106 107 108 109 110 111 112 113 114 115 116 117 118 119 120 121 122 123

        580           590           600           610           620
         •             •             •             •             •
GAG GAC AAG GAT GCT GTG GCC TTC ACC TGT GAA CCT GAG GTT CAG AAC ACA ACC TAC
Glu Asp Lys Asp Ala Val Ala Phe Thr Cys Glu Pro Glu Val Gln Asn Thr Thr Tyr
124 125 126 127 128 129 130 131 132 133 134 135 136 137 138 139 140 141 142

        630           640           650           660           670           680
         •             •             •             •             •             •
CTG TGG TGG GTA AAT GGT CAG AGC CTC CCG GTC AGT CCC AGG CTG CAG CTG TCC AAT
Leu Trp Trp Val Asn Gly Gln Ser Leu Pro Val Ser Pro Arg Leu Gln Leu Ser Asn
143 144 145 146 147 148 149 150 151 152 153 154 155 156 157 158 159 160 161

        690           700           710           720           730           740
         •             •             •             •             •             •
GGC AAC AGG ACC CTC ACT CTA CTC AGC GTC AAA AGG AAC GAT GCA GGA TCG TAT GAA
Gly Asn Arg Thr Leu Thr Leu Leu Ser Val Lys Arg Asn Asp Ala Gly Ser Tyr Glu
162 163 164 165 166 167 168 169 170 171 172 173 174 175 176 177 178 179 180

        750           760           770           780           790           800
         •             •             •             •             •             •
TGT GAA ATA CAG AAC CCA GCG AGT GCC AAC CGC AGT GAC CCA GTC ACC CTG AAT GTC
Cys Glu Ile Gln Asn Pro Ala Ser Ala Asn Arg Ser Asp Pro Val Thr Leu Asn Val
181 182 183 184 185 186 187 188 189 190 191 192 193 194 195 196 197 198 199

        810           820           830           840           850
         •             •             •             •             •
CTC TAT GGC CCA GAT GGC CCC ACC ATT TCC CCC TCA AAG GCC AAT TAC CGT CCA GGG
Leu Tyr Gly Pro Asp Gly Pro Thr Ile Ser Pro Ser Lys Ala Asn Tyr Arg Pro Gly
200 201 202 203 204 205 206 207 208 209 210 211 212 213 214 215 216 217 218

        860           870           880           890           900           910
         •             •             •             •             •             •
GAA AAT CTG AAC CTC TCC TGC CAC GCA GCC TCT AAC CCA CCT GCA CAG TAC TCT TGG
Glu Asn Leu Asn Leu Ser Cys His Ala Ala Ser Asn Pro Pro Ala Gln Tyr Ser Trp
219 220 221 222 223 224 225 226 227 228 229 230 231 232 233 234 235 236 237
```

26

```
          920         930         940         950         960         970
           '           '           '           '           '           '
TTT ATC AAT GGG ACG TTC CAG CAA TCC ACA CAA GAG CTC TTT ATC CCC AAC ATC ACT
Phe Ile Asn Gly Thr Phe Gln Gln Ser Thr Gln Glu Leu Phe Ile Pro Asn Ile Thr
238 239 240 241 242 243 244 245 246 247 248 249 250 251 252 253 254 255 256

          980         990        1000        1010        1020
           '           '           '           '           '
GTG AAT AAT AGC GGA TCC TAT ATG TGC CAA GCC CAT AAC TCA GCC ACT GGC CTC AAT
Val Asn Asn Ser Gly Ser Tyr Met Cys Gln Ala His Asn Ser Ala Thr Gly Leu Asn
257 258 259 260 261 262 263 264 265 266 267 268 269 270 271 272 273 274 275

 1030        1040        1050        1060        1070        1080
   '           '           '           '           '           '
AGG·ACC ACA GTC ACG ATG ATC ACA GTC TCT GGA AGT GCT CCT GTC CTC TCA GCT GTG
Arg Thr Thr Val Thr Met Ile Thr Val Ser Gly Ser Ala Pro Val Leu Ser Ala Val
276 277 278 279 280 281 282 283 284 285 286 287 288 289 290 291 292 293 294

 1090        1100        1110        1120        1130        1140
   '           '           '           '           '           '
GCC ACC GTC GGC ATC ACG ATT GGA GTG CTG GCC AGG GTG GCT CTG ATA TAG CAG CCC
Ala Thr Val Gly Ile Thr Ile Gly Val Leu Ala Arg Val Ala Leu Ile ---
295 296 297 298 299 300 301 302 303 304 305 306 307 308 309 310

         1150        1160        1170        1180        1190
           '           '           '           '           '
TGG TGT ATT TTC GAT ATT TCA GGA AGA CTG GCA GAT TGG ACC AGA CCC TGA ATT CTT


1200        1210        1220        1230        1240        1250
   '           '           '           '           '           '
CTA GCT CCT CCA ATC CCA TTT TAT CCC ATG GAA CCA CTA AAA ACA AGG TCT GCT CTG


 1260        1270        1280        1290        1300        1310
   '           '           '           '           '           '
CTC CTG AAG CCC TAT ATG CTG GAG ATG GAC AAC TCA ATG AAA ATT TAA AGG AAA AAC


 1320        1330        1340        1350        1360        1370
   '           '           '           '           '           '
CCT CAG GCC TGA GGT GTG TGC CAC TCA GAG ACT TCA CCT AAC TAG AGA CAG GCA AAC


 1380        1390        1400        1410        1420
   '           '           '           '           '
TGC AAA CCA nnC CTC TTT CGC TTG GCA GGA TGA TGG TGT CAT TAG TAT TTC ACA AGA


1430        1440        1450        1460        1470        1480
   '           '           '           '           '           '
AGT AGC TTC AGA GGG TAA CTT AAC AGA GTA TCA GAT CTA TCT TGT CAA TCC CAA CGT


 1490        1500        1510        1520        1530        1540
   '           '           '           '           '           '
TTT ACA TAA AAT AAG CGA TCC TTT AGT GCA CCC AGT GAC TGA CAT TAG CAG CAT CTT
```

27

```
        1550        1560        1570        1580     .. 1590
          ‡           ‡           ‡           ‡          ‡
TAA CAC AGC CGT GTG TTC AAG TGT ACA GTG GTC CTT TTC AGA GTT GGn nnT ACT CCA


  1600      1610        1620        1630        1640        1650
   ‡         ‡           ‡           ‡           ‡           ‡
ACT GAA ATG TTA AGG AAG AAG ATA GAT CCA ATT AAA AAA AAT TAA AAC CAA TTT AAA


        1660        1670        1680        1690        1700        1710
          ‡           ‡           ‡           ‡           ‡           ‡.
AAA AAA AAA GAA CAC AGG AGA TTC CAG TCT ACT TGA GTT AGC ATA ATA CAG AAG TCC


        1720        1730        1740        1750        1760         -
          ‡           ‡           ‡           ‡           ‡
CCT CTA CTT TAA CTT TTA CAA AAA AGT AAC CTG AAC TAA TCT GAT GTT AAC CAA TGT


1770      1780.        1790        1800        1810        1820
   ‡         ‡           ‡           ‡           ‡           ‡
ATT TAT TTG TCT GGT TCT GTT TCC TTG TTC CAA TTT GAC AAA ACC CAC TGT TCT TGT


        1830        1840        1850        1860        1870        1880
          ‡           ‡           ‡           ‡           ‡           ‡
ATT GTA TTG CCC AGG GGG AGC TAT CAC TGT ACT TGT AGA GTG GTG CTG CTT TAA GTT


        1890        1900        1910        1920        1930         1940
          ‡           ‡           ‡           ‡           ‡            ‡
CAT AAA TCA CAA ATA AAA GCC AAT TAG CTC TAT AAC TAA AAA AAA AAA AAA AAA AAA


        1950        1960
          ‡           ‡
AAA AAA AAA AAA AAA AAA AAA AAA
```

Note: the "nn" at the positions 1380-1381 and the "nnn" at positions 1589-1591 represent, as yet, unsequenced regions of the 3'-translated region of cBT-20.

For residue sequence purposes, the first amino acid is Lys in row four, 11 amino acids from the left. Thereafter the amino acids are numbered consecutively.

**Claims**

1. An isolated nucleic acid which codes for a polypeptide belonging to the CEA gene family, selected from the sequences listed below, and fragments thereof comprising at least 50 sequential bases:

CEA-A

```
            10           20           30           40           50
             •            •            •            •            •
GG GGT TTA CAC AAC CAC CAC CCC ATC AAA CCC TTC ATC ACC AGC AAC AAC TCC AAC CCC
   Gly Leu His Asn His His Pro Ile Lys Pro Phe Ile Thr Ser Asn Asn Ser Asn Pro

   60           70           80           90          100          110
    •            •            •            •            •            •
GTG GAG GAT GAG GAT GCT GTA GCC TTA ACC TGT GAA CCT GAG ATT CAG AAC ACA ACC
Val Glu Asp Glu Asp Ala Val Ala Leu Thr Cys Glu Pro Glu Ile Gln Asn Thr Thr

     120          130          140          150          160          170
      •            •            •            •            •            •
TAC CTG TGG TGG GTA AAT AAT CAG AGC CTC CCG GTC AGT CCC AGG CTG CAG CTG TCC
Tyr Leu Trp Trp Val Asn Asn Gln Ser Leu Pro Val Ser Pro Arg Leu Gln Leu Ser

        180          190          200          210          220          230
         •            •            •            •            •            •
AAT GAC AAC AGG ACC CTC ACT CTA CTC AGT GTC ACA AGG AAT GAT GTA GGA CCC TAT
Asn Asp Asn Arg Thr Leu Thr Leu Leu Ser Val Thr Arg Asn Asp Val Gly Pro Tyr

           240          250          260          270          280
            •            •            •            •            •
GAG TGT GGA ATC CAG AAC GAA TTA AGT GTT GAC CAC AGC GAC CCA GTC ATC CTG AAT
Glu Cys Gly Ile Gln Asn Glu Leu Ser Val Asp His Ser Asp Pro Val Ile Leu Asn

   290          300          310          320          330          340
    •            •            •            •            •            •
GTC CTC TAT GGC CCA GAC GAC CCC ACC ATT TCC CCC TCA TAC ACC TAT TAC CGT CCA
Val Leu Tyr Gly Pro Asp Asp Pro Thr Ile Ser Pro Ser Tyr Thr Tyr Tyr Arg Pro

      350          360          370          380          390          400
       •            •            •            •            •            •
GGG GTG AAC CTC AGC CTC TCC TGC CAT GCA GCC TCT AAC CCA CCT GCA CAG TAT TCT
Gly Val Asn Leu Ser Leu Ser Cys His Ala Ala Ser Asn Pro Pro Ala Gln Tyr Ser

         410          420          430          440          450
          •            •            •            •            •
TGG CTG ATT GAT GGG AAC ATC CAG CAA CAC ACA CAA GAG CTC TTT ATC TCC AAC ATC
Trp Leu Ile Asp Gly Asn Ile Gln Gln His Thr Gln Glu Leu Phe Ile Ser Asn Ile

   460          470          480          490          500          510
    •            •            •            •            •            •
ACT GAG AAG AAC AGC GGA CTC TAT ACC TGC CAG GCC AAT AAC TCA GCC AGT GGC CAC
Thr Glu Lys Asn Ser Gly Leu Tyr Thr Cys Gln Ala Asn Asn Ser Ala Ser Gly His

      520          530          540          550          560          570
       •            •            •            •            •            •
AGC AGG ACT ACA GTC AAG ACA ATC ACA GTC TCT GCG GAC GTG CCC AAG CCC TCC ATC
Ser Arg Thr Thr Val Lys Thr Ile Thr Val Ser Ala Asp Val Pro Lys Pro Ser Ile

         580          590          600          610          620
          •            •            •            •            •
TCC AGC AAC AAC TCC AAA CCC GTG GAG GAC AAG GAT GCT GTG GCC TTC CAC TGT GAA
Ser Ser Asn Asn Ser Lys Pro Val Glu Asp Lys Asp Ala Val Ala Phe His Cys Glu

   630          640          650          660          670          680
    •            •            •            •            •            •
CCT GAG GCT CAG AAC ACA ACC TAC CTG TGG TGG GTA AAT GGT CAG AGC CTC CCA GTC
Pro Glu Ala Gln Asn Thr Thr Tyr Leu Trp Trp Val Asn Gly Gln Ser Leu Pro Val

      690          700          710          720          730          740
       •            •            •            •            •            •
AGT CCC AGG CTG CAG CTG TCC AAT GGC AAC AGG ACC CTC ACT CTA TTC AAT GTC ACA
Ser Pro Arg Leu Gln Leu Ser Asn Gly Asn Arg Thr Leu Thr Leu Phe Asn Val Thr

         750          760          770          780          790          800
          •            •            •            •            •            •
AGA AAT GAC GCA AGA GCC TAT GTA TGT GGA ATC CAG AAC TCA GTG AGT GCA AAC CGC
Arg Asn Asp Ala Arg Ala Tyr Val Cys Gly Ile Gln Asn Ser Val Ser Ala Asn Arg

            810          820          830          840          850
             •            •            •            •            •
AGT GAC CCA GTC ACC CTG GAT GTC CTC TAT GGG CCG GAC ACC CCC ATC ATT TCC CCC
Ser Asp Pro Val Thr Leu Asp Val Leu Tyr Gly Pro Asp Thr Pro Ile Ile Ser Pro

860
 •
CCC CC
Pro
```

EP 0 263 933 B1

CEA-B

```
                10          20          30          40          50
                *           *           *           *           *
C ACC ATG GAG TCT CCC TCG GCC CCT CTC CAC AGA TGG TGC ATC CCC TGG CAG AGG CTC
  Met Glu Ser Pro Ser Ala Pro Leu His Arg Trp Cys Ile Pro Trp Gln Arg Leu

     60          70          80          90          100         110
     *           *           *           *           *           *
CTG CTC ACA GCC TCA CTT CTA ACC TTC TGG AAC CCG CCC ACC ACT GCC AAG CTC ACT
Leu Leu Thr Ala Ser Leu Leu Thr Phe Trp Asn Pro Pro Thr Thr Ala Lys Leu Thr

     120         130         140         150         160         170
     *           *           *           *           *           *
ATT GAA TCC ACG CCG TTC AAT GTC GCA GAG GGG AAG GAG GTG CTT CTA CTT GTC CAC
Ile Glu Ser Thr Pro Phe Asn Val Ala Glu Gly Lys Glu Val Leu Leu Leu Val His

     180         190         200         210         220
     *           *           *           *           *
AAT CTG CCC CAG CAT CTT TTT GGC TAC AGC TGG TAC AAA GGT GAA AGA GTG GAT GGC
Asn Leu Pro Gln His Leu Phe Gly Tyr Ser Trp Tyr Lys Gly Glu Arg Val Asp Gly

230         240         250         260         270         280
  *           *           *           *           *           *
AAC CGT CAA ATT ATA GGA TAT GTA ATA GGA ACT CAA CAA GCT ACC CCA GGG CCC GCA
Asn Arg Gln Ile Ile Gly Tyr Val Ile Gly Thr Gln Gln Ala Thr Pro Gly Pro Ala

     290         300         310         320         330         340
     *           *           *           *           *           *
TAC AGT GGT CGA GAG ATA ATA TAC CCC AAT GCA TCC CTG CTG ATC CAG AAC ATC ATC
Tyr Ser Gly Arg Glu Ile Ile Tyr Pro Asn Ala Ser Leu Leu Ile Gln Asn Ile Ile

     350         360         370         380         390         400
     *           *           *           *           *           *
CAG AAT GAC ACA GGA TTC TAC ACC CTA CAC GTC ATA AAG TCA GAT CTT GTG AAT GAA
Gln Asn Asp Thr Gly Phe Tyr Thr Leu His Val Ile Lys Ser Asp Leu Val Asn Glu

     410         420         430         440         450
     *           *           *           *           *
GAA GCA ACT GGC CAG TTC CGG GTA TAC CCG GAG CTG CCC AAG CCC TCC ATC TCC AGC
Glu Ala Thr Gly Gln Phe Arg Val Tyr Pro Glu Leu Pro Lys Pro Ser Ile Ser Ser

460         470         480         490         500         510
  *           *           *           *           *           *
AAC AAC TCC AAA CCC GTG GAG GAC AAG GAT GCT GTG GCC TTC ACC TGT GAA CCT GAG
Asn Asn Ser Lys Pro Val Glu Asp Lys Asp Ala Val Ala Phe Thr Cys Glu Pro Glu

     520         530         540         550         560         570
     *           *           *           *           *           *
ACT CAG GAC GCA ACC TAC CTG TGG TGG GTA AAC AAT CAG AGC CTC CCG GTC AGT CCC
Thr Gln Asp Ala Thr Tyr Leu Trp Trp Val Asn Asn Gln Ser Leu Pro Val Ser Pro

            560         570         600         610         620
             *           *           *           *           *
AGG CTG CAG CTG TCC AAT GGC AAC AGG ACC CTC ACT CTA TTC AAT GTC ACA AGA AAT
Arg Leu Gln Leu Ser Asn Gly Asn Arg Thr Leu Thr Leu Phe Asn Val Thr Arg Asn

630         640         650         660         670         680
  * .         *           *           *           *           *
AAA CAA GCA AGC TAC AAA TGT GAA ACC CAG AAC CCA GTG AGT GCC AGG CGC AGT GAT
Lys Gln Ala Ser Tyr Lys Cys Glu Thr Gln Asn Pro Val Ser Ala Arg Arg Ser Asp

     690         700         710         720         730         740
     *           *           *           *           *           *
TCA GTC ATC CTG AAT GTC CTC TAT GGC CCG GAT GCC CCC ACC ATT TCC CCT CTA AAC
Ser Val Ile Leu Asn Val Leu Tyr Gly Pro Asp Ala Pro Thr Ile Ser Pro Leu Asn

     750         760         770         780         790
     *           *           *           *           *
ACA TCT TAC AGA TCA GGG GAA AAT CTG AAC CTC TCC TGC CAC GCA GCC TCT AAC CCA
Thr Ser Tyr Arg Ser Gly Glu Asn Leu Asn Leu Ser Cys His Ala Ala Ser Asn Pro

800         810         820         830         840         850
  *           *           *           *           *           *
CCT GCA CAG TAC TCT TGG TTT GTC AAT GGG ACT TTC CAG CAA TCC ACC CAA GAG CTC
Pro Ala Gln Tyr Ser Trp Phe Val Asn Gly Thr Phe Gln Gln Ser Thr Gln Glu Leu

     860         870         880         890         900         910
     *           *           *           *           *           *
TTT ATC CCC AAC ATC ACT GTG AAT AAT AGT GGA TCC TAT ACG TGC CAA GCC CAT AAC
Phe Ile Pro Asn Ile Thr Val Asn Asn Ser Gly Ser Tyr Thr Cys Gln Ala His Asn
```

30

```
          720              730              940              950              760              970
           •                •                •                •                •                •
TCA GAC ACT GGC CTC AAT AGG ACC ACA GTC ACG ACG ATC ACA GTC TAT GCA GAG CCA
Ser Asp Thr Gly Leu Asn Arg Thr Thr Val Thr Thr Ile Thr Val Tyr Ala Glu Pro

               980              990             1000             1010             1020
                •                •                •                •                •
CCC AAA CCC TTC ATC ACC AGC AAC AAC TCC AAC CCC GTG GAG GAT GAG GAT GCT GTA
Pro Lys Pro Phe Ile Thr Ser Asn Asn Ser Asn Pro Val Glu Asp Glu Asp Ala Val

    1030             1040             1050             1060             1070             1080
     •                •                •                •                •                •
GCC TTA ACC TGT GAA CCT GAG ATT CAG AAC ACA ACC TAC CTG TGG TGG GTA AAT AAT
Ala Leu Thr Cys Glu Pro Glu Ile Gln Asn Thr Thr Tyr Leu Trp Trp Val Asn Asn

       1090             1100             1110             1120             1130             1140
        •                •                •                •                •                •
CAA AGC CTC CCG GTC AGT CCC AGG CTG CAG CTG TCC AAT AAC AAC AGG ACC CTC ACT
Gln Ser Leu Pro Val Ser Pro Arg Leu Gln Leu Ser Asn Asp Asn Arg Thr Leu Thr

          1150             1160             1170             1180             1190
           •                •                •                •                •
CTA CTC AGT GTC ACA AGG AAT GAT GTA GGA CCC TAT GAG TGT GGA ATC CAG AAC GAA
Leu Leu Ser Val Thr Arg Asn Asp Val Gly Pro Tyr Glu Cys Gly Ile Gln Asn Glu

    1200             1210             1220             1230             1240             1250
     •                •                •                •                •                •
TTA AGT GTT GAC CAC AGC GAC CCA GTC ATC CTG AAT GTC CTC TAT GGC CCA GAC GAC
Leu Ser Val Asp His Ser Asp Pro Val Ile Leu Asn Val Leu Tyr Gly Pro Asp Asp

       1260             1270             1280             1290             1300             1310
        •                •                •                •                •                •
CCC ACC ATT TCC CCC TCA TAC ACC TAT TAC CGT CCA GGG GTG AAC CTC AGC CTC TCC
Pro Thr Ile Ser Pro Ser Tyr Thr Tyr Tyr Arg Pro Gly Val Asn Leu Ser Leu Ser

          1320             1330             1340             1350             1360
           •                •                •                •                •
TGC CAT GCA GCC TCT AAC CCA CCT GCA CAG TAT TCT TGG CTG ATT GAT GGG AAC ATC
Cys His Ala Ala Ser Asn Pro Pro Ala Gln Tyr Ser Trp Leu Ile Asp Gly Asn Ile

1370             1380             1390             1400             1410             1420
 •                •                •                •                •                •
CAG CAA CAC ACA CAA GAG CTC TTT ATC TCC AAC ATC ACT GAG AAG AAC AGC GGA CTC
Gln Gln His Thr Gln Glu Leu Phe Ile Ser Asn Ile Thr Glu Lys Asn Ser Gly Leu

    1430             1440             1450             1460             1470             1480
     •                •                •                •                •                •
TAT ACC TGC CAG GCC AAT AAC TCA GCC AGT GGC CAC AGC AGG ACT ACA GTC AAG ACA
Tyr Thr Cys Gln Ala Asn Asn Ser Ala Ser Gly His Ser Arg Thr Thr Val Lys Thr

       1490             1500             1510             1520             1530             1540
        •                •                •                •                •                •
ATC ACA GTC TCT GCG GAC GTG CCC AAG CCC TCC ATC TCC AGC AAC AAC TCC AAA CCC
Ile Thr Val Ser Ala Asp Val Pro Lys Pro Ser Ile Ser Ser Asn Asn Ser Lys Pro

          1550             1560             1570             1580             1590
           •                •                •                •                •
GTG GAG GAC AAG GAT GCT GTG GCC TTC ACC TGT GAA CCT GAG GCT CAG AAC ACA ACC
Val Glu Asp Lys Asp Ala Val Ala Phe Thr Cys Glu Pro Glu Ala Gln Asn Thr Thr

1600             1610             1620             1630             1640             1650
 •                •                •                •                •                •
TAC CTG TGG TGG GTA AAT GGT CAG AGC CTC CCA GTC AGT CCC AGG CTG CAG CTG TCC
Tyr Leu Trp Trp Val Asn Gly Gln Ser Leu Pro Val Ser Pro Arg Leu Gln Leu Ser

    1660             1670             1680             1690             1700             1710
     •                •                •                •                •                •
AAT GGC AAC AGG ACC CTC ACT CTA TTC AAT GTC ACA AGA AAT GAC GCA AGA GCC TAT
Asn Gly Asn Arg Thr Leu Thr Leu Phe Asn Val Thr Arg Asn Asp Ala Arg Ala Tyr

       1720             1730             1740             1750             1760
        •                •                •                •                •
GTA TGT GGA ATC CAG AAC TCA GTG AGT GCA AAC CGC AGT GAC CCA GTC ACC CTG GAT
Val Cys Gly Ile Gln Asn Ser Val Ser Ala Asn Arg Ser Asp Pro Val Thr Leu Asp

1770             1780             1790             1800             1810             1820
 •                •                •                •                •                •
GTC CTC TAT GGG CCG GAC ACC CCC ATC ATT TCC CCC CCA GAC TCG TCT TAC CTT TCG
Val Leu Tyr Gly Pro Asp Thr Pro Ile Ile Ser Pro Pro Asp Ser Ser Tyr Leu Ser

    1830             1840             1850             1860             1870             1880
     •                •                •                •                •                •
GGA GCG AAC CTC AAC CTC TCC TGC CAC TCG GCC TCT AAC CCA TCC CCG CAG TAT TCT
Gly Ala Asn Leu Asn Leu Ser Cys His Ser Ala Ser Asn Pro Ser Pro Gln Tyr Ser
```

```
            1890          1900          1910          1920          1930
             •             •             •             •             •
TGG CGT ATC AAT GGG ATA CCG CAG CAA CAC ACA CAA GTT CTC TTT ATC GCC AAA ATC
Trp Arg Ile Asn Gly Ile Pro Gln Gln His Thr Gln Val Leu Phe Ile Ala Lys Ile

    1940          1950          1960          1970          1980          1990
     •             •             •             •             •             •
ACG CCA AAT AAT AAC GGG ACC TAT GCC TGT TTT GTC TCT AAC TTG GCT ACT GGC CGC
Thr Pro Asn Asn Asn Gly Thr Tyr Ala Cys Phe Val Ser Asn Leu Ala Thr Gly Arg

        2000          2010          2020          2030          2040          2050
         •             •             •             •             •             •
AAT AAT TCC ATA GTC AAG AGC ATC ACA GTC TCT GCA TCT GGA ACT TCT CCT GGT CTC
Asn Asn Ser Ile Val Lys Ser Ile Thr Val Ser Ala Ser Gly Thr Ser Pro Gly Leu

            2060          2070          2080          2090          2100          2110
             •             •             •             •             •             •
TCA GCT GGG GCC ACT GTC GGC ATC ATG ATT GGA GTG CTG GTT GGG GTT GCT CTG ATA
Ser Ala Gly Ala Thr Val Gly Ile Met Ile Gly Val Leu Val Gly Val Ala Leu Ile

                2120          2130          2140          2150          2160
                 •             •             •             •             •
TAG CAG CCC TGG TGT AGT TTC TTC ATT TCA GGA AGA CTG ACA GTT GTT TTG CTT CTT

        2170          2180          2190          2200          2210          2220
         •             •             •             •             •             •
CCT TAA AGC ATT TGC AAC AGC TAC AGT CTA AAA TTG CTT CTT TAC CAA GGA TAT TTA

            2230          2240          2250          2260          2270          2280
             •             •             •             •             •             •
CAG AAA ATA CTC TGA CCA GAG ATC GAG ACC ATC CTA GCC AAC ATC GTG AAA CCC CAT

                2290          2300          2310          2320          2330
                 •             •             •             •             •
CTC TAC TAA AAA TAC AAA AAT GAG CTG GGC TTG GTG GCG CGC ACC TGT AGT CCC AGT

    2340          2350          2360          2370          2380          2390
     •             •             •             •             •             •
TAC TCG GGA GGC TGA GGC AGG AGA ATC GCT TGA ACC CGG GAG GTG GAG ATT GCA GTG

        2400          2410          2420          2430          2440          2450
         •             •             •             •             •             •
AGC CCA GAT CGC ACC ACT GCA CTC CAG TCT GGC AAC AGA GCA AGA CTC CAT CTC AAA

            2460          2470          2480          2490          2500
             •             •             •             •             •
AAG AAA AGA AAA GAA GAC TCT GAC CTG TAC TCT TGA ATA CAA GTT TCT GAT ACC ACT

    2510          2520          2530          2540          2550          2560
     •             •             •             •             •             •
GCA CTG TCT GAG AAT TTC CAA AAC TTT AAT GAA CTA ACT GAC AGC TTC ATG AAA CTG

        2570          2580          2590          2600          2610          2620
         •             •             •             •             •             •
TCC ACC AAG ATC AAG CAG AGA AAA TAA TTA ATT TCA TGG GGA CTA AAT GAA CTA ATG

            2630          2640          2650          2660          2670          2680
             •             •             •             •             •             •
AGG ATA ATA TTT TCA TAA TTT TTT ATT TGA AAT TTT GCT GAT TCT TTA AAT GTC TTG

            2690          2700          2710          2720          2730
             •             •             •             •             •
TTT CCC AGA TTT CAG GAA ACT TTT TTT CTT TTA AGC TAT CCA CTC TTA CAG CAA TTT

    2740          2750          2760          2770          2780          2790
     •             •             •             •             •             •
GAT AAA ATA TAC TTT TGT GAA CAA AAA TTG AGA CAT TTA CAT TTT ATC CCT ATG TGG

        2800          2810          2820          2830
         •             •             •             •
TCG CTC CAG ACT TGG GAA ACT ATT CAT GAA TAT TTA TAT TGT ATG
```

CEA-C

```
          10        20        30        40        50
          |         |         |         |         |
AA TTC GGG CGG GAG CAG CCG TGC TCG AAG CGT TCC TGG AGC CCA AGC TCT CCT CCA CAG


          60        70        80        90        100       110
          |         |         |         |         |         |
GTG AAG ACA GGG CCA GCA GGA GAC ACC ATG GGG CAC CTC TCA GCC CCA CTT CAC AGA
                                    Met Gly His Leu Ser Ala Pro Leu His Arg


          120       130       140       150       160       170
          |         |         |         |         |         |
GTG CGT GTA CCC TGG CAG GGG CTT CTG CTC ACA GCC TCA CTT CTA ACC TTC TGG AAC
Val Arg Val Pro Trp Gln Gly Leu Leu Leu Thr Ala Ser Leu Leu Thr Phe Trp Asn


          180       190       200       210       220       230
          |         |         |         |         |         |
CCG CCC ACC ACT GCC CAG CTC ACT ACT GAA TCC ATG CCA TTC AAT GTT GCA GAG GGG
Pro Pro Thr Thr Ala Gln Leu Thr Thr Glu Ser Met Pro Phe Asn Val Ala Glu Gly
                    1   2   3   4   5   6   7   8   9  10  11  12  13  14


          240       250       260       270       280
          |         |         |         |         |
AAG GAG GTT CTT CTC CTT GTC CAC AAT CTG CCC CAG CAA CTT TTT GGC TAC AGC TGG
Lys Glu Val Leu Leu Leu Val His Asn Leu Pro Gln Gln Leu Phe Gly Tyr Ser Trp
 15  16  17  18  19  20  31  32  33  34  35  36  37  38  39  36  37  32  33


          290       300       310       320       330       340
          |         |         |         |         |         |
TAC AAA GGG GAA AGA GTG GAT GGC AAC CGT CAA ATT GTA GGA TAT GCA ATA GGA ACT
Tyr Lys Gly Glu Arg Val Asp Gly Asn Arg Gln Ile Val Gly Tyr Ala Ile Gly Thr
 34  35  36  37  38  39  38  41  42  43  44  45  46  47  48  49  50  51  52


          350       360       370       380       390       400
          |         |         |         |         |         |
CAA CAA GCT ACC CCA GGG CCC GCA AAC AGC GGT CGA GAG ACA ATA TAC CCC AAT GCA
Gln Gln Ala Thr Pro Gly Pro Ala Asn Ser Gly Arg Glu Thr Ile Tyr Pro Asn Ala
 53  54  55  56  57  58  59  60  61  62  63  64  65  66  67  68  69  70  71


          410       420       430       440       450
          |         |         |         |         |
TCC CTG CTG ATC CAG AAC GTC ACC CAG AAT GAC ACA GGA TTC TAC ACC CTA CAA GTC
Ser Leu Leu Ile Gln Asn Val Thr Gln Asn Asp Thr Gly Phe Tyr Thr Leu Gln Val
 72  73  74  75  76  77  78  79  80  81  82  83  84  85  86  87  88  89  90


          460       470       480       490       500       510
          |         |         |         |         |         |
ATA AAG TCA GAT CTT GTG AAT GAA GAA GCA ACT GGA CAG TTC CAT GTA TAC CCG GAG
Ile Lys Ser Asp Leu Val Asn Glu Glu Ala Thr Gly Gln Phe His Val Tyr Pro Glu
 91  92  93  94  95  96  97  98  99 100 101 102 103 104 105 106 107 108 109


          520       530       540       550       560       570
          |         |         |         |         |         |
CTG CCC AAG CCC TCC ATC TCC AGC AAC AAC TCC AAC CCT GTG GAG GAC AAG GAT GCT
Leu Pro Lys Pro Ser Ile Ser Ser Asn Asn Ser Asn Pro Val Glu Asp Lys Asp Ala
110 111 112 113 114 115 116 117 118 119 120 121 122 123 124 125 126 127 128
```

```
        580         590         600         610         620
         |           |           |           |           |
GTG GCC TTC ACC TGT GAA CCT GAG ACT CAG GAC ACA ACC TAC CTG TGG TGG ATA AAC
Val Ala Phe Thr Cys Glu Pro Glu Thr Gln Asp Thr Thr Tyr Leu Trp Trp Ile Asn
129 130 131 132 133 134 135 136 137 138 139 140 141 142 143 144 145 146 147

        630         640         650         660         670         680
         |           |           |           |           |           |
AAT CAG AGC CTC CCG GTC AGT CCC AGG CTG CAG CTG TCC AAT GGC AAC AGG ACC CTC
Asn Gln Ser Leu Pro Val Ser Pro Arg Leu Gln Leu Ser Asn Gly Asn Arg Thr Leu
148 149 150 151 152 153 154 155 156 157 158 159 160 161 162 163 164 165 166

        690         700         710         720         730         740
         |           |           |           |           |           |
ACT CTA CTC AGT GTC ACA AGG AAT GAC ACA GGA CCC TAT GAG TGT GAA ATA CAG AAC
Thr Leu Leu Ser Val Thr Arg Asn Asp Thr Gly Pro Tyr Glu Cys Glu Ile Gln Asn
167 168 169 170 171 172 173 174 175 176 177 178 179 180 181 182 183 184 185

        750         760         770         780         790         800
         |           |           |           |           |           |
CCA GTG AGT GCG AAC CGC AGT GAC CCA GTC ACC TTG AAT GTC ACC TAT GGC CCG GAC
Pro Val Ser Ala Asn Arg Ser Asp Pro Val Thr Leu Asn Val Thr Tyr Gly Pro Asp
186 187 188 189 190 191 192 193 194 195 196 197 198 199 200 201 202 203 204

        810         820         830         840         850
         |           |           |           |           |
ACC CCC ACC ATT TCC CCT TCA GAC ACC TAT TAC CGT CCA GGG GCA AAC CTC AGC CTC
Thr Pro Thr Ile Ser Pro Ser Asp Thr Tyr Tyr Arg Pro Gly Ala Asn Leu Ser Leu
205 206 207 208 209 210 211 212 213 214 215 216 217 218 219 220 221 222 223

        860         870         880         890         900         910
         |           |           |           |           |           |
TCC TGC TAT GCA GCC TCT AAC CCA CCT GCA CAG TAC TCC TGG CTT ATC AAT GGA ACA
Ser Cys Tyr Ala Ala Ser Asn Pro Pro Ala Gln Tyr Ser Trp Leu Ile Asn Gly Thr
224 225 226 227 228 229 230 231 232 233 234 235 236 237 238 239 240 241 242

        920         930         940         950         960         970
         |           |           |           |           |           |
TTC CAG CAA AGC ACA CAA GAG CTC TTT ATC CCT AAC ATC ACT GTG AAT AAT AGT GGA
Phe Gln Gln Ser Thr Gln Glu Leu Phe Ile Pro Asn Ile Thr Val Asn Asn Ser Gly
243 244 245 246 247 248 249 250 251 252 253 254 255 256 257 258 259 260 261

        980         990        1000        1010        1020
         |           |           |           |           |
TCC TAT ACC TGC CAC GCC AAT AAC TCA GTC ACT GGC TGC AAC AGG ACC ACA GTC AAG
Ser Tyr Thr Cys His Ala Asn Asn Ser Val Thr Gly Cys Asn Arg Thr Thr Val Lys
262 263 264 265 266 267 268 269 270 271 272 273 274 275 276 277 278 279 280

1030        1040        1050        1060        1070        1080
 |           |           |           |           |           |
ACG ATC ATA GTC ACT GAG CTA AGT CCA GTA GTA GCA AAG CCC CAA ATC AAA GCC AGC
Thr Ile Ile Val Thr Glu Leu Ser Pro Val Val Ala Lys Pro Gln Ile Lys Ala Ser
281 282 283 284 285 286 287 288 289 290 291 292 293 294 295 296 297 298 299
```

```
          1090          1100          1110          1120          1130          1140
            '             '             '             '             '             '
AAG ACC ACA GTC ACA GGA GAT AAG GAC TCT GTG AAC CTG ACC TGC TCC ACA AAT GAC
Lys Thr Thr Val Thr Gly Asp Lys Asp Ser Val Asn Leu Thr Cys Ser Thr Asn Asp
300 301 302 303 304 305 306 307 308 309 310 311 312 313 314 315 316 317 318

            1150          1160          1170          1180          1190
              '             '             '             '             '
ACT GGA ATC TCC ATC CGT TGG TTC TTC AAA AAC CAG AGT CTC CCG TCC TCG GAG AGG
Thr Gly Ile Ser Ile Arg Trp Phe Phe Lys Asn Gln Ser Leu Pro Ser Ser Glu Arg
319 320 321 322 323 324 325 326 327 328 329 330 331 332 333 334 335 336 337

     1200          1210          1220          1230          1240          1250
       '             '             '             '             '             '
ATG AAG CTG TCC CAG GGC AAC ACC ACC CTC AGC ATA AAC CCT GTC AAG AGG GAG GAT
Met Lys Leu Ser Gln Gly Asn Thr Thr Leu Ser Ile Asn Pro Val Lys Arg Glu Asp
338 339 340 341 342 343 344 345 346 347 348 349 350 351 352 353 354 355 356

       1260          1270          1280          1290          1300          1310
         '             '             '             '             '             '
GCT GGG ACG TAT TGG TGT GAG GTC TTA ACC CAA TCA GTA AGA ACC AAA GCG ACC CCA
Ala Gly Thr Tyr Trp Cys Glu Val Leu Thr Gln Ser Val Arg Thr Lys Ala Thr Pro
357 358 359 360 361 362 363 364 365 366 367 368 369 370 371 372 373 374 375

         1320          1330          1340          1350          1360          1370
           '             '             '             '             '             '
TCA TGG CTG AAC GTA AAC TAT AAT GCT CTA CCA CAA GAA AAT GGC CTC TCA CCT GGG
Ser Trp Leu Asn Val Asn Tyr Asn Ala Leu Pro Gln Glu Asn Gly Leu Ser Pro Gly
376 377 378 379 320 321 322 323 324 325 326 327 328 329 390 391 392 393 394

           1380          1390          1400          1410          1420
             '             '             '             '             '
GCC ATT GCT GGC ATT GTG ATT GGA GTA GTG GCC CTG GTT GCT CTG ATA GCA GTA GCC
Ala Ile Ala Gly Ile Val Ile Gly Val Val Ala Leu Val Ala Leu Ile Ala Val Ala
395 396 397 398 399 400 401 402 403 404 405 406 407 408 409 410 411 412 413

     1430          1440          1450          1460          1470          1480
       '             '             '             '             '             '
CTG GCA TGT TTT CTG CAT TTC GGG AAG ACC GGC AGG GCA AGC GAC CAG CGT GAT CTC
Leu Ala Cys Phe Leu His Phe Gly Lys Thr Gly Arg Ala Ser Asp Gln Arg Asp Leu
414 415 416 417 418 419 420 421 422 423 424 425 426 427 428 429 430 431 432

       1490          1500          1510          1520          1530          1540
         '             '             '             '             '             '
ACA GAG CAC AAA CCC TCA GTC TCC AAC CAC ACT CAG GAC CAC TCC AAT GAC CCA CCT
Thr Glu His Lys Pro Ser Val Ser Asn His Thr Gln Asp His Ser Asn Asp Pro Pro
433 434 435 436 437 438 439 440 441 442 443 444 445 446 447 448 449 450 451

         1550          1560          1570          1580          1590
           '             '             '             '             '
AAC AAG ATG AAT GAA GTT ACT TAT TCT ACC CTG AAC TTT GAA GCC CAG CAA CCC ACA
Asn Lys Met Asn Glu Val Thr Tyr Ser Thr Leu Asn Phe Glu Ala Gln Gln Pro Thr
452 453 454 455 456 457 458 459 460 461 462 463 464 465 466 467 468 469 470

   1600          1610          1620          1630          1640          1650
     '             '             '             '             '             '
CAA CCA ACT TCA GCC TCC CCA TCC CTA ACA GCC ACA GAA ATA ATT TAT TCA GAA GTA
Gln Pro Thr Ser Ala Ser Pro Ser Leu Thr Ala Thr Glu Ile Ile Tyr Ser Glu Val
471 472 473 474 475 476 477 478 479 480 481 482 483 484 485 486 487 488 489
```

```
      1660        1670        1680        1690        1700        1710
       ‡           ‡           ‡           ‡           ‡           ‡
  AAA AAG CAG TAA TGA AAC CTG TCC TGC TCA CTG CAG TGC TGA TGT ATT TCA AGT CTC
  Lys Lys Gln ---
  470 471 472
             1720        1730        1740        1750        1760
              ‡           ‡           ‡           ‡           ‡
  TCA CCC TCA TCA CTA GGA GAT TCC TTT CCC CTG TAG GGG TAG AGG GGT GGG GAC AGA


  1770        1780        1790        1800        1810        1820
   ‡           ‡           ‡           ‡           ‡           ‡
  AAC AGC TTT CTC CTA CTC TTC CTT CCT AAT AGG CAT CTC CAG GCT GCC TGG TCA CTG


      1830        1840        1850        1860        1870        1880
       ‡           ‡           ‡           ‡           ‡           ‡
  CCC CTC TCT CAG TGT CAA TAG ATG AAA GTA CAT TGG GAG TCT GTA GGA AAC CCA ACC


      1890        1900        1910        1920        1930        1940
       ‡           ‡           ‡           ‡           ‡           ‡
  TTC TTG TCA TTG AAA TTT GGC AAA GCT GAC TTT GGG AAA GAG GGA CCA GAA CTT CCC.


      1950        1960        1970        1980        1990
       ‡           ‡           ‡           ‡           ‡
  CTC CCT TCC CCT TTT CCC AAC CTG GAC TTG TTT TAA ACT TGC CTG TTC AGA GCA CTC


  2000        2010        2020        2030        2040        2050
   ‡           ‡           ‡           ‡           ‡.          ‡
  ATT CCT TCC CAC CCC CAG TCC TGT CCT ATC ACT CTA ATT CGG ATT TGC CAT AGC CTT


      2060        2070        2080        2090        2100        2110
       ‡           ‡           ‡           ‡           ‡           ‡
  GAG GTT ATG TCC TTT TCC ATT AAG TAC ATG TGC CAG GAA ACA GCG AGA GAG AGA AAG


      2120        2130        2140        2150        2160
       ‡           ‡           ‡           ‡           ‡
  TAA ACG GCA GTA ATG CTT CTC CTA TTT CTC CAA AGC CTT GTG TGA ACT AGC AAA GAG


  2170        2180        2190        2200        2210        2220
   ‡           ‡           ‡           ‡           ‡           ‡
  AAG AAA ACC AAA TAT ATA ACC AAT AGT GAA ATG CCA CAG GTT TGT CCA CTG TCA GGG


      2230        2240        2250        2260        2270        2280
       ‡           ‡           ‡           ‡           ‡         ‡  ‡
  TTG TCT ACC TGT AGG ATC AGG GTC TAA GCA CCT TGG TGC TTA GCT AGA ATA CCA CCT
```

36

```
        2290          2300          2310          2320          2330
         •             •             •             •             •
AAT CCT TCT GGC AAG CCT GTC TTC AGA GAA CCC ACT AGA AGC AAC TAG GAA AAA TCA ·


2340          2350          2360          2370          2380          2390
 •             •             •             •             •             •
CTT GCC AAA ATC CAA GGC AAT TCC TGA TGG AAA ATG CAA AAG CAC ATA TAT GTT TTA


        2400          2410          2420
         •             •             •
ATA TCT TTA TGG GCT CTG TTC AAG GCA GTG CTG
```

CEA-D

```
        10          20          30 ~        40          50
         '           '           '           '           '
CC GGG GGA CAC GCA GGG CCA ACA GTC ACA GCA GCC CTG ACC AGA GCA TTC CTG GAG CTC


60          70          80 ·       · 90         100         110
 '  .        '  .        '           '           '   .        '
AAG CTC TCT ACA AAG AGG TGG ACA GAG AAG ACA GCA GAG ACC ATG GGA CCC CCC TCA
                                                        Met Gly Pro Pro Ser


   120         130         .140        150         160         170
    '           '           '           '           '           '
GCC CCT CCC TGC AGA TTG CAT GTC CCC TGG AAG GAG GTC CTG CTC ACA GCC TCA CTT
Ala Pro Pro Cys Arg Leu His Val Pro Trp Lys Glu Val Leu Leu Thr Ala Ser Leu


      180         190         200         210 .       220         230
       '           '           '         · '          '  '        '
 CTA ACC TTC TGG AAC CCA CCC ACC ACT GCC AAG CTC ACT ATT GAA TCC ACG CCA TTC
 Leu Thr Phe Trp Asn Pro Pro Thr Thr Ala Lys Leu Thr Ile Glu Ser Thr Pro Phe
                                       1  2  3  4  5  6  7 ·8  9
      240         250       · 260        270         280 ·
       '           '           '           '           '
AAT GTC GCA GAG GGG AAG GAG GTT CTT CTA CTC GCC CAC AAC CTG CCC CAG AAT CGT
Asn Val Ala Glu Gly Lys Glu Val Leu Leu Leu Ala His Asn Leu Pro Gln Asn Arg
10  11  12  13  14  15  16  17  18  19  20  21 .22 23  24 25  26  27  28


   290         300         310         320         330         340
    '           '           '           '           '           '
AIT GGT TAC AGC TGG TAC AAA GGC GAA AGA GTG GAT GGC AAC AGT CTA ATT GTA GGA
 Ile Gly Tyr Ser Trp Tyr Lys Gly Glu Arg Val Asp Gly Asn Ser Leu Ile Val Gly
 29  30  31  32  33  34  35  36  37  38  39  40  41  42  43  44  45  46  47

      350         360         370         380         390         400
       '           '           '           '           '           ' .
TAT GTA ATA GGA ACT CAA CAA GCT ACC CCA GGG CCC GCA TAC AGT GGT CGA GAG ACA
Tyr Val Ile Gly Thr Gln Gln Ala Thr Pro Gly Pro Ala Tyr Ser Gly Arg Glu Thr
48  49  50  51  52  53  54  55  56  57  58  59  60  61  62  63  64  65  66


      410         420         430         440         450
       '           '           '           '           '
ATA TAC CCC AAT GCA TCC CTG CTG ATC CAG AAC GTC ACC CAG AAT GAC ACA GGA TTC
Ile Tyr Pro Asn Ala Ser Leu Leu Ile Gln Asn Val Thr Gln Asn Asp Thr Gly Phe
67  68  69  70  71  72  73  74  75  76  77  78  79  80  81  82  83  84  85

460         470         480         490         500         510
 '           '           '           '           '           '  ·
TAC ACC CTA CAA GTC ATA AAG TCA GAT CTT GTG AAT GAA GAA GCA ACC GGA CAG TTC
Tyr Thr Leu Gln Val Ile Lys Ser Asp Leu Val Asn Glu Glu Ala Thr Gly Gln Phe
86  87  88  89  90  91  92  93  94  95  96  97  98  99 100 101 102 103 104
      520         530         540         550         560         570
       '           '           '           '           '           '
CAT GTA TAC CCG GAG CTG CCC AAG CCC TCC ATC TCC AGC AAC AAC TCC AAC CCC GTG
His Val Tyr Pro Glu Leu Pro Lys Pro Ser Ile Ser Ser Asn Asn Ser Asn Pro Val
105 106 107 108 109 110 111 112 113 114 115 116 117 118 119 120 121 122 123
```

EP 0 263 933 B1

```
          580         590         600         610         620
           '           '           '           '           '
GAG GAC AAG GAT GCT GTG GCC TTC ACC TGT GAA CCT GAG GTT CAG AAC ACA ACC TAC
Glu Asp Lys Asp Ala Val Ala Phe Thr Cys Glu Pro Glu Val Gln Asn Thr Thr Tyr
124 125-126 127 128 129 130 131 132 133 134 135 136 137 138 139 140 141 142

  630         640         650         660         670         680
   '           '           '           '           '           '
CTG TGG TGG GTA AAT GGT CAG AGC CTC CCG GTC AGT CCC AGG CTG CAG CTG TCC AAT
Leu Trp Trp Val Asn Gly Gln Ser Leu Pro Val Ser Pro Arg Leu Gln Leu Ser Asn
143 144 145 146 147 148 149 150 151 152 153 154 155 156 157 158 159 160 161

  690         700         710         720         730         740
   '           '           '           '           '           '
GGC AAC AGG ACC CTC ACT CTA CTC AGC GTC AAA AGG AAC GAT GCA GGA TCG TAT GAA
Gly Asn Arg Thr Leu Thr Leu Leu Ser Val Lys Arg Asn Asp Ala Gly Ser Tyr Glu
162 163 164 165 166 167 168 169 170 171 172 173 174 175 176 177 178 179 180

      750         760         770         780         790         800
       '           '           '           '           '           '
TGT GAA ATA CAG AAC CCA GCG AGT GCC AAC CGC AGT GAC CCA GTC ACC CTG AAT GTC
Cys Glu Ile Gln Asn Pro Ala Ser Ala Asn Arg Ser Asp Pro Val Thr Leu Asn Val
181 182 183 184 185 186 187 188 189 190 191 192 193 194 195 196 197 198 199

      810         820         830         840         850
       '           '           '           '           '
CTC TAT GGC CCA GAT GGC CCC ACC ATT TCC CCC TCA AAG GCC AAT TAC CGT CCA GGG
Leu Tyr Gly Pro Asp Gly Pro Thr Ile Ser Pro Ser Lys Ala Asn Tyr Arg Pro Gly
200 201 202 203 204 205 206 207 208 209 210 211 212 213 214 215 216 217 218

  860         870         880         890         900         910
   '           '           '           '           '           '
GAA AAT CTG AAC CTC TCC TGC CAC GCA GCC TCT AAC CCA CCT GCA CAG TAC TCT TGG
Glu Asn Leu Asn Leu Ser Cys His Ala Ala Ser Asn Pro Pro Ala Gln Tyr Ser Trp
219 220 221 222 223 224 225 226 227 228 229 230 231 232 233 234 235 236 237

      920         930         940         950         960         970
       '           '           '           '           '           '
TTT ATC AAT GGG ACG TTC CAG CAA TCC ACA CAA GAG CTC TTT ATC CCC AAC ATC ACT
Phe Ile Asn Gly Thr Phe Gln Gln Ser Thr Gln Glu Leu Phe Ile Pro Asn Ile Thr
238 239 240 241 242 243 244 245 246 247 248 249 250 251 252 253 254 255 256

      980         990        1000        1010        1020
       '           '           '           '           '
GTG AAT AAT AGC GGA TCC TAT ATG TGC CAA GCC CAT AAC TCA GCC ACT GGC CTC AAT
Val Asn Asn Ser Gly Ser Tyr Met Cys Gln Ala His Asn Ser Ala Thr Gly Leu Asn
257 258 259 260 261 262 263 264 265 266 267 268 269 270 271 272 273 274 275

 1030        1040        1050        1060        1070        1080
   '           '           '           '           '           '
AGG ACC ACA GTC ACG ATG ATC ACA GTC TCT GGA AGT GCT CCT GTC CTC TCA GCT GTG
Arg Thr Thr Val Thr Met Ile Thr Val Ser Gly Ser Ala Pro Val Leu Ser Ala Val
276 277 278 279 280 281 282 283 284 285 286 287 288 289 290 291 292 293 294

 1090        1100        1110        1120        1130        1140
   '           '           '           '           '           '
GCC ACC GTC GGC ATC ACG ATT GGA GTG CTG GCC AGG GTG GCT CTG ATA TAG CAG CCC
Ala Thr Val Gly Ile Thr Ile Gly Val Leu Ala Arg Val Ala Leu Ile ---
295 296 297 298 299 300 301 302 303 304 305 306 307 308 309 310
```

39

EP 0 263 933 B1

```
        1150         1160         1170         1180         1190
          I            I            I            I            I
    TGG TGT ATT TTC GAT ATT TCA GGA AGA CTG GCA GAT TGG ACC AGA CCC TGA ATT CTT


    1200        1210        1220        1230        1240        1250
      I           I           I           I           I           I
    CTA GCT CCT CCA ATC CCA TTT TAT CCC ATG GAA CCA CTA AAA ACA AGG TCT GCT CTG


      1260        1270        1280        1290        1300        1310
        I           I           I           I           I           I
    CTC CTG AAG CCC TAT ATG CTG GAG ATG GAC AAC TCA ATG AAA ATT TAA AGG AAA AAC


      1320        1330        1340        1350        1360        1370
        I           I           I           I           I           I
    CCT CAG GCC TGA GGT GTG TGC CAC TCA GAG ACT TCA CCT AAC TAG AGA CAG GCA AAC


              1380        1390        1400        1410        1420
                I           I           I           I           I
    TGC AAA CCA nnC CTC TTT CGC TTG GCA GGA TGA TGG TGT CAT TAG TAT TTC ACA AGA


    1430        1440        1450        1460        1470        1480
      I           I           I           I           I           I
    AGT AGC TTC AGn GGG TAA CTT AAC AGA GTA TCA GAT CTA TCT TGT CAA TCC CAA CGT


          1490        1500        1510        1520        1530        1540
            I           I           I           I           I           I
    TTT ACA TAA AAT AAG CGA TCC TTT AGT GCA CCC AGT GAC TGA CAT TAG CAG CAT CTT


              1550        1560        1570        1580        1590
                I           I           I           I           I
    ' TAA CAC AGC CGT GTG TTC AAG TGT ACA GTG GTC CTT TTC AGA GTT GGn nnT ACT CCA


    1600        1610        1620        1630        1640        1650
      I           I           I           I           I           I
    ACT GAA ATG TTA AGG AAG AAG ATA GAT CCA ATT AAA AAA AAT TAA AAC CAA TTT AAA


      1660        1670        1680        1690        1700        1710
        I           I           I           I           I           I
    AAA AAA AAA GAA CAC AGG AGA TTC CAG TCT ACT TGA GTT AGC ATA ATA CAG AAG TCC


          1720        1730        1740        1750        1760
            I           I           I           I           I
    CCT CTA CTT TAA CTT TTA CAA AAA AGT AAC CTG AAC TAA TCT GAT GTT AAC CAA TGT
```

40

EP 0 263 933 B1

```
    1770         1780.        1790         1800         1810         1820
     ?            ?            ?            ?            ?            ?
   ATT TAT TTG TCT GGT TCT GTT TCC TTG TTC CAA TTT GAC AAA ACC CAC TGT TCT TGT


    1830         1840         1850         1860         1870         1880
     ?            ?            ?            ?            ?            ?.
   ATT GTA TTG CCC AGG GGG AGC TAT CAC TGT ACT TGT AGA GTG GTG CTG CTT TAA GTT


    1890        - 1900        1910         1920         1930         1940
     ?            ?            ?            ?            ?            ?
   CAT AAA TCA CAA ATA AAA GCC AAT TAG CTC TAT AAC TAA AAA AAA AAA AAA AAA AAA


    1950         1960
     ?            ?
   AAA AAA AAA AAG AAA AAA AAA AAA
```

2. A replicable recombinant cloning vehicle having an insert comprising a nucleic acid according to claim 1.

3. A cell that is transfected, infected, or injected with a replicable recombinant cloning vehicle according to claim 2.

4. Cell lines having the ATCC designation ATCC 67312 and ATCC 67169.

5. A method for preparing a polypeptide belonging to the CEA family, said method comprising the steps of
   (a) culturing the cell of Claim 3 or the cell lines of Claim 4 and
   (b) recovering the CEA polypeptide expressed by said cell or cell line.

6. A peptide having an amino acid sequence derived from the sequences as recited in claim 1, said peptide selected from the group of

CEA-B  (i) amino acid residues 135 to and including 386.
       (ii) amino acid residues 174 to and including 220.
       (iii) amino acid residues 285 to and including 386.
       (iv) amino acid residues 174 to and including 200.
       (v) amino acid residues 208 to and including 216.
       (vi) amino acid residues 285 to and including 290.
       (vii) amino acid residues 292 to and including 308.
       (viii) amino acid residues 312 to and including 321.
       (ix) amino acid residues 360 to and including 372.
       (x) amino acid residues 380 to and including 386.
CEA-C  (i) amino acid residues 135 to and including 386.
       (ii) amino acid residues 411 to and including 492.
       (iii) amino acid residues 174 to and including 220.
       (iv) amino acid residues 174 to and including 200.
       (v) amino acid residues 285 to and including 389.
       (vi) amino acid residues 208 to and including 222.
       (vii) amino acid residues 285 to and including 292.
       (viii) amino acid residues 295 to and including 311.
       (ix) amino acid residues 315 to and including 328.
       (x) amino acid residues 331 to and including 345.
       (xi) amino acid residues 348 to and including 360.
       (xii) amino acid residues 363 to and including 377.

41

(xiii) amino acid residues 383 to and including 390.

CEA-D    (i) amino acid residues 135 to and including 285.
(ii) amino acid residues 168 to and including 220.
(iii) amino acid residues 168 to and including 196.
(iv) amino acid residues 210 to and including 220. and
(v) amino acid residues 264 to and including 283.

7. A method for preparing an antibody directed against a polypeptide belonging to the CEA family said method comprising the steps of
(a) preparing said polypeptide by the method of Claim 5
(b) injecting said polypeptide into a host capable of producing antibodies and
(c) recovering said antibodies.

8. A method for preparing a monoclonal antibody capable of recognizing a polypeptide belonging to the CEA family said method comprising the steps of
(a) preparing said polypeptide by the method of Claim 5
(b) injecting said polypeptide into a host capable of producing antibodies
(c) recovering antibody-producing lymphocytes from said host
(d) fusing said lymphocytes with a myeloma cell line, thus obtaining antibody-producing hybridoma cells
(e) cultivating the hybridoma cells and
(f) recovering the monoclonal antibodies produced by the cells.

9. A method for preparing an antibody directed against a polypeptide belonging to the CEA family, said method comprising the steps of
(a) injecting a peptide of Claim 6 into a host capable of producing antibodies and
(b) recovering said antibodies.

10. A method for preparing a monoclonal antibody capable of recognizing a polypeptide belonging to the CEA family said method comprising the steps of
(a) injecting a peptide of Claim 6 into a host capable of producing antibodies
(b) recovering antibody-producing lymphocytes from said host
(c) fusing said lymphocytes with a myeloma cell line, thus obtaining antibody-producing hybridoma cells
(d) cultivating the hybridoma cells and
(e) recovering the monoclonal antibodies produced by the cells.

11. The method according to claim 7 to 11 wherein the antibody is specific for a particular polypeptide belonging to the CEA gene family.

12. An immunoassay method for detecting in a test sample a polypeptide belonging to the CEA family said method comprising the steps of
(a) preparing an antibody directed against said polypeptide by the method of any of Claims 7- 11
(b) detecting binding thereof to CEA contained in the sample.

13. A method for preparing a test kit comprising a polypeptide belonging to the CEA family or an antibody directed against said polypeptide, said method comprising the steps of
(a) preparing said polypeptide by the method of Claim 5 or said antibody by the method of any one of Claims 7-11 and
(b) preparing a test kit containing said polypeptide or antibody.

14. A method for preparing a pharmaceutical product comprising a polypeptide belonging to the CEA family or an antibody directed against said polypeptide, said method comprising the steps of
(a) preparing said polypeptide by the method of Claim 5 or said antibody by the method of any one of Claims 7-11 and
(b) preparing a test kit containing said polypeptide or antibody.

15. Use of a nucleic acid according to claims 1 in a nucleic hybridization assay.

**EP 0 263 933 B1**

**16.** A pharmaceutical product or a test kit comprising one or more of the peptides of Claim 6.

**Patentansprüche**

**1.** Isolierte Nukleinsäure, die ein zur CEA-Genfamilie gehörendes Polypeptid codiert, ausgewählt aus den unten aufgezählten Sequenzen und zumindest 50 konsekutive Basen umfassenden Fragmenten davon:

EP 0 263 933 B1

CEA-A

```
              10            20            30            40            50
               •             •             •             •             •
GG GGT TTA CAC AAC CAC CAC CCC ATC AAA CCC TTC ATC ACC AGC AAC AAC TCC AAC CCC
   Gly Leu His Asn His His Pro Ile Lys Pro Phe Ile Thr Ser Asn Asn Ser Asn Pro

   60            70            80            90            100           110
    •             •             •             •             •             •
GTG GAG GAT GAG GAT GCT GTA GCC TTA ACC TGT GAA CCT GAG ATT CAG AAC ACA ACC
Val Glu Asp Glu Asp Ala Val Ala Leu Thr Cys Glu Pro Glu Ile Gln Asn Thr Thr

      120           130           140           150           160           170
       •             •             •             •             •             •
TAC CTG TGG TGG GTA AAT AAT CAG AGC CTC CCG GTC AGT CCC AGG CTG CAG CTG TCC
Tyr Leu Trp Trp Val Asn Asn Gln Ser Leu Pro Val Ser Pro Arg Leu Gln Leu Ser

         180           190           200           210           220           230
          •             •             •             •             •             •
AAT GAC AAC AGG ACC CTC ACT CTA CTC AGT GTC ACA AGG AAT GAT GTA GGA CCC TAT
Asn Asp Asn Arg Thr Leu Thr Leu Leu Ser Val Thr Arg Asn Asp Val Gly Pro Tyr

            240           250           260           270           280
             •             •             •             •             •
GAG TGT GGA ATC CAG AAC GAA TTA AGT GTT GAC CAC AGC GAC CCA GTC ATC CTG AAT
Glu Cys Gly Ile Gln Asn Glu Leu Ser Val Asp His Ser Asp Pro Val Ile Leu Asn

   290           300           310           320           330           340
    •             •             •             •             •             •
GTC CTC TAT GGC CCA GAC GAC CCC ACC ATT TCC CCC TCA TAC ACC TAT TAC CGT CCA
Val Leu Tyr Gly Pro Asp Asp Pro Thr Ile Ser Pro Ser Tyr Thr Tyr Tyr Arg Pro

      350           360           370           380           390           400
       •             •             •             •             •             •
GGG GTG AAC CTC AGC CTC TCC TGC CAT GCA GCC TCT AAC CCA CCT GCA CAG TAT TCT
Gly Val Asn Leu Ser Leu Ser Cys His Ala Ala Ser Asn Pro Pro Ala Gln Tyr Ser

         410           420           430           440           450
          •             •             •             •             •
TGG CTG ATT GAT GGG AAC ATC CAG CAA CAC ACA CAA GAG CTC TTT ATC TCC AAC ATC
Trp Leu Ile Asp Gly Asn Ile Gln Gln His Thr Gln Glu Leu Phe Ile Ser Asn Ile

   460           470           480           490           500           510
    •             •             •             •             •             •
ACT GAG AAG AAC AGC GGA CTC TAT ACC TGC CAG GCC AAT AAC TCA GCC AGT GGC CAC
Thr Glu Lys Asn Ser Gly Leu Tyr Thr Cys Gln Ala Asn Asn Ser Ala Ser Gly His

      520           530           540           550           560           570
       •             •             •             •             •             •
AGC AGG ACT ACA GTC AAG ACA ATC ACA GTC TCT GCG GAC GTG CCC AAG CCC TCC ATC
Ser Arg Thr Thr Val Lys Thr Ile Thr Val Ser Ala Asp Val Pro Lys Pro Ser Ile

         580           590           600           610           620
          •             •             •             •             •
TCC AGC AAC AAC TCC AAA CCC GTG GAG GAC AAG GAT GCT GTG GCC TTC CAC TGT GAA
Ser Ser Asn Asn Ser Lys Pro Val Glu Asp Lys Asp Ala Val Ala Phe His Cys Glu

630           640           650           660           670           680
 •             •             •             •             •             •
CCT GAG GCT CAG AAC ACA ACC TAC CTG TGG TGG GTA AAT GGT CAG AGC CTC CCA GTC
Pro Glu Ala Gln Asn Thr Thr Tyr Leu Trp Trp Val Asn Gly Gln Ser Leu Pro Val

   690           700           710           720           730           740
    •             •             •             •             •             •
AGT CCC AGG CTG CAG CTG TCC AAT GGC AAC AGG ACC CTC ACT CTA TTC AAT GTC ACA
Ser Pro Arg Leu Gln Leu Ser Asn Gly Asn Arg Thr Leu Thr Leu Phe Asn Val Thr

      750           760           770           780           790           800
       •             •             •             •             •             •
AGA AAT GAC GCA AGA GCC TAT GTA TGT GGA ATC CAG AAC TCA GTG AGT GCA AAC CGC
Arg Asn Asp Ala Arg Ala Tyr Val Cys Gly Ile Gln Asn Ser Val Ser Ala Asn Arg

         810           820           830           840           850
          •             •             •             •             •
AGT GAC CCA GTC ACC CTG GAT GTC CTC TAT GGG CCG GAC ACC CCC ATC ATT TCC CCC
Ser Asp Pro Val Thr Leu Asp Val Leu Tyr Gly Pro Asp Thr Pro Ile Ile Ser Pro

   860
    •
CCC CC
Pro
```

44

CEA-B

```
              10          20          30          40           50
C ACC ATG GAG TCT CCC TCG GCC CCT CTC CAC AGA TGG TGC ATC CCC TGG CAG AGG CTC
    Met Glu Ser Ser Ala Pro Leu His Arg Trp Cys Ile Pro Trp Gln Arg Leu

     60          70          80          90         100         110
CTG CTC ACA GCC TCA CTT CTA ACC TTC TGG AAC CCG CCC ACC ACT GCC AAG CTC ACT
Leu Leu Thr Ala Ser Leu Leu Thr Phe Trp Asn Pro Pro Thr Thr Ala Lys Leu Thr

     120         130         140         150         160         170
ATT GAA TCC ACG CCG TTC AAT GTC GCA GAG GGG AAG GAG GTG CTT CTA CTT GTC CAC
Ile Glu Ser Thr Pro Phe Asn Val Ala Glu Gly Lys Glu Val Leu Leu Leu Val His

     180         190         200         210         220
AAT CTG CCC CAG CAT CTT TTT GGC TAC AGC TGG TAC AAA GGT GAA AGA GTG GAT GGC
Asn Leu Pro Gln His Leu Phe Gly Tyr Ser Trp Tyr Lys Gly Glu Arg Val Asp Gly

 230         240         250         260         270          280
AAC CGT CAA ATT ATA GGA TAT GTA ATA GGA ACT CAA CAA GCT ACC CCA GGG CCC GCA
Asn Arg Gln Ile Ile Gly Tyr Val Ile Gly Thr Gln Gln Ala Thr Pro Gly Pro Ala

     290         300         310         320         330         340
TAC AGT GGT CGA GAG ATA ATA TAC CCC AAT GCA TCC CTG CTG ATC CAG AAC ATC ATC
Tyr Ser Gly Arg Glu Ile Ile Tyr Pro Asn Ala Ser Leu Leu Ile Gln Asn Ile Ile

     350         360         370         380         390         400
CAG AAT GAC ACA GGA TTC TAC ACC CTA CAC GTC ATA AAG TCA GAT CTT GTG AAT GAA
Gln Asn Asp Thr Gly Phe Tyr Thr Leu His Val Ile Lys Ser Asp Leu Val Asn Glu

     410         420         430         440         450
GAA GCA ACT GGC CAG TTC CGG GTA TAC CCG GAG CTG CCC AAG CCC TCC ATC TCC AGC
Glu Ala Thr Gly Gln Phe Arg Val Tyr Pro Glu Leu Pro Lys Pro Ser Ile Ser Ser

 460         470         480         490         500         510
AAC AAC TCC AAA CCC GTG GAG GAC AAG GAT GCT GTG GCC TTC ACC TGT GAA CCT GAG
Asn Asn Ser Lys Pro Val Glu Asp Lys Asp Ala Val Ala Phe Thr Cys Glu Pro Glu

     520         530         540         550         560         570
ACT CAG GAC GCA ACC TAC CTG TGG TGG GTA AAC AAT CAG AGC CTC CCG GTC AGT CCC
Thr Gln Asp Ala Thr Tyr Leu Trp Trp Val Asn Asn Gln Ser Leu Pro Val Ser Pro

     580         590         600         610         620
AGG CTG CAG CTG TCC AAT GGC AAC AGG ACC CTC ACT CTA TTC AAT GTC ACA AGA AAT
Arg Leu Gln Leu Ser Asn Gly Asn Arg Thr Leu Thr Leu Phe Asn Val Thr Arg Asn

 630         640         650         660         670         680
GAA CAA GCA AGC TAC AAA TGT GAA ACC CAG AAC CCA GTG AGT GCC AGG CGC AGT GAT
Glu Gln Ala Ser Tyr Lys Cys Glu Thr Gln Asn Pro Val Ser Ala Arg Arg Ser Asp

     690         700         710         720         730         740
TCA GTC ATC CTG AAT GTC CTC TAT GGC CCG GAT GCC CCC ACC ATT TCC CCT CTA AAC
Ser Val Ile Leu Asn Val Leu Tyr Gly Pro Asp Ala Pro Thr Ile Ser Pro Leu Asn

     750         760         770         780         790
ACA TCT TAC AGA TCA GGG GAA AAT CTG AAC CTC TCC TGC CAC GCA GCC TCT AAC CCA
Thr Ser Tyr Arg Ser Gly Glu Asn Leu Asn Leu Ser Cys His Ala Ala Ser Asn Pro

 800         810         820         830         840         850
CCT GCA CAG TAC TCT TGG TTT GTC AAT GGG ACT TTC CAG CAA TCC ACC CAA GAG CTC
Pro Ala Gln Tyr Ser Trp Phe Val Asn Gly Thr Phe Gln Gln Ser Thr Gln Glu Leu

     860         870         880         890         900         910
TTT ATC CCC AAC ATC ACT GTG AAT AAT AGT GGA TCC TAT ACG TGC CAA GCC CAT AAC
Phe Ile Pro Asn Ile Thr Val Asn Asn Ser Gly Ser Tyr Thr Cys Gln Ala His Asn
```

EP 0 263 933 B1

```
                720            930            940            950            960            970
                 *              *              *              *              *              *
TCA GAC ACT GGC CTC AAT AGG ACC ACA GTC ACG ACG ATC ACA GTC TAT GCA GAG CCA
Ser Asp Thr Gly Leu Asn Arg Thr Thr Val Thr Thr Ile Thr Val Tyr Ala Glu Pro

                      980            990            1000           1010           1020
                       *              *              *              *              *
CCC AAA CCC TTC ATC ACC AGC AAC AAC TCC AAC CCC GTG GAG GAT GAG GAT GCT GTA
Pro Lys Pro Phe Ile Thr Ser Asn Asn Ser Asn Pro Val Glu Asp Glu Asp Ala Val

   1030           1040           1050           1060           1070           1080
    *              *              *              *              *              *
GCC TTA ACC TGT GAA CCT GAG ATT CAG AAC ACA ACC TAC CTG TGG TGG GTA AAT AAT
Ala Leu Thr Cys Glu Pro Glu Ile Gln Asn Thr Thr Tyr Leu Trp Trp Val Asn Asn

       1090       .    1100           1110           1120           1130           1140
        *              *              *              *              *              *
CAA AGC CTC CCG GTC AGT CCC AGG CTG CAG CTG TCC AAT AAC AAC AGG ACC CTC ACT
Gln Ser Leu Pro Val Ser Pro Arg Leu Gln Leu Ser Asn Asp Asn Arg Thr Leu Thr

           1150           1160           1170           1180           1190
            *              *              *              *              *
CTA CTC AGT GTC ACA AGG AAT GAT GTA GGA CCC TAT GAG TGT TGA ATC CAG AAC GAA
Leu Leu Ser Val Thr Arg Asn Asp Val Gly Pro Tyr Glu Cys Gly Ile Gln Asn Glu

1200           1210           1220           1230           1240           1250
 *              *              *              *              *              *
TTA AGT GTT GAC CAC AGC GAC CCA GTC ATC CTG AAT GTC CTC TAT GGC CCA GAC GAC
Leu Ser Val Asp His Ser Asp Pro Val Ile Leu Asn Val Leu Tyr Gly Pro Asp Asp

       1260           1270           1280           1290           1300           1310
        *              *              *              *              *              *
CCC ACC ATT TCC CCC TCA TAC ACC TAT TAC CGT CCA GGG GTG AAC CTC AGC CTC TCC
Pro Thr Ile Ser Pro Ser Tyr Thr Tyr Tyr Arg Pro Gly Val Asn Leu Ser Leu Ser

           1320           1330           1340           1350           1360
            *              *              *              *              *
TGC CAT GCA GCC TCT AAC CCA CCT GCA CAG TAT TCT TGG CTG ATT GAT GGG AAC ATC
Cys His Ala Ala Ser Asn Pro Pro Ala Gln Tyr Ser Trp Leu Ile Asp Gly Asn Ile

1370           1380           1390           1400           1410           1420
 *              *              *              *              *              *
CAG CAA CAC ACA CAA GAG CTC TTT ATC TCC AAC ATC ACT GAG AAG AAC AGC GGA CTC
Gln Gln His Thr Gln Glu Leu Phe Ile Ser Asn Ile Thr Glu Lys Asn Ser Gly Leu

       1430           1440           1450           1460           1470           1480
        *              *              *              *              *              *
TAT ACC TGC CAG GCC AAT AAC TCA GCC AGT GGC CAC AGC AGG ACT ACA GTC AAG ACA
Tyr Thr Cys Gln Ala Asn Asn Ser Ala Ser Gly His Ser Arg Thr Thr Val Lys Thr

           1490           1500           1510           1520           1530           1540
            *              *              *              *              *              *
ATC ACA GTC TCT GCG GAC GTG CCC AAG CCC TCC ATC TCC AGC AAC AAC TCC AAA CCC
Ile Thr Val Ser Ala Asp Val Pro Lys Pro Ser Ile Ser Ser Asn Asn Ser Lys Pro

           1550           1560           1570           1580           1590
            *              *              *              *              *
GTG GAG GAC AAG GAT GCT GTG GCC TTC ACC TGT GAA CCT GAG GCT CAG AAC ACA ACC
Val Glu Asp Lys Asp Ala Val Ala Phe Thr Cys Glu Pro Glu Ala Gln Asn Thr Thr

1600           1610           1620           1630           1640           1650
 *              *              *              *              *              *
TAC CTG TGG TGG GTA AAT GGT CAG AGC CTC CCA GTC AGT CCC AGG CTG CAG CTG TCC
Tyr Leu Trp Trp Val Asn Gly Gln Ser Leu Pro Val Ser Pro Arg Leu Gln Leu Ser

       1660           1670           1680           1690           1700           1710
        *              *              *              *              *              *
AAT GGC AAC AGG ACC CTC ACT CTA TTC AAT GTC ACA AGA AAT GAC GCA AGA GCC TAT
Asn Gly Asn Arg Thr Leu Thr Leu Phe Asn Val Thr Arg Asn Asp Ala Arg Ala Tyr

           1720           1730           1740           1750           1760
            *              *              *              *              *
GTA TGT GGA ATC CAG AAC TCA GTG AGT GCA AAC CGC AGT GAC CCA GTC ACC CTG GAT
Val Cys Gly Ile Gln Asn Ser Val Ser Ala Asn Arg Ser Asp Pro Val Thr Leu Asp

1770           1780           1790           1800           1810           1820
 *              *              *              *              *              *
GTC CTC TAT GGG CCG GAC ACC CCC ATC ATT TCC CCC CCA GAC TCG TCT TAC CTT TCG
Val Leu Tyr Gly Pro Asp Thr Pro Ile Ile Ser Pro Pro Asp Ser Ser Tyr Leu Ser

       1830           1840           1850           1860           1870           1880
        *              *              *              *              *              *
GGA GCG AAC CTC AAC CTC TCC TGC CAC TCG GCC TCT AAC CCA TCC CCG CAG TAT TCT
Gly Ala Asn Leu Asn Leu Ser Cys His Ser Ala Ser Asn Pro Ser Pro Gln Tyr Ser
```

46

EP 0 263 933 B1

```
        1890            1900            1910            1920            1930
         *               *               *               *               *
TGG CGT ATC AAT GGG ATA CCG CAG CAA CAC ACA CAA GTT CTC TTT ATC GCC AAA ATC
Trp Arg Ile Asn Gly Ile Pro Gln Gln His Thr Gln Val Leu Phe Ile Ala Lys Ile

  1940            1950            1960            1970            1980            1990
   *               *               *               *               *               *
ACG CCA AAT AAT AAC GGG ACC TAT GCC TGT TTT GTC TCT AAC TTG GCT ACT GGC CGC
Thr Pro Asn Asn Asn Gly Thr Tyr Ala Cys Phe Val Ser Asn Leu Ala Thr Gly Arg

     2000            2010            2020            2030            2040            2050
      *               *               *               *               *               *
AAT AAT TCC ATA GTC AAG AGC ATC ACA GTC TCT GCA TCT GGA ACT TCT CCT GGT CTC
Asn Asn Ser Ile Val Lys Ser Ile Thr Val Ser Ala Ser Gly Thr Ser Pro Gly Leu

     2060            2070            2080            2090            2100            2110
      *               *               *               *               *               *
TCA GCT GGG GCC ACT GTC GGC ATC ATG ATT GGA GTG CTG GTT GGG GTT GCT CTG ATA
Ser Ala Gly Ala Thr Val Gly Ile Met Ile Gly Val Leu Val Gly Val Ala Leu Ile

          2120            2130            2140            2150            2160
           *               *               *               *               *
TAG CAG CCC TGG TGT AGT TTC TTC ATT TCA GGA AGA CTG ACA GTT GTT TTG CTT CTT
____

2170            2180            2190            2200            2210            2220
 *               *               *               *               *               *
CCT TAA AGC ATT TGC AAC AGC TAC AGT CTA AAA TTG CTT CTT TAC CAA GGA TGT TTA

     2230            2240            2250            2260            2270            2280
      *               *               *               *               *               *
CAG AAA ATA CTC TGA CCA GAG ATC GAG ACC ATC CTA GCC AAC ATC GTG AAA CCC CAT

          2290            2300            2310            2320            2330
           *               *               *               *               *
CTC TAC TAA AAA TAC AAA AAT GAG CTG GGC TTG GTG GCG CGC ACC TGT AGT CCC AGT

2340            2350            2360            2370            2380            2390
 *               *               *               *               *               *
TAC TCG GGA GGC TGA GGC AGG AGA ATC GCT TGA ACC CGG GAG GTG GAG ATT GCA GTG

    2400            2410            2420            2430            2440            2450
     *               *               *               *               *               *
AGC CCA GAT CGC ACC ACT GCA CTC CAG TCT GGC AAC AGA GCA AGA CTC CAT CTC AAA

          2460            2470            2480            2490            2500
           *               *               *               *               *
AAG AAA AGA AAA GAA GAC TCT GAC CTG TAC TCT TGA ATA CAA GTT TCT GAT ACC ACT

2510            2520            2530            2540            2550            2560
 *               *               *               *               *               *
GCA CTG TCT GAG AAT TTC CAA AAC TTT AAT GAA CTA ACT GAC AGC TTC ATG AAA CTG

  2570            2580            2590            2600            2610            2620
   *               *               *               *               *               *
TCC ACC AAG ATC AAG CAG AGA AAA TAA TTA ATT TCA TGG GGA CTA AAT GAA CTA ATG

     2630            2640            2650            2660            2670            2680
      *               *               *               *               *               *
AGG ATA ATA TTT TCA TAA TTT TTT ATT TGA AAT TTT GCT GAT TCT TTA AAT GTC TTG

          2690            2700            2710            2720            2730
           *               *               *               *               *
TTT CCC AGA TTT CAG GAA ACT TTT TTT CTT TTA AGC TAT CCA CTC TTA CAG CAA TTT

2740            2750            2760            2770            2780            2790
 *               *               *               *               *               *
GAT AAA ATA TAC TTT TGT GAA CAA AAA TTG AGA CAT TTA CAT TTT ATC CCT ATG TGG

     2800            2810            2820            2830
      *               *               *               *
TCG CTC CAG ACT TGG GAA ACT ATT CAT GAA TAT TTA TAT TGT ATG
```

47

# EP 0 263 933 B1

CEA-C

```
        10        20        30        40        50
        '         '         '         '         '
AA TTC GGG CGG GAG CAG CCG TGC TCG AAG CGT TCC TGG AGC CCA AGC TCT CCT CCA CAG


   60        70        80        90        100       110
   '         '         '         '         '         '
GTG AAG ACA GGG CCA GCA GGA GAC ACC ATG GGG CAC CTC TCA GCC CCA CTT CAC AGA
                                     Met Gly His Leu Ser Ala Pro Leu His Arg


   120       130       110       150       160       170
   '         '         '         '         '         '
GTG CGT GTA CCC TGG CAG GGG CTT CTG CTC ACA GCC TCA CTT CTA ACC TTC TGG AAC
Val Arg Val Pro Trp Gln Gly Leu Leu Leu Thr Ala Ser Leu Leu Thr Phe Trp Asn


   180       170       200       210       220       230
   '         '         '         '         '         '
CCG CCC ACC ACT GCC CAG CTC ACT ACT GAA TCC ATG CCA TTC AAT GTT GCA GAG GGG
Pro Pro Thr Thr Ala Gln Leu Thr Thr Glu Ser Met Pro Phe Asn Val Ala Glu Gly
                    1   2   3   4   5   6   7   8   9   10  11  12  13  14


        240       250       260       270       280
        '         '         '         '         '
AAG GAG GTT CTT CTC CTT GTC CAC AAT CTG CCC CAG CAA CTT TTT GGC TAC AGC TGG
Lys Glu Val Leu Leu Leu Val His Asn Leu Pro Gln Gln Leu Phe Gly Tyr Ser Trp
15  16  17  18  19  20  21  22  23  24  25  26  27  28  29  30  31  32  33

   270       300       310       320       330       340
   '         '         '         '         '         '
TAC AAA GGG GAA AGA GTG GAT GGC AAC CGT CAA ATT GTA GGA TAT GCA ATA GGA ACT
Tyr Lys Gly Glu Arg Val Asp Gly Asn Arg Gln Ile Val Gly Tyr Ala Ile Gly Thr
34  35  36  37  38  39  40  41  42  43  44  45  46  47  48  49  50  51  52


   350       360       370       380       390       100
   '         '         '         '         '         '
CAA CAA GCT ACC CCA GGG CCC GCA AAC AGC GGT CGA GAG ACA ATA TAC CCC AAT GCA
Gln Gln Ala Thr Pro Gly Pro Ala Asn Ser Gly Arg Glu Thr Ile Tyr Pro Asn Ala
53  54  55  56  57  58  59  60  61  62  63  64  65  66  67  68  69  70  71


   110       120       130       110       150
   '         '         '         '         '
TCC CTG CTG ATC CAG AAC GTC ACC CAG AAT GAC ACA GGA TTC TAC ACC CTA CAA GTC
Ser Leu Leu Ile Gln Asn Val Thr Gln Asn Asp Thr Gly Phe Tyr Thr Leu Gln Val
72  73  74  75  76  77  78  79  80  81  82  83  84  85  86  87  88  89  90


160       470       480       190       500       510
'         '         '         '         '         '
ATA AAG TCA GAT CTT GTG AAT GAA GAA GCA ACT GGA CAG TTC CAT GTA TAC CCG GAG
Ile Lys Ser Asp Leu Val Asn Glu Glu Ala Thr Gly Gln Phe His Val Tyr Pro Glu
91  92  93  94  95  96  97  98  99  100 101 102 103 104 105 106 107 108 109


   520       530       540       550       560       570
   '         '         '         '         '         '
CTG CCC AAG CCC TCC ATC TCC AGC AAC AAC TCC AAC CCT GTG GAG GAC AAG GAT GCT
Leu Pro Lys Pro Ser Ile Ser Ser Asn Asn Ser Asn Pro Val Glu Asp Lys Asp Ala
110 111 112 113 114 115 116 117 118 119 120 121 122 123 124 125 126 127 128
```

48

```
            580         590         600         610         620
             |           |           |           |           |
GTG GCC TTC ACC TGT GAA CCT GAG ACT CAG GAC ACA ACC TAC CTG TGG TGG ATA AAC
Val Ala Phe Thr Cys Glu Pro Glu Thr Gln Asp Thr Thr Tyr Leu Trp Trp Ile Asn
129 130 131 132 133 134 135 136 137 138 139 140 141 142 143 144 145 146 147

630         640         650         660         670         680
 |           |           |           |           |           |
AAT CAG AGC CTC CCG GTC AGT CCC AGG CTG CAG CTG TCC AAT GGC AAC AGG ACC CTC
Asn Gln Ser Leu Pro Val Ser Pro Arg Leu Gln Leu Ser Asn Gly Asn Arg Thr Leu
148 149 150 151 152 153 154 155 156 157 158 159 160 161 162 163 164 165 166

        690         700         710         720         730         740
         |           |           |           |           |           |
ACT CTA CTC AGT GTC ACA AGG AAT GAC ACA GGA CCC TAT GAG TGT GAA ATA CAG AAC
Thr Leu Leu Ser Val Thr Arg Asn Asp Thr Gly Pro Tyr Glu Cys Glu Ile Gln Asn
167 168 169 170 171 172 173 174 175 176 177 178 179 180 181 182 183 184 185

        750         760         770         780         790         800
         |           |           |           |           |           |
CCA GTG AGT GCG AAC CGC AGT GAC CCA GTC ACC TTG AAT GTC ACC TAT GGC CCG GAC
Pro Val Ser Ala Asn Arg Ser Asp Pro Val Thr Leu Asn Val Thr Tyr Gly Pro Asp
186 187 188 189 190 191 192 193 194 195 196 197 198 199 200 201 202 203 204

        810         820         830         840         850
         |           |           |           |           |
ACC CCC ACC ATT TCC CCT TCA GAC ACC TAT TAC CGT CCA GGG GCA AAC CTC AGC CTC
Thr Pro Thr Ile Ser Pro Ser Asp Thr Tyr Tyr Arg Pro Gly Ala Asn Leu Ser Leu
205 206 207 208 209 210 211 212 213 214 215 216 217 218 219 220 221 222 223

860         870         880         890         900         910
 |           |           |           |           |           |
TCC TGC TAT GCA GCC TCT AAC CCA CCT GCA CAG TAC TCC TGG CTT ATC AAT GGA ACA
Ser Cys Tyr Ala Ala Ser Asn Pro Pro Ala Gln Tyr Ser Trp Leu Ile Asn Gly Thr
224 225 226 227 228 229 230 231 232 233 234 235 236 237 238 239 240 241 242

        920         930         940         950         960         970
         |           |           |           |           |           |
TTC CAG CAA AGC ACA CAA GAG CTC TTT ATC CCT AAC ATC ACT GTG AAT AAT AGT GGA
Phe Gln Gln Ser Thr Gln Glu Leu Phe Ile Pro Asn Ile Thr Val Asn Asn Ser Gly
243 244 245 246 247 248 249 250 251 252 253 254 255 256 257 258 259 260 261

        980         990         1000        1010        1020
         |           |           |           |           |
TCC TAT ACC TGC CAC GCC AAT AAC TCA GTC ACT GGC TGC AAC AGG ACC ACA GTC AAG
Ser Tyr Thr Cys His Ala Asn Asn Ser Val Thr Gly Cys Asn Arg Thr Thr Val Lys
262 263 264 265 266 267 268 269 270 271 272 273 274 275 276 277 278 279 280

1030        1040        1050        1060        1070        1080
 |           |           |           |           |           |
ACG ATC ATA GTC ACT GAG CTA AGT CCA GTA GTA GCA AAG CCC CAA ATC AAA GCC AGC
Thr Ile Ile Val Thr Glu Leu Ser Pro Val Val Ala Lys Pro Gln Ile Lys Ala Ser
281 282 283 284 285 286 287 288 289 290 291 292 293 294 295 296 297 298 299
```

```
            1090          1100         1110         1120          1130         1140
             |             |            |            |             |            |
AAG ACC ACA GTC ACA GGA GAT AAG GAC TCT GTG AAC CTG ACC TGC TCC ACA AAT GAC
Lys Thr Thr Val Thr Gly Asp Lys Asp Ser Val Asn Leu Thr Cys Ser Thr Asn Asp
300 301 302 303 304 305 306 307 308 309 310 311 312 313 314 315 316 317 318

               1150          1160         1170         1180         1190
                |             |            |            |            |
ACT GGA ATC TCC ATC CGT TGG TTC TTC AAA AAC CAG AGT CTC CCG TCC TCG GAG AGG
Thr Gly Ile Ser Ile Arg Trp Phe Phe Lys Asn Gln Ser Leu Pro Ser Ser Glu Arg
319 320 321 322 323 324 325 326 327 328 329 330 331 332 333 334 335 336 337

  1200          1210         1220         1230         1240         1250
   |             |            |            |            |            |
ATG AAG CTG TCC CAG GGC AAC ACC ACC CTC AGC ATA AAC CCT GTC AAG AGG GAG GAT
Met Lys Leu Ser Gln Gly Asn Thr Thr Leu Ser Ile Asn Pro Val Lys Arg Glu Asp
338 339 340 341 342 343 344 345 346 347 348 349 350 351 352 353 354 355 356

   1260          1270         1280         1290         1300         1310
    |             |            |            |            |            |
GCT GGG ACG TAT TGG TGT GAG GTC TTA ACC CAA TCA GTA AGA ACC AAA GCG ACC CCA
Ala Gly Thr Tyr Trp Cys Glu Val Leu Thr Gln Ser Val Arg Thr Lys Ala Thr Pro
357 358 359 360 361 362 363 364 365 366 367 368 369 370 371 372 373 374 375

   1320          1330         1340         1350         1360         1370
    |             |            |            |            |            |
TCA TGG CTG AAC GTA AAC TAT AAT GCT CTA CCA CAA GAA AAT GGC CTC TCA CCT GGG
Ser Trp Leu Asn Val Asn Tyr Asn Ala Leu Pro Gln Glu Asn Gly Leu Ser Pro Gly
376 377 378 379 380 381 382 383 384 385 386 387 388 389 390 391 392 393 394

               1380          1390         1400         1410         1420
                |             |            |            |            |
GCC ATT GCT GGC ATT GTG ATT GGA GTA GTG GCC CTG GTT GCT CTG ATA GCA GTA GCC
Ala Ile Ala Gly Ile Val Ile Gly Val Val Ala Leu Val Ala Leu Ile Ala Val Ala
395 396 397 398 399 400 401 402 403 404 405 406 407 408 409 410 411 412 413

  1430          1440         1450         1460         1470         1480
   |             |            |            |            |            |
CTG GCA TGT TTT CTG CAT TTC GGG AAG ACC GGC AGG GCA AGC GAC CAG CGT GAT CTC
Leu Ala Cys Phe Leu His Phe Gly Lys Thr Gly Arg Ala Ser Asp Gln Arg Asp Leu
414 415 416 417 418 419 420 421 422 423 424 425 426 427 428 429 430 431 432

               1490          1500         1510         1520         1530         1540
                |             |            |            |            |            |
ACA GAG CAC AAA CCC TCA GTC TCC AAC CAC ACT CAG GAC CAC TCC AAT GAC CCA CCT
Thr Glu His Lys Pro Ser Val Ser Asn His Thr Gln Asp His Ser Asn Asp Pro Pro
433 434 435 436 437 438 439 440 441 442 443 444 445 446 447 448 449 450 451

               1550          1560         1570         1580         1590
                |             |            |            |            |
AAC AAG ATG AAT GAA GTT ACT TAT TCT ACC CTG AAC TTT GAA GCC CAG CAA CCC ACA
Asn Lys Met Asn Glu Val Thr Tyr Ser Thr Leu Asn Phe Glu Ala Gln Gln Pro Thr
452 453 454 455 456 457 458 459 460 461 462 463 464 465 466 467 468 469 470

  1600          1610         1620         1630         1640         1650
   |             |            |            |            |            |
CAA CCA ACT TCA GCC TCC CCA TCC CTA ACA GCC ACA GAA ATA ATT TAT TCA GAA GTA
Gln Pro Thr Ser Ala Ser Pro Ser Leu Thr Ala Thr Glu Ile Ile Tyr Ser Glu Val
471 472 473 474 475 476 477 478 479 480 481 482 483 484 485 486 487 488 489
```

```
     1660        1670        1680        1690        1700        1710
      t           t           t           t           t           t
AAA AAG CAG TAA TGA AAC CTG TCC TGC TCA CTG CAG TGC TGA TGT ATT TCA AGT CTC
Lys Lys Gln ---
478 479 480
```

```
            1720        1730        1740        1750        1760
             t           t           t           t           t
TCA CCC TCA TCA CTA GGA GAT TCC TTT CCC CTG TAG GGG TAG AGG GGT GGG GAC AGA
```

```
1770        1780        1790        1800        1810       1820
 t           t           t           t           t          t
AAC AGC TTT CTC CTA CTC TTC CTT CCT AAT AGG CAT CTC CAG GCT GCC TGG TCA CTG
```

```
       1830        1840        1850        1860        1870        1880
        t           t           t           t           t           t
CCC CTC TCT CAG TGT CAA TAG ATG AAA GTA CAT TGG GAG TCT GTA GGA AAC CCA ACC
```

```
           1890        1900        1910        1920        1930        1940
            t           t           t           t           t           t
TTC TTG TCA TTG AAA TTT GGC AAA GCT GAC TTT GGG AAA GAG GGA CCA GAA CTT CCC.
```

```
               1950        1960        1970        1980        1990
                t           t           t           t           t
CTC CCT TCC CCT TTT CCC AAC CTG GAC TTG TTT TAA ACT TGC CTG TTC AGA GCA CTC
```

```
2000        2010        2020        2030        2040        2050
 t           t           t           t           t           t
ATT CCT TCC CAC CCC CAG TCC TGT CCT ATC ACT CTA ATT CGG ATT TGC CAT AGC CTT
```

```
     2060        2070        2080        2090        2100        2110
      t           t           t           t           t           t
GAG GTT ATG TCC TTT TCC ATT AAG TAC ATG TGC CAG GAA ACA GCG AGA GAG AGA AAG
```

```
         2120        2130        2140        2150        2160
          t           t           t           t           t
TAA ACG GCA GTA ATG CTT CTC CTA TTT CTC CAA AGC CTT GTG TGA ACT AGC AAA GAG
```

```
2170        2180        2190        2200        2210        2220
 t           t           t           t           t           t
AAG AAA ACC AAA TAT ATA ACC AAT AGT GAA ATG CCA CAG GTT TGT CCA CTG TCA GGG
```

```
         2230        2240        2250        2260        2270        2280
          t           t           t           t           t       t   t
TTG TCT ACC TGT AGG ATC AGG GTC TAA GCA CCT TGG TGC TTA GCT AGA ATA CCA CCT
```

51

EP 0 263 933 B1

```
      2290        2300        2310        2320        2330
       ‖           ‖           ‖           ‖           ‖
AAT CCT TCT GGC AAG CCT GTC TTC AGA GAA CCC ACT AGA AGC AAC TAG GAA AAA TCA

      2340        2350        2360        2370        2380        2390
       ‖           ‖           ‖           ‖           ‖           ‖
CTT GCC AAA ATC CAA GGC AAT TCC TGA TGG AAA ATG CAA AAG CAC ATA TAT GTT TTA

      2400        2410        2420
       ‖           ‖           ‖
ATA TCT TTA TGG GCT CTG TTC AAG GCA GTG CTG
```

52

CEA-D

```
           10            20           30 ~         40           50
            |             |            |            |            |
CC GGG GGA CAC GCA GGG CCA ACA GTC ACA GCA GCC CTG ACC AGA GCA TTC CTG GAG CTC


   60           70           80 ·          90          100          110
    |  .         |  .         |            |            |            |
AAG CTC TCT ACA AAG AGG TGG ACA GAG AAG ACA GCA GAG ACC ATG GGA CCC CCC TCA
                                                        Met Gly Pro Pro Ser

    120          130         .140          150          160          170
     |            |            |            |            |            |
GCC CCT CCC TGC AGA TTG CAT GTC CCC TGG AAG GAG GTC CTG CTC ACA GCC TCA CTT
Ala Pro Pro Cys Arg Leu His Val Pro Trp Lys Glu Val Leu Leu Thr Ala Ser Leu


     180          190          200          210 .        220          230
      |            |            |            |            |  ·          |
CTA ACC TTC TGG AAC CCA CCC ACC ACT GCC AAG CTC ACT ATT GAA TCC ACG CCA TTC
Leu Thr Phe Trp Asn Pro Pro Thr Thr Ala Lys Leu Thr Ile Glu Ser Thr Pro Phe
                                            1   2   3   4   5   6   7   8   9

         240          250         · 260         270          280 ·
          |            |            |            |            |
AAT GTC GCA GAG GGG AAG GAG GTT CTT CTA CTC GCC CAC AAC CTG CCC CAG AAT CGT
Asn Val Ala Glu Gly Lys Glu Val Leu Leu Leu Ala His Asn Leu Pro Gln Asn Arg
 10  11  12  13  14  15  16  17  18  19  20  21  22  23  24  25  26  27  28


   290          300          310          320          330          340
    |            |            |            |            |            |
ATT GGT TAC AGC TGG TAC AAA GGC GAA AGA GTG GAT GGC AAC AGT CTA ATT GTA GGA
Ile Gly Tyr Ser Trp Tyr Lys Gly Glu Arg Val Asp Gly Asn Ser Leu Ile Val Gly
 29  30  31  32  33  34  35  36  37  38  39  40  41  42  43  44  45  46  47


     350          360          370          380          390          400
      |            |            |            |            |            |
TAT GTA ATA GGA ACT CAA CAA GCT ACC CCA GGG CCC GCA TAC AGT GGT CGA GAG ACA
Tyr Val Ile Gly Thr Gln Gln Ala Thr Pro Gly Pro Ala Tyr Ser Gly Arg Glu Thr
 48  49  50  51  52  53  54  55  56  57  58  59  60  61  62  63  64  65  66


         410          420          430          440          450
          |            |            |            |            |
ATA TAC CCC AAT GCA TCC CTG CTG ATC CAG AAC GTC ACC CAG AAT GAC ACA GGA TTC
Ile Tyr Pro Asn Ala Ser Leu Leu Ile Gln Asn Val Thr Gln Asn Asp Thr Gly Phe
 67  68  69  70  71  72  73  74  75  76  77  78  79  80  81  82  83  84  85


   460          470          480          490          500          510
    |            |            |            |            |            |
TAC ACC CTA CAA GTC ATA AAG TCA GAT CTT GTG AAT GAA GAA GCA ACC GGA CAG TTC
Tyr Thr Leu Gln Val Ile Lys Ser Asp Leu Val Asn Glu Glu Ala Thr Gly Gln Phe
 86  87  88  89  90  91  92  93  94  95  96  97  98  99 100 101 102 103 104


     520          530          540          550          560          570
      |            |            |            |            |            |
CAT GTA TAC CCG GAG CTG CCC AAG CCC TCC ATC TCC AGC AAC AAC TCC AAC CCC GTG
His Val Tyr Pro Glu Leu Pro Lys Pro Ser Ile Ser Ser Asn Asn Ser Asn Pro Val
105 106 107 108 109 110 111 112 113 114 115 116 117 118 119 120 121 122 123
```

```
         580        590        600        610        620
          |          |          |          |          |
GAG GAC AAG GAT GCT GTG GCC TTC ACC TGT GAA CCT GAG GTT CAG AAC ACA ACC TAC
Glu Asp Lys Asp Ala Val Ala Phe Thr Cys Glu Pro Glu Val Gln Asn Thr Thr Tyr
124 135-126 127 128 129 130 131 132 133 134 135 136 137 138 139 140 141 142

   630        640        650        660        670        680
    |          |          |          |          |          |
CTG TGG TGG GTA AAT GGT CAG AGC CTC CCG GTC AGT CCC AGG CTG CAG CTG TCC AAT
Leu Trp Trp Val Asn Gly Gln Ser Leu Pro Val Ser Pro Arg Leu Gln Leu Ser Asn
143 144 145 146 147 148 149 150 151 152 153 154 155 156 157 158 159 160 161

   690        700        710        720        730        740
    |          |          |          |          |          |
GGC AAC AGG ACC CTC ACT CTA CTC AGC GTC AAA AGG AAC GAT GCA GGA TCG TAT GAA
Gly Asn Arg Thr Leu Thr Leu Leu Ser Val Lys Arg Asn Asp Ala Gly Ser Tyr Glu
162 163 164 165 166 167 168 169 170 171 172 173 174 175 176 177 178 179 180

       750        760        770        780        790        800
        |          |          |          |          |          |
TGT GAA ATA CAG AAC CCA GCG AGT GCC AAC CGC AGT GAC CCA GTC ACC CTG AAT GTC
Cys Glu Ile Gln Asn Pro Ala Ser Ala Asn Arg Ser Asp Pro Val Thr Leu Asn Val
181 182 183 184 185 186 187 188 189 190 191 192 193 194 195 196 197 198 199

       810        820        830        840        850
        |          |          |          |          |
CTC TAT GGC CCA GAT GGC CCC ACC ATT TCC CCC TCA AAG GCC AAT TAC CGT CCA GGG
Leu Tyr Gly Pro Asp Gly Pro Thr Ile Ser Pro Ser Lys Ala Asn Tyr Arg Pro Gly
200 201 202 203 204 205 206 207 208 209 210 211 212 213 214 215 216 217 218

   860        870        880        890        900        910
    |          |          |          |          |          |
GAA AAT CTG AAC CTC TCC TGC CAC GCA GCC TCT AAC CCA CCT GCA CAG TAC TCT TGG
Glu Asn Leu Asn Leu Ser Cys His Ala Ala Ser Asn Pro Pro Ala Gln Tyr Ser Trp
219 220 221 222 223 224 225 226 227 228 229 230 231 232 233 234 235 236 237

     920        930        940        950        960        970
      |          |          |          |          |          |
TTT ATC AAT GGG ACG TTC CAG CAA TCC ACA CAA GAG CTC TTT GTC CCC AAC ATC ACT
Phe Ile Asn Gly Thr Phe Gln Gln Ser Thr Gln Glu Leu Phe Ile Pro Asn Ile Thr
238 239 240 241 242 243 244 245 246 247 248 249 250 251 252 253 254 255 256

       980        990       1000       1010       1020
        |          |          |          |          |
GTG AAT AAT AGC GGA TCC TAT ATG TGC CAA GCC CAT AAC TCA GCC ACT GGC CTC AAT
Val Asn Asn Ser Gly Ser Tyr Met Cys Gln Ala His Asn Ser Ala Thr Gly Leu Asn
257 258 259 260 261 262 263 264 265 266 267 268 269 270 271 272 273 274 275

  1030       1040       1050       1060       1070       1080
    |          |          |          |          |          |
AGG ACC ACA GTC ACG ATG ATC ACA GTC TCT GGA AGT GCT CCT GTC CTC TCA GCT GTG
Arg Thr Thr Val Thr Met Ile Thr Val Ser Gly Ser Ala Pro Val Leu Ser Ala Val
276 277 278 279 280 281 282 283 284 285 286 287 288 289 290 291 292 293 294

  1090       1100       1110       1120       1130       1140
    |          |          |          |          |          |
GCC ACC GTC GGC ATC ACG ATT GGA GTG CTG GCC AGG GTG GCT CTG ATA TAG CAG CCC
Ala Thr Val Gly Ile Thr Ile Gly Val Leu Ala Arg Val Ala Leu Ile ---
295 296 297 298 299 300 301 302 303 304 305 306 307 308 309 310
```

54

```
        1150         1160        1170        1180        1190
          '                                              .
          !          !           !           !           !
     TGG TGT ATT TTC GAT ATT TCA GGA AGA CTG GCA GAT TGG ACC AGA CCC TGA ATT CTT


    1200        1210        1220        1230        1240        1250
   .  !          !           !           !         .  !           t
   ;  CTA GCT CCT CCA ATC CCA TTT TAT CCC ATG GAA CCA CTA AAA ACA AGG TCT GCT CTG


        1260        1270        1280        1290        1300        1310
          !           !           !           !           !           !
     CTC CTG AAG CCC TAT ATG CTG GAG ATG GAC AAC TCA ATG AAA ATT TAA AGG AAA AAC


        1320        1330        1340        1350        1360        1370
          !           !     .     !     .     !           !     .     !
     CCT CAG GCC TGA GGT GTG TGC CAC TCA GAG ACT TCA CCT AAC TAG AGA CAG GCA AAC
                                                                    .


              1380        1390        1400        1410        1420
      .         !           !           !           !           !
     TGC AAA CCA nnC CTC TTT CGC TTG GCA GGA TGA TGG TGT CAT TAG TAT TTC ACA AGA
                                            .                                   ..


    1430        1440        1450        1460        1470        1480
      !           !           !           !           !           !
     AGT AGC TTC AGA GGG TAA CTT AAC AGA GTA TCA GAT CTA TCT TGT CAA TCC CAA CGT


        1490        1500        1510        1520        1530        1540
          !           !           !           !           !           !  .
     TTT ACA TAA AAT AAG CGA TCC TTT AGT GCA CCC AGT GAC TGA CAT TAG CAG CAT CTT


              1550        1560        1570        1580        1590
      .         !           !           !           !           !
     ' TAA CAC AGC CGT GTG TTC AAG TGT ACA GTG GTC CTT TTC AGA GTT GGn nnT ACT CCA


    1600        1610        1620        1630        1640        1650
      !           !           !           !           !           !
     ACT GAA ATG TTA AGG AAG AAG ATA GAT CCA ATT AAA AAA AAT TAA AAC CAA TTT AAA


        1660        1670        1680        1690        1700        1710
          !           !           !           !           !           t.
     AAA AAA AAA GAA CAC AGG AGA TTC CAG TCT ACT TGA GTT AGC ATA ATA CAG AAG TCC


              1720        1730        1740        1750        1760          -
          .    !          t.          !           !           !      .
     CCT CTA CTT TAA CTT TTA CAA AAA AGT AAC CTG AAC TAA TCT GAT GTT AAC CAA TGT
```

```
       1770        1780.        1790        .1800        1810        1820
        I           I            I           I           I           I
      ATT TAT TTG TCT GGT TCT GTT TCC TTG TTC CAA TTT GAC AAA ACC CAC TGT TCT TGT


       1830        1840        1850        1860        1870        1880
        I           I           I           I           I           I.
      ATT GTA TTG CCC AGG GGG AGC TAT CAC TGT ACT TGT AGA GTG GTG CTG CTT TAA GTT


       1890        1900        1910        1920        1930        1940
        I           I           I           I           I           I
      CAT AAA TCA CAA ATA AAA GCC AAT TAG CTC TAT AAC TAA AAA AAA AAA AAA AAA AAA


       1950        1960
        I           I
      AGA AAA AAA AAG AAA AAA AAA AAA
```

**2.** Replizierbares rekombinantes Cloniervehikel mit einem Insert, die eine Nukleinsäure gemäß Anspruch 1 umfaßt.

**3.** Zelle, die mit einem replizierbaren rekombinanten Cloniervehikel gemäß Anspruch 2 transfiziert, infiziert oder injiziert ist.

**4.** Zellinien mit der ATCC-Bezeichnung ATCC 67312 und ATCC 67169.

**5.** Verfahren zum Herstellen eines zur CEA-Familie gehörenden Polypeptides, umfassend die Schritte
    (a) des Kultivierens der Zelle des Anspruches 3 oder der Zellinien des Anspruches 4 und
    (b) des Gewinnens des durch die Zelle oder Zellinie exprimierten CEA-Polypeptides.

**6.** Peptid mit einer Aminosäurensequenz, abgeleitet von den in Anspruch 1 aufgeführten Sequenzen, wobei das Peptid ausgewählt ist aus der Gruppe von
    CEA-B    (i) Aminosäurenreste 135 bis und einschließlich 386.
                (ii) Aminosäurenreste 174 bis und einschließlich 220.
                (iii) Aminosäurenreste 285 bis und einschließlich 386.
                (iv) Aminosäurenreste 174 bis und einschließlich 200.
                (v) Aminosäurenreste 208 bis und einschließlich 216.
                (vi) Aminosäurenreste 285 bis und einschließlich 290.
                (vii) Aminosäurenreste 292 bis und einschließlich 308.
                (viii) Aminosäurenreste 312 bis und einschließlich 321.
                (ix) Aminosäurenreste 360 bis und einschließlich 372.
                (x) Aminosäurenreste 380 bis und einschließlich 386.
    CEA-C    (i) Aminosäurenreste 135 bis und einschließlich 386.
                (ii) Aminosäurenreste 411 bis und einschließlich 492.
                (iii) Aminosäurenreste 174 bis und einschließlich 220.
                (iv) Aminosäurenreste 174 bis und einschließlich 200.
                (v) Aminosäurenreste 285 bis und einschließlich 389.
                (vi) Aminosäurenreste 208 bis und einschließlich 222.
                (vii) Aminosäurenreste 285 bis und einschließlich 292.
                (viii) Aminosäurenreste 295 bis und einschließlich 311.
                (ix) Aminosäurenreste 315 bis und einschließlich 328.
                (x) Aminosäurenreste 331 bis und einschließlich 345.
                (xi) Aminosäurenreste 348 bis und einschließlich 360.
                (xii) Aminosäurenreste 363 bis und einschließlich 377.

(xiii) Aminosäurenreste 383 bis und einschließlich 390.

CEA-D (i) Aminosäurenreste 135 bis und einschließlich 285.

(ii) Aminosäurenreste 168 bis und einschließlich 220.

(iii) Aminosäurenreste 168 bis und einschließlich 196.

(iv) Aminosäurenreste 210 bis und einschließlich 220 und

(v) Aminosäurenreste 264 bis und einschließlich 283.

7. Verfahren zum Herstellen eines Antikörpers, der gegen ein zur CEA-Familie gehörendes Polypeptid gerichtet ist, umfassend die Schritte

(a) des Herstellens des Polypeptides durch das Verfahren des Anspruches 5

(b) des Injizierens des Polypeptides in einen Wirt, der imstande ist, Antikörper zu erzeugen und

(c) des Gewinnens der Antikörper.

8. Verfahren zum Herstellen eines monoklonalen Antikörpers, der imstande ist, ein zur CEA-Familie gehörendes Polypeptid zu erkennen, umfassend die Schritte

(a) des Herstellens des Polypeptides durch das Verfahren von Anspruch 5

(b) des Injizierens des Polypeptides in einen Wirt, der imstande ist, Antikörper zu erzeugen

(c) des Gewinnens antikörpererzeugender Lymphocyten von dem Wirt

(d) des Verschmelzens der Lyphocyten mit einer Myelomazellinie, wobei antikörpererzeugende Hybridom-Zellen erhalten werden

(e) des Kultivierens der Hybridom-Zellen und

(f) des Gewinnens der durch die Zellen erzeugten monoklonalen Antikörper

9. Verfahren zum Herstellen eines Antikörpers, der gegen ein zu der CEA-Familie gehörendes Polypeptid gerichtet ist, umfassend die Schritte

(a) des Injizierens eines Peptides von Anspruch 6 in einen Wirt, der imstande ist, Antikörper zu erzeugen und

(b) des Gewinnens der Antikörper.

10. Verfahren zum Herstellen eines monoklonalen Antikörpers, der imstande ist, ein zu der CEA-Familie gehörendes Polypeptid zu erkennen, umfassend die Schritte

(a) des Injizierens eines Peptides von Anspruch 6 in einen Wirt, der imstande ist, Antikörpern zu erzeugen

(b) des Gewinnens antikörpererzeugender Lymphocyten von dem Wirt

(c) des Verschmelzens der Lyphocyten mit einer Myelomazellinie, wobei antikörpererzeugende Hybridom-Zellen erhalten werden

(d) des Kultivierens der Hybridom-Zellen und

(e) des Gewinnens der durch die Zellen erzeugten monoklonalen Antikörper

11. Verfahren gemäß Anspruch 7-11, wobei der Antikörper für ein bestimmtes zur CEA-Genfamilie gehörendes Peptid spezifisch ist.

12. Immunoassayverfahren zum Nachweisen eines zur CEA-Familie gehörenden Polypeptides in einer Testprobe, umfassend die Schritte

(a) des Herstellens eines gegen das Polypeptid gerichteten Antikörpers durch das Verfahren von einem der Ansprüche 7 - 11

(b) des Nachweisens des Bindens davon an in der Probe enthaltenes CEA.

13. Verfahren zum Herstellen eines Testkits, der ein zu der CEA-Familie gehörendes Polypeptid oder einen gegen das Polypeptid gerichteten Antikörper umfaßt, umfassend die Schritte

(a) des Herstellens des Polypeptides durch das Verfahren des Anspruches 5 oder des Antikörpers durch das Verfahren von irgendeinem der Ansprüche 7-11 und

(b) des Herstellens des Testkits, der das Polypeptid oder den Antikörper enthält.

14. Verfahren zum Herstellen eines pharmazeutischen Produktes, das ein zur CEA-Familie gehörendes Polypeptid oder einen gegen das Polypeptid gerichteten Antikörper umfaßt, umfassend die Schritte

(a) des Herstellens des Polypeptides durch das Verfahren des Anspruches 5 oder des Antikörpers durch das Verfahren von irgendeinem der Ansprüche 7-11 und

EP 0 263 933 B1

(b) des Herstellens des Testkits, der das Polypeptid oder den Antikörper enthält.

15. Verwendung einer Nukleinsäure gemäß Anspruch 1 in einem Nukleinsäure-Hybridisierungsassay.

16. Pharmazeutisches Produkt oder Testkit umfassend ein oder mehrere der Peptide des Anspruches 6.

**Revendications**

1. Acide nucléique isolé qui code pour un polypeptide appartenant à la famille des gènes CEA, choisi parmi les séquences répertoriées ci-dessous, ainsi que des fragments du premier cité comprenant au moins 50 bases séquentielles :

## CEA-A

```
              10          20          30          40          50
              •           •           •           •           •
GG GGT TTA CAC AAC CAC CAC CCC ATC AAA CCC TTC ATC ACC AGC AAC AAC TCC AAC CCC
   Gly Leu His Asn His His Pro Ile Lys Pro Phe Ile Thr Ser Asn Asn Ser Asn Pro

     60          70          80          90          100         110
     •           •           •           •           •           •
GTG GAG GAT GAG GAT GCT GTA GCC TTA ACC TGT GAA CCT GAG ATT CAG AAC ACA ACC
Val Glu Asp Glu Asp Ala Val Ala Leu Thr Cys Glu Pro Glu Ile Gln Asn Thr Thr

     120         130         140         150         160         170
     •           •           •           •           •           •
TAC CTG TGG TGG GTA AAT AAT CAG AGC CTC CCG GTC AGT CCC AGG CTG CAG CTG TCC
Tyr Leu Trp Trp Val Asn Asn Gln Ser Leu Pro Val Ser Pro Arg Leu Gln Leu Ser

         180         190         200         210         220         230
         •           •           •           •           •           •
AAT GAC AAC AGG ACC CTC ACT CTA CTC AGT GTC ACA AGG AAT GAT GTA GGA CCC TAT
Asn Asp Asn Arg Thr Leu Thr Leu Leu Ser Val Thr Arg Asn Asp Val Gly Pro Tyr

              240         250         260         270         280
              •           •           •           •           •
GAG TGT GGA ATC CAG AAC GAA TTA AGT GTT GAC CAC AGC GAC CCA GTC ATC CTG AAT
Glu Cys Gly Ile Gln Asn Glu Leu Ser Val Asp His Ser Asp Pro Val Ile Leu Asn

     290         300         310         320         330         340
     •           •           •           •           •           •
GTC CTC TAT GGC CCA GAC GAC CCC ACC ATT TCC CCC TCA TAC ACC TAT TAC CGT CCA
Val Leu Tyr Gly Pro Asp Asp Pro Thr Ile Ser Pro Ser Tyr Thr Tyr Tyr Arg Pro

         350         360         370         380         390         400
         •           •           •           •           •           •
GGG GTG AAC CTC AGC CTC TCC TGC CAT GCA GCC TCT AAC CCA CCT GCA CAG TAT TCT
Gly Val Asn Leu Ser Leu Ser Cys His Ala Ala Ser Asn Pro Pro Ala Gln Tyr Ser

              410         420         430         440         450
              •           •           •           •           •
TGG CTG ATT GAT GGG AAC ATC CAG CAA CAC ACA CAA GAG CTC TTT ATC TCC AAC ATC
Trp Leu Ile Asp Gly Asn Ile Gln Gln His Thr Gln Glu Leu Phe Ile Ser Asn Ile

     460         470         480         490         500         510
     •           •           •           •           •           •
ACT GAG AAG AAC AGC GGA CTC TAT ACC TGC CAG GCC AAT AAC TCA GCC AGT GGC CAC
Thr Glu Lys Asn Ser Gly Leu Tyr Thr Cys Gln Ala Asn Asn Ser Ala Ser Gly His

         520         530         540         550         560         570
         •           •           •           •           •           •
AGC AGG ACT ACA GTC AAG ACA ATC ACA GTC TCT GCG GAC GTG CCC AAG CCC TCC ATC
Ser Arg Thr Thr Val Lys Thr Ile Thr Val Ser Ala Asp Val Pro Lys Pro Ser Ile

              580         590         600         610         620
              •           •           •           •           •
TCC AGC AAC AAC TCC AAA CCC GTG GAG GAC AAG GAT GCT GTG GCC TTC CAC TGT GAA
Ser Ser Asn Asn Ser Lys Pro Val Glu Asp Lys Asp Ala Val Ala Phe His Cys Glu

630         640         650         660         670         680
•           •           •           •           •           •
CCT GAG GCT CAG AAC ACA ACC TAC CTG TGG TGG GTA AAT GGT CAG AGC CTC CCA GTC
Pro Glu Ala Gln Asn Thr Thr Tyr Leu Trp Trp Val Asn Gly Gln Ser Leu Pro Val

     690         700         710         720         730         740
     •           •           •           •           •           •
AGT CCC AGG CTG CAG CTG TCC AAT GGC AAC AGG ACC CTC ACT CTA TTC AAT GTC ACA
Ser Pro Arg Leu Gln Leu Ser Asn Gly Asn Arg Thr Leu Thr Leu Phe Asn Val Thr

         750         760         770         780         790         800
         •           •           •           •           •           •
AGA AAT GAC GCA AGA GCC TAT GTA TGT GGA ATC CAG AAC TCA GTG AGT GCA AAC CGC
Arg Asn Asp Ala Arg Ala Tyr Val Cys Gly Ile Gln Asn Ser Val Ser Ala Asn Arg

              810         820         830         840         850
              •           •           •           •           •
AGT GAC CCA GTC ACC CTG GAT GTC CTC TAT GGG CCG GAC ACC CCC ATC ATT TCC CCC
Ser Asp Pro Val Thr Leu Asp Val Leu Tyr Gly Pro Asp Thr Pro Ile Ile Ser Pro

    860
    •
CCC CC
Pro
```

CEA-B

```
            10              20              30              40              50
C ACC ATG GAG TCT CCC TCG GCC·CCT CTC CAC AGA TGG TGC ATC CCC TGG CAG AGG .CTC
    Met Glu Ser Pro Ser Ala Pro Leu His Arg Trp Cys Ile Pro Trp Gln Arg Leu

      60              70              80              90             100             110
CTG CTC ACA GCC TCA CTT CTA ACC TTC TGG AAC CCG CCC ACC ACT GCC AAG CTC ACT
Leu Leu Thr Ala Ser Leu Leu Thr Phe Trp Asn Pro Pro Thr Thr Ala Lys Leu Thr
                                                                      1  2  3
      120             130             140             150             160            170
ATT GAA TCC ACG CCG TTC AAT GTC GCA GAG GGG AAG GAG GTG CTT CTA CTT GTC CAC
Ile Glu Ser Thr Pro Phe Asn Val Ala Glu Gly Lys Glu Val Leu Leu Leu Val His

          180             190             200             210             220
AAT CTG CCC CAG CAT CTT TTT GGC TAC AGC TGG TAC AAA GGT GAA AGA GTG GAT GGC
Asn Leu Pro Gln His Leu Phe Gly Tyr Ser Trp Tyr Lys Gly Glu Arg Val Asp Gly
```

```
         230          240          250    .    260          270          280    .
          •            •            •            •            •            •
AAC CGT CAA ATT ATA GGA TAT GTA ATA GGA ACT CAA CAA GCT ACC CCA GGG CCC GCA
Asn Arg Gln Ile Ile Gly Tyr Val Ile Gly Thr Gln Gln Ala Thr Pro Gly Pro Ala
         290          300          310          320          330    .    340
          •            •            •            •            •            •
TAC AGT GGT CGA GAG ATA ATA TAC CCC AAT GCA TCC CTG CTG ATC CAG AAC ATC ATC
Tyr Ser Gly Arg Glu Ile Ile Tyr Pro Asn Ala Ser Leu Leu Ile Gln Asn Ile Ile

         350  .       360          370          380          390    .    400
          •            •            •            •            •            •
CAG AAT GAC ACA GGA TTC TAC ACC CTA CAC GTC ATA AAG TCA AAT CTT GTG AAT GAA
Gln Asn Asp Thr Gly Phe Tyr Thr Leu His Val Ile Lys Ser Asn Leu Val Asn Glu

              410          420          430          440          450
               •            •            •            •            •
GAA GCA ACT GGC CAG TTC CGG GTA TAC CCG GAG CTG CCC AAG CCC TCC ATC TCC AGC
Glu Ala Thr Gly Gln Phe Arg Val Tyr Pro Glu Leu Pro Lys Pro Ser Ile Ser Ser

      460          470          480          490          500          510
       •            •            •            •            •            •
AAC AAC TCC AAA CCC GTG GAG GAC AAG GAT GCT GTG GCC TTC ACC TGT GAA CCT GAG
Asn Asn Ser Lys Pro Val Glu Asp Lys Asp Ala Val Ala Phe Thr Cys Glu Pro Glu

         520          530   .      540          550          560          570
          •            •            •            •            •            •
ACT CAG GAC GCA ACC TAC CTG TGG TGG GTA AAC AAT CAG AGC CTC CCG GTC AGT CCC
Thr Gln Asp Ala Thr Tyr Leu Trp Trp Val Asn Asn Gln Ser Leu Pro Val Ser Pro

              580          590          600          610          620
               •            •            •            •            •
AGG CTG CAG CTG TCC AAT GGC AAC AGG ACC CTC ACT CTA TTC AAT GTC ACA AGA AAT
Arg Leu Gln Leu Ser Asn Gly Asn Arg Thr Leu Thr Leu Phe Asn Val Thr Arg Asn

      630          640          650          660          670          680
       •            •            •            •            •            •
GAA CAA GCA AGC TAC AAA TGT GAA ACC CAG AAC CCA GTG AGT GCC AGG CGC AGT GAT
Glu Gln Ala Ser Tyr Lys Cys Gly Thr Gln Asn Pro Val Ser Ala Arg Arg Ser Asp

         690          700          710          720          730          740
          •            •            •            •            •            •
TCA GTC ATC CTG AAT GTC CTC TAT GGC CCG GAT GCC CCC ACC ATT TCC CCT CTA AAC
Ser Val Ile Leu Asn Val Leu Tyr Gly Pro Asp Ala Pro Thr Ile Ser Pro Leu Asn

         750          760          770          780          790
          •            •            •            •            •
ACA TCT TAC AGA TCA GGG GAA AAT CTG AAC CTC TCC TGC CAC GCA GCC TCT AAC CCA
Thr Ser Tyr Arg Ser Gly Glu Asn Leu Asn Leu Ser Cys His Ala Ala Ser Asn Pro

      800          810          820          830          840          850
       •            •            •            •            •            •
CCT GCA CAG TAC TCT TGG TTT GTC AAT GGG ACT TTC CAG CAA TCC ACC CAA GAG CTC
Pro Ala Gln Tyr Ser Trp Phe Val Asn Gly Thr Phe Gln Gln Ser Thr Gln Glu Leu

         860          870          880          890          900          910
          •            •            •            •            •            •
TTT ATC CCC AAC ATC ACT GTG AAT AAT AGT GGA TCC TAT ACG TGC CAA GCC CAT AAC
Phe Ile Pro Asn Ile Thr Val Asn Asn Ser Gly Ser Tyr Thr Cys Gln Ala His Asn

         920          930          940          950          960          970
          •            •            •            •            •            •
TCA GAC ACT GGC CTC AAT AGG ACC ACA GTC ACG ACG ATC ACA GTC TAT GCA GAG CCA
Ser Asp Thr Gly Leu Asn Arg Thr Thr Val Thr Thr Ile Thr Val Tyr Ala Glu Pro

              980          990          1000         1010         1020
               •            •            •            •            •
CCC AAA CCC TTC ATC ACC AGC AAC AAC TCC AAC CCC GTG GAG GAT GAG GAT GCT GTA
Pro Lys Pro Phe Ile Thr Ser Asn Asn Ser Asn Pro Val Glu Asp Glu Asp Ala Val
                                                                      Val
      1030         1040         1050         1060         1070         1080
       •            •            •            •            •            •
GCC TTA ACC TGT GAA CCT GAG ATT CAG AAC ACA ACC TAC CTG TGG TGG GTA AAT AAT
Ala Leu Thr Cys Glu Pro Glu Ile Gln Asn Thr Thr Tyr Leu Trp Trp Val Asn Asn

      1090         1100         1110         1120         1130         1140
       •            •            •            •            •            •
CAG AGC CTC CCG GTC AGT CCC AGG CTG CAG CTG TCC AAT GAC AAC AGG ACC CTC ACT
Gln Ser Leu Pro Val Ser Pro Arg Leu Gln Leu Ser Asn Asp Asn Arg Thr Leu Thr

         1150         1160         1170         1180         1190
          •            •            •            •            •
CTA CTC AGT GTC ACA AGG AAT GAT GTA GGA CCC TAT GAG TGT GGA ATC CAG AAC GAA
Leu Leu Ser Val Thr Arg Asn Asp Val Gly Pro Tyr Glu Cys Gly Ile Gln Asn Glu

   1200         1210         1220         1230         1240         1250
    •            •            •            •            •            •
TTA AGT GTT GAC CAC AGC GAC CCA GTC ATC CTG AAT GTC CTC TAT GGC CCA GAC AAC
Leu Ser Val Asp His Ser Asp Pro Val Ile Leu Asn Val Leu Tyr Gly Pro Asp Asn
```

```
     1260          1270          1280          1290          1300          1310
      •             •             o             •             •             •
CCC ACC ATT TCC CCC TCA TAC ACC TAT TAC CGT CCA GGG GTG AAC CTC AGC CTC TCC
Pro Thr Ile Ser Pro Ser Tyr Thr Tyr Tyr Arg Pro Gly Val Asn Leu Ser Leu Ser

           1320          1330          1340          1350          1360
            •             •             •             •             •
TGC CAT GCA GCC TCT AAC CCA CCT GCA CAG TAT TCT TGG CTG ATT GAT GGG AAC ATC
Cys His Ala Ala Ser Asn Pro Pro Ala Gln Tyr Ser Trp Leu Ile Asp Gly Asn Ile

  1370          1380          1390          1400          1410          1420
   •             •             •             •             •             •
CAG CAA CAC ACA CAA GAG CTC TTT ATC TCC AAC ATC ACT GAG AAG AAC AGC GGA CTC
Gln Gln His Thr Gln Glu Leu Phe Ile Ser Asn Ile Thr Glu Lys Asn Ser Gly Leu

     1430          1440          1450          1460          1470          1480
      •             •             •             •             •             •
TAT ACC TGC CAG GCC AAT AAC TCA GCC AGT GGC CAC AGC AGG ACT ACA GTC AAG ACA
Tyr Thr Cys Gln Ala Asn Asn Ser Ala Ser Gly His Ser Arg Thr Thr Val Lys Thr

        1490          1500          1510          1520          1530          1540
         •             •             •             •             •             •
ATC ACA GTC TCT GCG GAC GTG CCC AAG CCC TCC ATC TCC AGC AAC AAC TCC AAA CCC
Ile Thr Val Ser Ala Asp Val Pro Lys Pro Ser Ile Ser Ser Asn Asn Ser Lys Pro

           1550          1560          1570          1580          1590
            •             •             •             •             •
GTG GAG GAC AAG GAT GCT GTG GCC TTC ACC TGT GAA CCT GAG GCT CAG AAC ACA ACC
Val Glu Asp Lys Asp Ala Val Ala Phe Thr Cys Glu Pro Glu Ala Gln Asn Thr Thr

  1600          1610          1620          1630          1640          1650
   •             •             •             •             •             •
TAC CTG TGG TGG GTA AAT GGT CAG AGC CTC CCA GTC AGT CCC AGG CTG CAG CTG TCC
Tyr Leu Trp Trp Val Asn Gly Gln Ser Leu Pro Val Ser Pro Arg Leu Gln Leu Ser

     1660          1670          1680          1690          1700          1710
      •             •             •             •             •             •
AAT GGC AAC AGG ACC CTC ACT CTA TTC AAT GTC ACA AGA AAT GAC GCA AGA GCC TAT
Asn Gly Asn Arg Thr Leu Thr Leu Phe Asn Val Thr Arg Asn Asp Ala Arg Ala Tyr

           1720          1730          1740          1750          1760
            •             •             •             •             •
GTA TGT GGA ATC CAG AAC TCA GTG AGT GCA AAC CGC AGT GAC CCA GTC ACC CTG GAT
Val Cys Gly Ile Gln Asn Ser Val Ser Ala Asn Arg Ser Asp Pro Val Thr Leu Asp

  1770          1780          1790          1800          1810          1820
   •             •             •             •             •             •
GTC CTC TAT GGG CCG GAC ACC CCC ATC ATT TCC CCC CCA GAC TCG TCT TAC CTT TCG
Val Leu Tyr Gly Pro Asp Thr Pro Ile Ile Ser Pro Pro Asp Ser Ser Tyr Leu Ser

     1830          1840          1850          1860          1870          1880
      •             •             •             •             •             •
GGA GCG AAC CTC AAC CTC TCC TGC CAC TCG GCC TCT AAC CCA TCC CCG CAG TAT TCT
Gly Ala Asn Leu Asn Leu Ser Cys His Ser Ala Ser Asn Pro Ser Pro Gln Tyr Ser

        1890          1900          1910          1920          1930
         •             •             •             •             •
TGG CGT ATC AAT GGG ATA CCG CAG CAA CAC ACA CAA GTT CTC TTT ATC GCC AAA ATC
Trp Arg Ile Asn Gly Ile Pro Gln Gln His Thr Gln Val Leu Phe Ile Ala Lys Ile

  1940          1950          1960          1970          1980          1990
   •             •             •             •             •             •
ACG CCA AAT AAT AAC GGG ACC TAT GCC TGT TTT GTC TCT AAC TTG GCT ACT GGC CGC
Thr Pro Asn Asn Asn Gly Thr Tyr Ala Cys Phe Val Ser Asn Leu Ala Thr Gly Arg

     2000          2010          2020          2030          2040          2050
      •             •             •             •             •             •
AAT AAT TCC ATA GTC AAG AGC ATC ACA GTC TCT GCA TCT GGA ACT TCT CCT GGT CTC
Asn Asn Ser Ile Val Lys Ser Ile Thr Val Ser Ala Ser Gly Thr Ser Pro Gly Leu

        2060          2070          2080          2090          2100          2110
         •             •             •             •             •             •
TCA GCT GGG GCC ACT GTC GGC ATC ATG ATT GGA GTG CTG GTT GGG GTT GCT CTG ATA
Ser Ala Gly Ala Thr Val Gly Ile Met Ile Gly Val Leu Val Gly Val Ala Leu Ile

           2120          2130          2140          2150          2160
            •             •             •             •             •
TAG CAG CCC TGG TGT AGT TTC TTC ATT TCA GGA AGA CTG ACA GTT GTT TTG CTT CTT
```

```
      2170          2180          2190          2200          2210          2220
CCT TAA AGC ATT TGC AAC AGC TAC AGT CTA AAA TTG CTT CTT TAC CAA GGA TAT TTA

       2230          2240          2250          2260          2270          2280
CAG AAA ATA CTC TGA CCA GAG ATC GAG ACC ATC CTA GCC AAC ATC GTG AAA CCC CAT

              2290          2300          2310          2320          2330
CTC TAC TAA AAA TAC AAA AAT GAG CTG GGC TTG GTG GCG CGC ACC TGT AGT CCC AGT

  2340          2350          2360          2370          2380          2390
TAC TCG GGA GGC TGA GGC AGG AGA ATC GCT TGA ACC CGG GAG GTG GAG ATT GCA GTG

       2400          2410          2420          2430          2440          2450
AGC CCA GAT CGC ACC ACT GCA CTC CAG TCT GGC AAC AGA GCA AGA CTC CAT CTC AAA

            2460          2470          2480          2490          2500
AAG AAA AGA AAA GAA GAC TCT GAC CTG TAC TCT TGA ATA CAA GTT TCT GAT ACC ACT

  2510          2520          2530          2540          2550          2560
GCA CTG TCT GAG AAT TTC CAA AAC TTT AAT GAA CTA ACT GAC AGC TTC ATG AAA CTG

     2570          2580          2590          2600          2610          2620
TCC ACC AAG ATC AAG CAG AGA AAA TAA TTA ATT TCA TGG GGA CTA AAT GAA CTA ATG

            2630          2640          2650          2660          2670          2680
AGG ATA ATA TTT TCA TAA TTT TTT ATT TGA AAT TTT GCT GAT TCT TTA AAT GTC TTG

              2690          2700          2710          2720          2730
TTT CCC AGA TTT CAG GAA ACT TTT TTT CTT TTA AGC TAT CCA CTC TTA CAG CAA TTT

   2740          2750          2760          2770          2780          2790
GAT AAA ATA TAC TTT TGT GAA CAA AAA TTG AGA CAT TTA CAT TTT ATC CCT ATG TGG

       2800          2810          2820          2830
TCG CTC CAG ACT TGG GAA ACT ATT CAT CAA TAT TTA TAT TGT ATG
```

63

CEA-C

```
                  10           20          30          40          50
                  '            '           '           '           '
AA TTC GGG CGG GAG CAG CCG TGC TCG AAG CGT TCC TGG AGC CCA AGC TCT CCT CCA CAG


60           70          80          90          100         110
'            '           '           '           '           '
GTG AAG ACA GGG CCA GCA GGA GAC ACC ATG GGG CAC CTC TCA GCC CCA CTT CAC AGA
                                        Met Gly His Leu Ser Ala Pro Leu His Arg


120          130         140         150         160         170
'            '           '           '           '           '
GTG CGT GTA CCC TGG CAG GGG CTT CTG CTC ACA GCC TCA CTT CTA ACC TTC TGG AAC
Val Arg Val Pro Trp Gln Gly Leu Leu Leu Thr Ala Ser Leu Leu Thr Phe Trp Asn


180          190         200         210         220         230
'            '           '           '           '           '
CCG CCC ACC ACT GCC CAG CTC ACT ACT GAA TCC ATG CCA TTC AAT GTT GCA GAG GGG
Pro Pro Thr Thr Ala Gln Leu Thr Thr Glu Ser Met Pro Phe Asn Val Ala Glu Gly


             240         250         260         270         280
             '           '           '           '           '
AAG GAG GTT CTT CTC CTT GTC CAC AAT CTG CCC CAG CAA CTT TTT GGC TAC AGC TGG
Lys Glu Val Leu Leu Leu Val His Asn Leu Pro Gln Gln Leu Phe Gly Tyr Ser Trp


290          300         310         320         330         340
'            '           '           '           '           '
TAC AAA GGG GAA AGA GTG GAT GGC AAC CGT CAA ATT GTA GGA TAT GCA ATA GGA ACT
Tyr Lys Gly Glu Arg Val Asp Gly Asn Arg Gln Ile Val Gly Tyr Ala Ile Gly Thr


             350         360         370         380         390         400
             '           '           '           '           '           '
CAA CAA GCT ACC CCA GGG CCC GCA AAC AGC GGT CGA GAG ACA ATA TAC CCC AAT GCA
Gln Gln Ala Thr Pro Gly Pro Ala Asn Ser Gly Arg Glu Thr Ile Tyr Pro Asn Ala


             410         420         430         440         450
             '           '           '           '           '
TCC CTG CTG ATC CAG AAC GTC ACC CAG AAT GAC ACA GGA TTC TAC ACC CTA CAA GTC
Ser Leu Leu Ile Gln Asn Val Thr Gln Asn Asp Thr Gly Phe Tyr Thr Leu Gln Val


460          470         480         490         500         510
'            '           '           '           '           '
ATA AAG TCA GAT CTT GTG AAT GAA GAA GCA ACT GGA CAG TTC CAT GTA TAC CCG GAG
Ile Lys Ser Asp Leu Val Asn Glu Glu Ala Thr Gly Gln Phe His Val Tyr Pro Glu


520          530         540         550         560         570
'            '           '           '           '           '
CTG CCC AAG CCC TCC ATC TCC AGC AAC AAC TCC AAC CCT GTG GAG GAC AAG GAT GCT
Leu Pro Lys Pro Ser Ile Ser Ser Asn Asn Ser Asn Pro Val Glu Asp Lys Asp Ala
```

```
        580         590         600         610         620
         ¡           ¡           ¡           ¡           ¡
GTG GCC TTC ACC TGT GAA CCT GAG ACT CAG GAC ACA ACC·TAC CTG TGG TGG ATA AAC
Val Ala Phe Thr Cys Glu Pro Glu Thr Gln Asp Thr Thr Tyr Leu Trp Trp Ile Asn


        630         640         650         660         670         680
         ¡           ¡           ¡           ¡           ¡           ¡
AAT CAG AGC CTC CCG GTC AGT CCC AGG CTG CAG CTG TCC AAT GGC AAC AGG ACC CTC
Asn Gln Ser Leu Pro Val Ser Pro Arg Leu Gln Leu Ser Asn Gly Asn Arg Thr Leu


        690         700         710         720         730         740
         ¡           ¡           ¡           ¡           ¡           ¡
ACT CTA CTC AGT GTC ACA AGG AAT GAC ACA GGA CCC TAT GAG TGT GAA ATA CAG AAC
Thr Leu Leu Ser Val Thr Arg Asn Asp Thr Gly Pro Tyr Glu Cys Glu Ile Gln Asn


        750         760         770         780         790         800
         ¡           ¡           ¡           ¡           ¡           ¡
CCA GTG AGT GCG AAC CGC AGT GAC CCA GTC ACC TTG AAT GTC ACC TAT GGC CCG GAC
Pro Val Ser Ala Asn Arg Ser Asp Pro Val Thr Leu Asn Val Thr Tyr Gly Pro Asp


        810         820         830         840         850
         ¡           ¡           ¡           ¡           ¡
ACC CCC ACC ATT TCC CCT TCA GAC ACC TAT TAC CGT CCA GGG GCA AAC CTC AGC CTC
Thr Pro Thr Ile Ser Pro Ser Asp Thr Tyr Tyr Arg Pro Gly Ala Asn Leu Ser Leu


        860         870         880         890         900        910
         ¡           ¡           ¡           ¡           ¡          ¡
TCC TGC TAT GCA GCC TCT AAC CCA CCT GCA CAG TAC TCC TGG CTT ATC AAT GGA ACA
Ser Cys Tyr Ala Ala Ser Asn Pro Pro Ala Gln Tyr Ser Trp Leu Ile Asn Gly Thr


        920         930         940         950         960        970
         ¡           ¡           ¡           ¡           ¡          ¡
TTC CAG CAA AGC ACA CAA GAG CTC TTT ATC CCT AAC ATC ACT GTG AAT AAT AGT GGA
Phe Gln Gln Ser Thr Gln Glu Leu Phe Ile Pro Asn Ile Thr Val Asn Asn Ser Gly


        980         990        1000        1010        1020
         ¡           ¡           ¡           ¡           ¡
TCC TAT ACC TGC CAC GCC AAT AAC TCA GTC ACT GGC TGC AAC AGG ACC ACA GTC AAG
Ser Tyr Thr Cys His Ala Asn Asn Ser Val Thr Gly Cys Asn Arg Thr Thr Val Lys


       1030        1040        1050        1060        1070        1080
         ¡           ¡           ¡           ¡           ¡           ¡
ACG ATC ATA GTC ACT GAG CTA AGT CCA GTA GTA GCA AAG CCC CAA ATC AAA GCC AGC
Thr Ile Ile Val Thr Glu Leu Ser Pro Val Val Ala Lys Pro Gln Ile Lys Ala Ser
```

```
        1090          1100          1110          1120·         1130          1140
         '             '             '             '             '             '
AAG ACC ACA GTC ACA GGA GAT AAG GAC TCT GTG AAC CTG ACC TGC TCC ACA AAT GAC
Lys Thr Thr Val Thr Gly Asn Lys Asp Ser Val Asn Leu Thr Cys Ser Thr Asn Asp


          1150          1160          1170          1180          1190
           '             '             '             '             '
ACT GGA ATC TCC ATC CGT TGG TTC TTC AAA AAC CAG AGT CTC CCG TCC TCG GAG AGG
Thr Gly Ile Ser Ile Arg Trp Phe Phe Lys Asn Gln Ser Leu Pro Ser Ser Glu Arg


   1200          1210          1220          1230          1240          1250
    '             '             '             '             '             '
ATG AAG CTG TCC CAG GGC AAC ACC ACC CTC AGC ATA AAC CCT GTC AAG AGG GAG GAT
Met Lys Leu Ser Gln Gly Asn Thr Thr Leu Ser Ile Asn Pro Val Lys Arg Glu Asp


     1260          1270          1280          1290          1300          1310
      '             '             '             '             '             '
GCT GGG ACG TAT TGG TGT GAG GTC TTA ACC CAA TCA GTA AGA ACC AAA GCG ACC CCA
Ala Gly Thr Tyr Trp Cys Glu Val Leu Thr Gln Ser Val Arg Thr Lys Ala Thr Pro


       1320          1330          1340          1350          1360          1370
        '             '             '             '             '             '
TCA TGG CTG AAC GTA AAC TAT AAT GCT CTA CCA CAA GAA AAT GGC CTC TCA CCT GGG
Ser Trp Leu Asn Val Asn Tyr Asn Ala Leu Pro Gln Glu Asn Gly Leu Ser Pro Gly


         1380          1390          1400          1410          1420
          '             '             '             '             '
GCC ATT GCT GGC ATT GTG ATT GGA GTA GTG GCC CTG GTT GCT CTG ATA GCA GTA GCC
Ala Ile Ala Gly Ile Val Ile Gly Val Val Ala Leu Val Ala Leu Ile Ala Val Ala


   1430          1440          1450          1460          1470          1480
    '             '             '             '             '             '
CTG GCA TGT TTT CTG CAT TTC GGG AAG ACC GGC AGG GCA AGC GAC CAG CGT GAT CTC
Leu Ala Cys Phe Leu His Phe Gly Lys Thr Gly Arg Ala Ser Asp Gln Arg Asp Leu


         1490          1500          1510          1520          1530          1540
          '             '             '             '             '             '
ACA GAG CAC AAA CCC TCA GTC TCC AAC CAC ACT CAG GAC CAC TCC AAT GAC CCA CCT
Thr Glu His Lys Pro Ser Val Ser Asn His Thr Gln Asp His Ser Asn Asp Pro Pro


         1550          1560          1570          1580          1590
          '             '             '             '             '
AAC AAG ATG AAT GAA GTT ACT TAT TCT ACC CTG AAC TTT GAA GCC CAG CAA CCC ACA
Asn Lys Met Asn Glu Val Thr Tyr Ser Thr Leu Asn Phe Glu Ala Gln Gln Pro Thr


   1600          1610          1620          1630          1640          1650
    '             '             '             '             '             '
CAA CCA ACT TCA GCC TCC CCA TCC CTA ACA GCC ACA GAA ATA ATT TAT TCA GAA GTA
Gln Pro Thr Ser Ala Ser Pro Ser Leu Thr Ala Thr Glu Ile Ile Tyr Ser Glu Val
```

66

```
      1660          1670          1680          1690          1700          1710
       '             '             '             '             '             '
AAA AAG CAG TAA TGA AAC CTG TCC TGC TCA CTG CAG TGC TGA TGT ATT TCA AGT CTC
Lys Lys Gln ---

              1720          1730          1740          1750          1760
               '             '             '             '             '
      TCA CCC TCA TCA CTA GGA GAT TCC TTT CCC CTG TAG GGG TAG AGG GGT GGG GAC AGA


1770          1780          1790          1800          1810          1820
 '             '             '             '             '             '
AAC AGC TTT CTC CTA CTC TTC CTT CCT AAT AGG CAT CTC CAG GCT GCC TGG TCA CTG


      1830          1840          1850          1860          1870          1880
       '             '             '             '             '             '
CCC CTC TCT CAG TGT CAA TAG ATG AAA GTA CAT TGG GAG TCT GTA GGA AAC CCA ACC


              1890          1900          1910          1920          1930          1940
               '             '             '             '             '             '
      TTC TTG TCA TTG AAA TTT GGC AAA GCT GAC TTT GGG AAA GAG GGA CCA GAA CTT CCC.


              1950          1960          1970          1980          1990
               '             '             '             '             '
CTC CCT TCC CCT TTT CCC AAC CTG GAC TTG TTT TAA ACT TGC CTG TTC AGA GCA CTC


2000          2010          2020          2030          2040          2050
 '             '             '             '             '             '
ATT CCT TCC CAC CCC CAG TCC TGT CCT ATC ACT CTA ATT CGG ATT TGC CAT AGC CTT


      2060          2070          2080          2090          2100          2110
       '             '             '             '             '             '
GAG GTT ATG TCC TTT TCC ATT AAG TAC ATG TGC CAG GAA ACA GCG AGA GAG AGA AAG


              2120          2130          2140          2150          2160
               '             '             '             '             '
      TAA ACG GCA GTA ATG CTT CTC CTA TTT CTC CAA AGC CTT GTG TGA ACT AGC AAA GAG


2170          2180          2190          2200          2210          2220
 '             '             '             '             '             '
AAG AAA ACC AAA TAT ATA ACC AAT AGT GAA ATG CCA CAG GTT TGT CCA CTG TCA GGG


      2230          2240          2250          2260          2270          2280
       '             '             '             '             '           '   '
      TTG TCT ACC TGT AGG ATC AGG GTC TAA GCA CCT TGG TGC TTA GCT AGA ATA CCA CCT
```

67

```
        2290          2300         2310        2320        2330
          ,            ,            ,           ,           ,
     AAT CCT TCT GGC AAG CCT GTC TTC AGA GAA CCC ACT AGA AGC AAC TAG GAA AAA TCA

      2340         2350         2360        2370        2380        2390
        ,            ,            ,           ,           ,           ,
     CTT GCC AAA ATC CAA GGC AAT TCC TGA TGG AAA ATG CAA AAG CAC ATA TAT GTT TTA

        2400         2410        2420
          ,            ,           ,
     ATA TCT TTA TGG GCT CTG TTC AAG GCA GTG CTG
```

## CEA-D

```
              10        20        30        40        50
               .         .         .         .         .
CC GGG GGA CAC GCA GGG CCA ACA GTC ACA GCA GCC CTG ACC ACA GCA TTC CTG GAG CTC


              60        70        80        90       100       110
               .         .         .         .         .         .
AAG CTC TCT ACA AAG AGG TGG ACA GAG AAG ACA GCA GAG ACC ATG GGA CCC CCC TCA
                                                             Met Gly Pro Pro Ser


             120       130       140       150       160       170
               .         .         .         .         .         .
GCC CCT CCC TGC AGA TTG CAT GTC CCC TGG AAG GAG GTC CTG CTC ACA GCC TCA CTT
Ala Pro Pro Cys Arg Leu His Val Pro Trp Lys Glu Val Leu Leu Thr Ala Ser Leu


             180       190       200       210       220       230
               .         .         .         .         .         .
CTA ACC TTC TGG AAC CCA CCC ACC ACT GCC AAG CTC ACT ATT GAA TCC ACG CCA TTC
Leu Thr Phe Trp Asn Pro Pro Thr Thr Ala Lys Leu Thr Ile Glu Ser Thr Pro Phe


             240       250       260       270       280
               .         .         .         .         .
AAT GTC GCA GAG GGG AAG GAG GTT CTT CTA CTC GCC CAC AAC CTG CCC CAG AAT CGT
Asn Val Ala Glu Gly Lys Glu Val Leu Leu Leu Ala His Asn Leu Pro Gln Asn Arg


             290       300       310       320       330       340
               .         .         .         .         .         .
ATT GGT TAC AGC TGG TAC AAA GGC GAA AGA GTG GAT GGC AAC AGT CTA ATT GTA GGA
Ile Gly Tyr Ser Trp Tyr Lys Gly Glu Arg Val Asp Gly Asn Ser Leu Ile Val Gly


             350       360       370       380       390       400
               .         .         .         .         .         .
TAT GTA ATA GGA ACT CAA CAA GCT ACC CCA GGG CCC GCA TAC AGT GGT CGA GAG ACA
Tyr Val Ile Gly Thr Gln Gln Ala Thr Pro Gly Pro Ala Tyr Ser Gly Arg Glu Thr


             410       420       430       440       450
               .         .         .         .         .
ATA TAC CCC AAT GCA TCC CTG CTG ATC CAG AAC GTC ACC CAG AAT GAC ACA GGA TTC
Ile Tyr Pro Asn Ala Ser Leu Leu Ile Gln Asn Val Thr Gln Asn Asp Thr Gly Phe


             460       470       480       490       500       510
               .         .         .         .         .         .
TAC ACC CTA CAA GTC ATA AAG TCA GAT CTT GTG AAT GAA GAA GCA ACC GGA CAG TTC
Tyr Thr Leu Gln Val Ile Lys Ser Asp Leu Val Asn Glu Glu Ala Thr Gly Gln Phe


             520       530       540       550       560       570
               .         .         .         .         .         .
CAT GTA TAC CCA GAG CTG CCC AAG CCC TCC ATC TCC AGC AAC AAC TCC AAC CCC GTG
His Val Tyr Pro Glu Leu Pro Lys Pro Ser Ile Ser Ser Asn Asn Ser Asn Pro Val
```

```
              580         590         600         610         620
               '           '           '           '           '
GAG AAC AAG GAT GCT GTG GCC TTC ACC TGT GAA CCT GAG GTT CAG AAC ACA ACC TAC
Glu Asn Lys Asp Ala Val Ala Phe Thr Cys Glu Pro Glu Val Gln Asn Thr Thr Tyr

     630         640         650         660         670         680
      '           '           '           '           '           '
CTG TGG TGG GTA AAT GGT CAG AGC CTC CCG GTC AGT CCC AGG CTG CAG CTG TCC AAT
Leu Trp Trp Val Asn Gly Gln Ser Leu Pro Val Ser Pro Arg Leu Gln Leu Ser Asn

        690         700         710         720         730         740
         '           '           '           '           '           '
GGC AAC AGG ACC CTC ACT CTA CTC AGC GTC AAA AGG AAC GAT GCA GGA TCG TAT GAA
Gly Asn Arg Thr Leu Thr Leu Leu Ser Val Lys Arg Asn Asp Ala Gly Ser Tyr Glu

           750         760         770         780         790         800
            '           '           '           '           '           '
TGT GAA ATA CAG AAC CCA GCG AGT GCC AAC CGC AGT GAC CCA GTC ACC CTG AAT GTC
Cys Glu Ile Gln Asn Pro Ala Ser Ala Asn Arg Ser Asp Pro Val Thr Leu Asn Val

              810         820         830         840         850
               '           '           '           '           '
CTC TAT GGC CCA GAT GGC CCC ACC ATT TCC CCC TCA AAG GCC AAT TAC CGT CCA GGG
Leu Tyr Gly Pro Asp Gly Pro Thr Ile Ser Pro Ser Lys Ala Asn Tyr Arg Pro Gly

     860         870         880         890         900         910
      '           '           '           '           '           '
GAA AAT CTG AAC CTC TCC TGC CAC GCA GCC TCT AAC CCA CCT GCA CAG TAC TCT TGG
Glu Asn Leu Asn Leu Ser Cys His Ala Ala Ser Asn Pro Pro Ala Gln Tyr Ser Trp

        920         930         940         950         960         970
         '           '           '           '           '           '
TTT ATC AAT GGG ACG TTC CAG CAA TCC ACA CAA GAG CTC TTT ATC CCC AAC ATC ACT
Phe Ile Asn Gly Thr Phe Gln Gln Ser Thr Gln Glu Leu Phe Ile Pro Asn Ile Thr

           980         990        1000        1010        1020
            '           '           '           '           '
GTG AAT AAT AGC GGA TCC TAT ATG TGC CAA GCC CAT AAC TCA GCC ACT GGC CTC AAT
Val Asn Asn Ser Gly Ser Tyr Met Cys Gln Ala His Asn Ser Ala Thr Gly Leu Asn

   1030        1040        1050        1060        1070        1080
    '           '           '           '           '           '
AGG ACC ACA GTC ACG ATG ATC ACA GTC TCT GGA AGT GCT CCT GTC CTC TCA GCT GTG
Arg Thr Thr Val Thr Met Ile Thr Val Ser Gly Ser Ala Pro Val Leu Ser Ala Val

      1090        1100        1110        1120        1130        1140
       '           '           '           '           '           '
GCC ACC GTC GGC ATC ACG ATT GGA GTG CTG GCC AGG GTG GCT CTG ATA TAG CAG CCC
Ala Thr Val Gly Ile Thr Ile Gly Val Leu Ala Arg Val Ala Leu Ile ---
```

70

```
       1150         1160         1170         1180         1190
        '            '            '            '            '
TGG TGT ATT TTC GAT ATT TCA GGA AGA CTG GCA GAT TGG ACC AGA CCC TGA ATT CTT


 1200        1210         1220         1230         1240        1250
  '           '            '            '            '           '
CTA GCT CCT CCA ATC CCA TTT TAT CCC ATG GAA CCA CTA AAA ACA AGG TCT GCT CTG


   1260        1270        1280         1290        1300        1310
    '           '           '            '           '           '
CTC CTG AAG CCC TAT ATG CTG GAG ATG GAC AAC TCA ATG AAA ATT TAA AGG AAA AAC


   1320        1330        1340         1350        1360        1370
    '           '           '            '           '           '
CCT CAG GCC TGA GGT GTG TGC CAC TCA GAG ACT TCA CCT AAC TAG AGA CAG GCA AAC


        1380        1390         1400        1410        1420
         '           '            '           '           '
TGC AAA CCA nnC CTC TTT CGC TTG GCA GGA TGA TGG TGT CAT TAG TAT TTC ACA AGA


 1430        1440         1450        1460         1470        1480
  '           '            '           '            '           '
AGT AGC TTC AGA GGG TAA CTT AAC AGA GTA TCA GAT CTA TCT TGT CAA TCC CAA CGT


   1490        1500         1510        1520        1530        1540
    '           '            '           '           '           '
TTT ACA TAA AAT AAG CGA TCC TTT AGT GCA CCC AGT GAC TGA CAT TAG CAG CAT CTT


        1550         1560        1570         1580        1590
         '            '           '            '           '
' TAA CAC AGC CGT GTG TTC AAG TGT ACA GTG GTC CTT TTC AGA GTT GGn nnT ACT CCA


 1600        1610         1620        1630         1640        1650
  '           '            '           '            '           '
ACT GAA ATG TTA AGG AAG AAG ATA GAT CCA ATT AAA AAA AAT TAA AAC CAA TTT AAA


    1660        1670        1680         1690        1700        1710
     '           '           '            '           '           '
AAA AAA AAA GAA CAC AGG AGA TTC CAG TCT ACT TGA GTT AGC ATA ATA CAG AAG TCC


        1720        1730         1740        1750        1760
         '           '            '           '           '
CCT CTA CTT TAA CTT TTA CAA AAA AGT AAC CTG AAC TAA TCT GAT GTT AAC CAA TGT
```

71

EP 0 263 933 B1

```
   1770        1780.       1790        1800        1810        1820
    '           '           '           '           '           '
  ATT TAT TTG TCT GGT TCT GTT TCC TTG TTC CAA TTT GAC AAA ACC CAC TGT TCT TGT


   1830        1840        1850        1860        1870        1880
    '           '           '           '           '           '
  ATT GTA TTG CCC AGG GGG AGC TAT CAC TGT ACT TGT AGA GTG GTG CTG CTT TAA GTT


   1890        1900        1910        1920        1930        1940
    '           '           '           '           '           '
  CAT AAA TCA CAA ATA AAA GCC AAT TAG CTC TAT AAC TAA AAA AAA AAA AAA AAA AAA


   1950        1960
    '           '
  AAA AAA AAA AAG AAA AAA AAA AAA
```

**2.** Véhicule de clonage recombinant apte à une réplication, comportant un produit d'insertion comprenant un acide nucléique selon la revendication 1.

**3.** Cellule qui est transfectée, infectée par un véhicule de clonage recombinant apte à une réplication selon la revendication 2, ou encore à laquelle on a injecté ce dernier.

**4.** Lignées cellulaires dont les numéros de dépôt ATCC sont ATCC 67312 et ATCC 67169.

**5.** Procédé pour préparer un polypeptide appartenant à la famille des CEA, ledit procédé comprenant les étapes consistant à :
　　(a) cultiver la cellule de la revendication 3 ou les lignées cellulaires de la revendication 4 et
　　(b) récupérer le polypeptide du CEA exprimé par ladite cellule ou par lesdites lignées cellulaires.

**6.** Peptide possédant une séquence d'amino-acides dérivée des séquences telles que mentionnées à la revendication 1, ledit peptide étant choisi parmi le groupe comprenant
　　CEA-B　　(i) résidus d'amino-acides 135 jusqu'à, et y compris, 386.
　　　　　　(ii) résidus d'amino-acides 174 jusqu'à, et y compris, 220.
　　　　　　(iii) résidus d'amino-acides 285 jusqu'à, et y compris, 386.
　　　　　　(iv) résidus d'amino-acides 174 jusqu'à, et y compris, 200.
　　　　　　(v) résidus d'amino-acides 208 jusqu'à, et y compris, 216.
　　　　　　(vi) résidus d'amino-acides 285 jusqu'à, et y compris, 290.
　　　　　　(vii) résidus d'amino-acides 292 jusqu'à, et y compris, 308.
　　　　　　(viii) résidus d'amino-acides 312 jusqu'à, et y compris, 321.
　　　　　　(ix) résidus d'amino-acides 360 jusqu'à, et y compris, 372.
　　　　　　(x) résidus d'amino-acides 380 jusqu'à, et y compris, 386.
　　CEA-C　　(i) résidus d'amino-acides 135 jusqu'à, et y compris, 386.
　　　　　　(ii) résidus d'amino-acides 411 jusqu'à, et y compris, 492.
　　　　　　(iii) résidus d'amino-acides 174 jusqu'à, et y compris, 220.
　　　　　　(vi) résidus d'amino-acides 174 jusqu'à, et y compris, 200.
　　　　　　(v) résidus d'amino-acides 285 jusqu'à, et y compris, 389.
　　　　　　(vi) résidus d'amino-acides 208 jusqu'à, et y compris, 222.
　　　　　　(vii) résidus d'amino-acides 285 jusqu'à, et y compris, 292.
　　　　　　(viii) résidus d'amino-acides 295 jusqu'à, et y compris, 311.
　　　　　　(ix) résidus d'amino-acides 315 jusqu'à, et y compris, 328.
　　　　　　(x) résidus d'amino-acides 331 jusqu'à, et y compris, 345.
　　　　　　(xi) résidus d'amino-acides 348 jusqu'à, et y compris, 360.

72

(xii) résidus d'amino-acides 363 jusqu'à, et y compris, 377.

(xiii) résidus d'amino-acides 383 jusqu'à, et y compris, 390.

CEA-D    (i) résidus d'amino-acides 135 jusqu'à, et y compris, 285.

(ii) résidus d'amino-acides 168 jusqu'à, et y compris, 220.

(iii) résidus d'amino-acides 168 jusqu'à, et y compris, 196.

(vi) résidus d'amino-acides 210 jusqu'à, et y compris, 220 et

(v) résidus d'amino-acides 264 jusqu'à, et y compris, 283.

7. Procédé pour préparer un anticorps dirigé contre un polypeptide appartenant à la famille des CEA, ledit procédé comprenant les étapes consistant à :

(a) préparer ledit polypeptide par le procédé de la revendication 5,

(b) injecter ledit polypeptide dans un hôte capable de produire des anticorps, et

(c) récupérer lesdits anticorps.

8. Procédé pour préparer un anticorps monoclonal capable de reconnaître un polypeptide appartenant à la famille des CEA, ledit procédé comprenant les étapes consistant à :

(a) préparer ledit polypeptide par le procédé de la revendication 5,

(b) injecter ledit polypeptide dans un hôte capable de produire des anticorps,

(c) récupérer dudit hôte les lymphocytes producteurs d'anticorps,

(d) fusionner lesdits lymphocytes à une lignée cellulaire de myélome pour obtenir ainsi des cellules d'hybridome productrices d'anticorps,

(e) cultiver les cellules d'hybridome, et

(f) récupérer les anticorps monoclonaux produits par les cellules,

9. Procédé pour préparer un anticorps dirigé contre un polypeptide appartenant à la famille des CEA, ledit procédé comprenant les étapes consistant à :

(a) injecter un peptide de la revendication 6 dans un hôte capable de produire des anticorps et

(b) récupérer lesdits anticorps.

10. Procédé pour préparer un anticorps monoclonal capable de reconnaître un polypeptide appartenant à la famille des CEA, ledit procédé comprenant les étapes consistant à :

(a) injecter un peptide de la revendication 6 dans un hôte capable de produire des anticorps,

(b) récupérer dudit hôte les lymphocytes producteurs d'anticorps,

(c) fusionner lesdits lymphocytes avec une lignée cellulaire de myélome pour obtenir ainsi des cellules d'hybridome productrices d'anticorps,

(d) cultiver les cellules d'hybridome, et

(e) récupérer les anticorps monoclonaux produits par les cellules.

11. Procédé selon les revendications 7 à 11, dans lequel l'anticorps est spécifique pour un polypeptide particulier appartenant à la famille des gènes CEA.

12. Procédé d'immunodosage pour détecter, dans un échantillon d'essai, un polypeptide appartenant à la famille des CEA, ledit procédé comprenant les étapes consistant à :

(a) préparer un anticorps dirigé contre ledit polypeptide, par le procédé selon l'une quelconque des revendications 7 à 11,

(b) détecter la liaison de ce dernier à des CEA contenus dans l'échantillon.

13. Procédé pour préparer un nécessaire d'essai comprenant un polypeptide appartenant à la famille des CEA ou un anticorps dirigé contre ledit polypeptide, ledit procédé comprenant les étapes consistant à :

(a) préparer ledit polypeptide par le procédé de la revendication 5 ou ledit anticorps par le procédé selon l'une quelconque des revendications 7 à 11, et

(b) préparer un nécessaire d'essai contenant ledit polypeptide ou ledit anticorps.

14. Procédé pour préparer un produit pharmaceutique comprenant un polypeptide appartenant à la famille des CEA ou un anticorps dirigé contre ledit polypeptide, ledit procédé comprenant les étapes consistant à :

(a) préparer ledit polypeptide par le procédé de la revendication 5 ou ledit anticorps par le procédé selon l'une quelconque des revendications 7 à 11, et

(b) préparer un nécessaire d'essai contenant ledit polypeptide ou ledit anticorps.

**15.** Utilisation d'un acide nucléique selon la revendication 1, dans un dosage par hybridation nucléique.

**16.** Produit pharmaceutique ou nécessaire d'essai comprenant un ou plusieurs des peptides de la revendication 6.

# FIG. 1

```
          10            20            30            40            50
           •             •             •             •             •
GG GGT TTA CAC AAC CAC CAC CCC ATC AAA CCC TTC ATC ACC AGC AAC AAC TCC AAC CCC
Gly Leu His Asn His His Pro Ile Lys Pro Phe Ile Thr Ser Asn Asn Ser Asn Pro

  60            70            80            90           100           110
   •             •             •             •             •             •
GTG GAG GAT GAG GAT GCT GTA GCC TTA ACC TGT GAA CCT GAG ATT CAG AAC ACA ACC
Val Glu Asp Glu Asp Ala Val Ala Leu Thr Cys Glu Pro Glu Ile Gln Asn Thr Thr

      120           130           140           150           160           170
       •             •             •             •             •             •
TAC CTG TGG TGG GTA AAT AAT CAG AGC CTC CCG GTC AGT CCC AGG CTG CAG CTG TCC
Tyr Leu Trp Trp Val Asn Asn Gln Ser Leu Pro Val Ser Pro Arg Leu Gln Leu Ser

          180           190           200           210           220           230
           •             •             •             •             •             •
AAT GAC AAC AGG ACC CTC ACT CTA CTC AGT GTC ACA AGG AAT GAT GTA GGA CCC TAT
Asn Asp Asn Arg Thr Leu Thr Leu Leu Ser Val Thr Arg Asn Asp Val Gly Pro Tyr

              240           250           260           270           280
               •             •             •             •             •
GAG TGT GGA ATC CAG AAC GAA TTA AGT GTT GAC CAC AGC GAC CCA GTC ATC CTG AAT
Glu Cys Gly Ile Gln Asn Glu Leu Ser Val Asp His Ser Asp Pro Val Ile Leu Asn

  290           300           310           320           330           340
   •             •             •             •             •             •
GTC CTC TAT GGC CCA GAC GAC CCC ACC ATT TCC CCC TCA TAC ACC TAT TAC CGT CCA
Val Leu Tyr Gly Pro Asp Asp Pro Thr Ile Ser Pro Ser Tyr Thr Tyr Tyr Arg Pro

      350           360           370           380           390           400
       •             •             •             •             •             •
GGG GTG AAC CTC AGC CTC TCC TGC CAT GCA GCC TCT AAC CCA CCT GCA CAG TAT TCT
Gly Val Asn Leu Ser Leu Ser Cys His Ala Ala Ser Asn Pro Pro Ala Gln Tyr Ser

          410           420           430           440           450
           •             •             • •            •             •
TGG CTG ATT GAT GGG AAC ATC CAG CAA CAC ACA CAA GAG CTC TTT ATC TCC AAC ATC
Trp Leu Ile Asp Gly Asn Ile Gln Gln His Thr Gln Glu Leu Phe Ile Ser Asn Ile

  460           470           480           490           500           510
   •             •             •             •             •             •
ACT GAG AAG AAC AGC GGA CTC TAT ACC TGC CAG GCC AAT AAC TCA GCC AGT GGC CAC
Thr Glu Lys Asn Ser Gly Leu Tyr Thr Cys Gln Ala Asn Asn Ser Ala Ser Gly His

      520           530           540           550           560           570
       •             •             •             •             •             •
AGC AGG ACT ACA GTC AAG ACA ATC ACA GTC TCT GCG GAC GTG CCC AAG CCC TCC ATC
Ser Arg Thr Thr Val Lys Thr Ile Thr Val Ser Ala Asp Val Pro Lys Pro Ser Ile

          580           590           600           610           620
           •             •             •             •             •
TCC AGC AAC AAC TCC AAA CCC GTG GAG GAC AAG GAT GCT GTG GCC TTC CAC TGT GAA
Ser Ser Asn Asn Ser Lys Pro Val Glu Asp Lys Asp Ala Val Ala Phe His Cys Glu

630           640           650           660           670           680
 •             •             •             •             •             •
CCT GAG GCT CAG AAC ACA ACC TAC CTG TGG TGG GTA AAT GGT CAG AGC CTC CCA GTC
Pro Glu Ala Gln Asn Thr Thr Tyr Leu Trp Trp Val Asn Gly Gln Ser Leu Pro Val

  690           700           710           720           730           740
   •             •             •             •             •             •
AGT CCC AGG CTG CAG CTG TCC AAT GGC AAC AGG ACC CTC ACT CTA TTC AAT GTC ACA
Ser Pro Arg Leu Gln Leu Ser Asn Gly Asn Arg Thr Leu Thr Leu Phe Asn Val Thr

      750           760           770           780           790           800
       •             •             •             •             •             •
AGA AAT GAC GCA AGA GCC TAT GTA TGT GGA ATC CAG AAC TCA GTG AGT GCA AAC CGC
Arg Asn Asp Ala Arg Ala Tyr Val Cys Gly Ile Gln Asn Ser Val Ser Ala Asn Arg

          810           820           830           840           850
           •             •             •             •             •
AGT GAC CCA GTC ACC CTG GAT GTC CTC TAT GGG CCG GAC ACC CCC ATC ATT TCC CCC
Ser Asp Pro Val Thr Leu Asp Val Leu Tyr Gly Pro Asp Thr Pro Ile Ile Ser Pro

860
 •
CCC CC
Pro
```

FIG. 2  ENZYME IMMUNOASSAY OF CEA FUSION PROTEIN

EP 0 263 933 B1

FIG. 3    IMMUNOBLOT OF

CEA FUSION PROTEIN

FIG. 4

LoVo
control

kb
4.0 –
3.4 –
3.0 –

poly A$^+$
RNA

# FIG.5a

```
            10          20          30          40          50
             *           *           *           *           *
C ACC ATG GAG TCT CCC TCG GCC CCT CTC CAC AGA TGG TGC ATC CCC TGG CAG AGG CTC
  Met Glu Ser Pro Ser Ala Pro Leu His Arg Trp Cys Ile Pro Trp Gln Arg Leu

       60          70          80          90         100         110
        *           *           *           *           *           *
  CTG CTC ACA GCC TCA CTT CTA ACC TTC TGG AAC CCG CCC ACC ACT GCC AAG CTC ACT
  Leu Leu Thr Ala Ser Leu Leu Thr Phe Trp Asn Pro Pro Thr Thr Ala Lys Leu Thr
                                                                           1  2  3

      120         130         140         150         160         170
        *           *           *           *           *           *
  ATT GAA TCC ACG CCG TTC AAT GTC GCA GAG GGG AAG GAG GTG CTT CTA CTT GTC CAC
  Ile Glu Ser Thr Pro Phe Asn Val Ala Glu Gly Lys Glu Val Leu Leu Leu Val His

          180         190         200         210         220
            *           *           *           *           *
  AAT CTG CCC CAG CAT CTT TTT GGC TAC AGC TGG TAC AAA GGT GAA AGA GTG GAT GGC
  Asn Leu Pro Gln His Leu Phe Gly Tyr Ser Trp Tyr Lys Gly Glu Arg Val Asp Gly

  230         240         250         260         270         280
    *           *           *           *           *           *
  AAC CGT CAA ATT ATA GGA TAT GTA ATA GGA ACT CAA CAA GCT ACC CCA GGC CCC GCA
  Asn Arg Gln Ile Ile Gly Tyr Val Ile Gly Thr Gln Gln Ala Thr Pro Gly Pro Ala

      290         300         310         320         330         340
        *           *           *           *           *           *
  TAC AGT GGT CGA GAG ATA ATA TAC CCC AAT GCA TCC CTG CTG ATC CAG AAC ATC ATC
  Tyr Ser Gly Arg Glu Ile Ile Tyr Pro Asn Ala Ser Leu Leu Ile Gln Asn Ile Ile

          350         360         370         380         390         400
            *           *           *           *           *           *
  CAG AAT GAC ACA GGA TTC TAC ACC CTA CAC GTC ATA AAG TCA GAT CTT GTG AAT GAA
  Gln Asn Asp Thr Gly Phe Tyr Thr Leu His Val Ile Lys Ser Asp Leu Val Asn Glu

              410         420         430         440         450
                *           *           *           *           *
  GAA GCA ACT GGC CAG TTC CGG GTA TAC CCG GAG CTG CCC AAG CCC TCC ATC TCC AGC
  Glu Ala Thr Gly Gln Phe Arg Val Tyr Pro Glu Leu Pro Lys Pro Ser Ile Ser Ser

  460         470         480         490         500         510
    *           *           *           *           *           *
  AAC AAC TCC AAA CCC GTG GAG GAC AAG GAT GCT GTG GCC TTC ACC TGT GAA CCT GAG
  Asn Asn Ser Lys Pro Val Glu Asp Lys Asp Ala Val Ala Phe Thr Cys Glu Pro Glu

      520         530         540         550         560         570
        *           *           *           *           *           *
  ACT CAG GAC GCA ACC TAC CTG TGG TGG GTA AAC AAT CAG AGC CTC CCG GTC AGT CCC
  Thr Gln Asp Ala Thr Tyr Leu Trp Trp Val Asn Asn Gln Ser Leu Pro Val Ser Pro

          580         590         600         610         620
            *           *           *           *           *
  AGG CTG CAG CTG TCC AAT GGC AAC AGG ACC CTC ACT CTA TTC AAT GTC ACA AGA AAT
  Arg Leu Gln Leu Ser Asn Gly Asn Arg Thr Leu Thr Leu Phe Asn Val Thr Arg Asn

  630         640         650         660         670         680
    *           *           *           *           *           *
  GAA CAA GCA AGC TAC AAA TGT GAA ACC CAG AAC CCA GTG AGT GCC AGG CGC AGT GAT
  Glu Gln Ala Ser Tyr Lys Cys Glu Thr Gln Asn Pro Val Ser Ala Arg Arg Ser Asp

      690         700         710         720         730         740
        *           *           *           *           *           *
  TCA GTC ATC CTG AAT GTC CTC TAT GGC CCG GAT GCC CCC ACC ATT TCC CCT CTA AAC
  Ser Val Ile Leu Asn Val Leu Tyr Gly Pro Asp Ala Pro Thr Ile Ser Pro Leu Asn

          750         760         770         780         790
            *           *           *           *           *
  ACA TCT TAC AGA TCA GGG GAA AAT CTG AAC CTC TCC TGC CAC GCA GCC TCT AAC CCA
  Thr Ser Tyr Arg Ser Gly Glu Asn Leu Asn Leu Ser Cys His Ala Ala Ser Asn Pro

  800         810         820         830         840         850
    *           *           *           *           *           *
  CCT GCA CAG TAC TCT TGG TTT GTC AAT GGG ACT TTC CAG CAA TCC ACC CAA GAG CTC
  Pro Ala Gln Tyr Ser Trp Phe Val Asn Gly Thr Phe Gln Gln Ser Thr Gln Glu Leu

          860         870         880         890         900         910
            *           *           *           *           *           *
  TTT ATC CCC AAC ATC ACT GTG AAT AAT AGT GGA TCC TAT ACG TGC CAA GCC CAT AAC
  Phe Ile Pro Asn Ile Thr Val Asn Asn Ser Gly Ser Tyr Thr Cys Gln Ala His Asn
```

79

# FIG. 5b

```
         920           930           940           950           960           970
          *             ,  *           *             *             *             *
TCA GAC ACT GGC CTC AAT AGG ACC ACA GTC ACG ACG ATC ACA GTC TAT GCA GAG CCA
Ser Asp Thr Gly Leu Asn Arg Thr Thr Val Thr Thr Ile Thr Val Tyr Ala Glu Pro

              980           990          1000          1010          1020
               *             *             *             *             *
CCC AAA CCC TTC ATC ACC AGC AAC AAC TCC AAC CCC GTG GAG GAT GAG GAT GCT GTA
Pro Lys Pro Phe Ile Thr Ser Asn Asn Ser Asn Pro Val Glu Asp Glu Asp Ala Val

   1030          1040          1050          1060          1070          1080
    *             *             *             *             *             *
GCC TTA ACC TGT GAA CCT GAG ATT CAG AAC ACA ACC TAC CTG TGG TGG GTA AAT AAT
Ala Leu Thr Cys Glu Pro Glu Ile Gln Asn Thr Thr Tyr Leu Trp Trp Val Asn Asn

      1090          1100          1110          1120          1130          1140
       *             *             *             *             *             '
CAG AGC CTC CCG GTC AGT CCC AGG CTG CAG CTG TCC AAT GAC AAC AGG ACC CTC ACT
Gln Ser Leu Pro Val Ser Pro Arg Leu Gln Leu Ser Asn Asp Asn Arg Thr Leu Thr

         1150          1160          1170          1180          1190
          *             '             '             '             *
CTA CTC AGT GTC ACA AGG AAT GAT GTA GGA CCC TAT GAG TGT GGA ATC CAG AAC GAA
Leu Leu Ser Val Thr Arg Asn Asp Val Gly Pro Tyr Glu Cys Gly Ile Gln Asn Glu

   1200          1210          1220          1230          1240          1250
    *             '             *             '             *             '
TTA AGT GTT GAC CAC AGC GAC CCA GTC ATC CTG AAT GTC CTC TAT GGC CCA GAC GAC
Leu Ser Val Asp His Ser Asp Pro Val Ile Leu Asn Val Leu Tyr Gly Pro Asp Asp

      1260          1270          1280          1290          1300          1310
       *             *             *             *             '             *
CCC ACC ATT TCC CCC TCA TAC ACC TAT TAC CGT CCA GGG GTG AAC CTC AGC CTC TCC
Pro Thr Ile Ser Pro Ser Tyr Thr Tyr Tyr Arg Pro Gly Val Asn Leu Ser Leu Ser

         1320          1330          1340          1350          1360
          *             *             *             *             *
TGC CAT GCA GCC TCT AAC CCA CCT GCA CAG TAT TCT TGG CTG ATT GAT GGG AAC ATC
Cys His Ala Ala Ser Asn Pro Pro Ala Gln Tyr Ser Trp Leu Ile Asp Gly Asn Ile

   1370          1380          1390          1400          1410          1420
    *             *             *             *             *             *
CAG CAA CAC ACA CAA GAG CTC TTT ATC TCC AAC ATC ACT GAG AAG AAC AGC GGA CTC
Gln Gln His Thr Gln Glu Leu Phe Ile Ser Asn Ile Thr Glu Lys Asn Ser Gly Leu

      1430          1440          1450          1460          1470          1480
       *             *             *             *             *             *
TAT ACC TGC CAG GCC AAT AAC TCA GCC AGT GGC CAC AGC AGG ACT ACA GTC AAG ACA
Tyr Thr Cys Gln Ala Asn Asn Ser Ala Ser Gly His Ser Arg Thr Thr Val Lys Thr

         1490          1500          1510          1520          1530          1540
          *             *             *             *             *             *
ATC ACA GTC TCT GCG GAC GTG CCC AAG CCC TCC ATC TCC AGC AAC AAC TCC AAA CCC
Ile Thr Val Ser Ala Asp Val Pro Lys Pro Ser Ile Ser Ser Asn Asn Ser Lys Pro

         1550          1560          1570          1580          1590
          *             *             *             *             *
GTG GAG GAC AAG GAT GCT GTG GCC TTC ACC TGT GAA CCT GAG GCT CAG AAC ACA ACC
Val Glu Asp Lys Asp Ala Val Ala Phe Thr Cys Glu Pro Glu Ala Gln Asn Thr Thr

   1600          1610          1620          1630          1640          1650
    *             *             *             *             *             *
TAC CTG TGG TGG GTA AAT GGT CAG AGC CTC CCA GTC AGT CCC AGG CTG CAG CTG TCC
Tyr Leu Trp Trp Val Asn Gly Gln Ser Leu Pro Val Ser Pro Arg Leu Gln Leu Ser

      1660          1670          1680          1690          1700          1710
       *             *             *             *             *             *
AAT GGC AAC AGG ACC CTC ACT CTA TTC AAT GTC ACA AGA AAT GAC GCA AGA GCC TAT
Asn Gly Asn Arg Thr Leu Thr Leu Phe Asn Val Thr Arg Asn Asp Ala Arg Ala Tyr

         1720          1730          1740          1750          1760
          *             *             *             *             *
GTA TGT GGA ATC CAG AAC TCA GTG AGT GCA AAC CGC AGT GAC CCA GTC ACC CTG GAT
Val Cys Gly Ile Gln Asn Ser Val Ser Ala Asn Arg Ser Asp Pro Val Thr Leu Asp

   1770          1780          1790          1800          1810          1820
    *             *             *             *             *             *
GTC CTC TAT GGA CCG GAC ACC CCC ATC ATT TCC CCC CCA GAC TCG TCT TAC CTT TCG
Val Leu Tyr Gly Pro Asp Thr Pro Ile Ile Ser Pro Pro Asp Ser Ser Tyr Leu Ser

         1830          1840          1850          1860          1870          1880
          *             *             *             *             *             *
GGA GCG AAC CTC AAC CTC TCC TGC CAC TCG GCC TCT AAC CCA TCC CCG CAG TAT TCT
Gly Ala Asn Leu Asn Leu Ser Cys His Ser Ala Ser Asn Pro Ser Pro Gln Tyr Ser
```

80

# FIG. 5c

```
        1890          1900          1910          1920          1930
         *            *            *            *            *
TGG CGT ATC AAT GGG ATA CCG CAG CAA CAC ACA CAA GTT CTC TTT ATC GCC AAA ATC
Trp Arg Ile Asn Gly Ile Pro Gln Gln His Thr Gln Val Leu Phe Ile Ala Lys Ile

1940          1950          1960          1970          1980          1990
 *            *            *            *            *            *
ACG CCA AAT AAT AAC GGG ACC TAT GCC TGT TTT GTC TCT AAC TTG GCT ACT GGC CGC
Thr Pro Asn Asn Asn Gly Thr Tyr Ala Cys Phe Val Ser Asn Leu Ala Thr Gly Arg

        2000          2010          2020          2030          2040          2050
         *            *            *            *            *            *
AAT AAT TCC ATA GTC AAG AGC ATC ACA GTC TCT GCA TCT GGA ACT TCT CCT GGT CTC
Asn Asn Ser Ile Val Lys Ser Ile Thr Val Ser Ala Ser Gly Thr Ser Pro Gly Leu

        2060          2070          2080          2090          2100          2110
         *            *            *            *            *            *
TCA GCT GGG GCC ACT GTC GGC ATC ATG ATT GGA GTG CTG GTT GGG GTT GCT CTG ATA
Ser Ala Gly Ala Thr Val Gly Ile Met Ile Gly Val Leu Val Gly Val Ala Leu Ile

              2120          2130          2140          2150          2160
               *            *            *            *            *
TAG CAG CCC TGG TGT AGT TTC TTC ATT TCA GGA AGA CTG ACA GTT GTT TTG CTT CTT

2170          2180          2190          2200          2210          2220
 *            *            *            *            *            *
CCT TAA AGC ATT TGC AAC AGC TAC AGT CTA AAA TTG CTT CTT TAC CAA GGA TGT TTA

        2230          2240          2250          2260          2270          2280
         *            *            *            *            *            *
CAG AAA ATA CTC TGA CCA GAG ATC GAG ACC ATC CTA GCC AAC ATC GTG AAA CCC CAT

              2290          2300          2310          2320          2330
               *            *            *            *            *
CTC TAC TAA AAA TAC AAA AAT GAG CTG GGC TTG GTG GCG CGC ACC TGT AGT CCC AGT

2340          2350          2360          2370          2380          2390
 *            *            *            *            *            *
TAC TCG GGA GGC TGA GGC AGG AGA ATC GCT TGA ACC CGG GAG GTG GAG ATT GCA GTG

        2400          2410          2420          2430          2440          2450
         *            *            *            *            *            *
AGC CCA GAT CGC ACC ACT GCA CTC CAG TCT GGC AAC AGA GCA AGA CTC CAT CTC AAA

              2460          2470          2480          2490          2500
               *            *            *            *            *
AAG AAA AGA AAA GAA GAC TCT GAC CTG TAC TCT TGA ATA CAA GTT TCT GAT ACC ACT

2510          2520          2530          2540          2550          2560
 *            *            *            *            *            *
GCA CTG TCT GAG AAT TTC CAA AAC TTT AAT GAA CTA ACT GAC AGC TTC ATG AAA CTG

        2570          2580          2590          2600          2610          2620
         *            *            *            *            *            *
TCC ACC AAG ATC AAG CAG AGA AAA TAA TTA ATT TCA TGG GGA CTA AAT GAA CTA ATG

              2630          2640          2650          2660          2670          2680
               *            *            *            *            *            *
AGG ATA ATA TTT TCA TAA TTT TTT ATT TGA AAT TTT GCT GAT TCT TTA AAT GTC TTG

              2690          2700          2710          2720          2730
               *            *            *            *            *
TTT CCC AGA TTT CAG GAA ACT TTT TTT CTT TTA AGC TAT CCA CTC TTA CAG CAA TTT

2740          2750          2760          2770          2780          2790
 *            *            *            *            *            *
GAT AAA ATA TAC TTT TGT GAA CAA AAA TTG AGA CAT TTA CAT TTT ATC CCT ATG TGG

              2800          2810          2820          2830
               *            *            *            *
TCG CTC CAG ACT TGG GAA ACT ATT CAT GAA TAT TTA TAT TGT ATG
```